# EUROPÄISCHE PATENTSCHRIFT

(11) **EP 2 483 267 B1**
(45) Veröffentlichungstag und Bekanntmachung des Hinweises auf die Patenterteilung: **28.08.2013**
(21) Anmeldenummer: 11730614.2
(22) Anmeldetag: 24.06.2011
(51) Int. Cl.: C07D 409/14, C07D 417/14, C07D 495/04, C07D 495/14, C07D 498/04, C07D 519/00, H01L 51/00, B82Y 10/00

(54) **VERDAMPFBARES ORGANISCH HALBLEITENDES MATERIAL UND DESSEN VERWENDUNG IN EINEM OPTOELEKTRONISCHEN BAUELEMENT**
EVAPORABLE ORGANICALLY SEMICONDUCTIVE MATERIAL AND USE THEREOF IN AN OPTOELECTRONIC COMPONENT
MATÉRIAU ORGANIQUE SEMI-CONDUCTEUR ÉVAPORABLE ET SON UTILISATION DANS UN COMPOSANT OPTO-ÉLECTRONIQUE

(30) Priorität: 24.06.2010 DE 102010030500
(43) Veröffentlichungstag der Anmeldung: 08.08.2012
(73) Patentinhaber: Heliatek GmbH, 01139 Dresden (DE)
(72) Erfinder: HILDEBRANDT, Dirk, 89233 Neu-Ulm (DE); MATTERSTEIG, Gunter, 89075 Ulm (DE); GERDES, Olga, 89081 Ulm (DE); VETTER, Serge, 89075 Ulm (DE); WEISS, Andre, 89081 Ulm (DE)
(74) Vertreter: Epping, Wilhelm
(86) Internationale Anmeldenummer: PCT/EP2011/060668
(87) Internationale Veröffentlichungsnummer: WO 2011/161262

(56) Entgegenhaltungen:
- WO-A1-2006/092134
- US-A1- 2003 021 912
- TING QI ET AL: "Synthesis and properties of fluorene or carbazole-based and dicyanovinyl-capped n-type organic semiconductors", JOURNAL OF MATERIALS CHEMISTRY, Bd. 18, Nr. 10, 1. Januar 2008 (2008-01-01), Seite 1131, XP55006872, ISSN: 0959-9428, DOI: 10.1039/b715920j
- MIKKEL JØRGENSEN ET AL: "Easy Access to 3,8-Diaryldifurano[2,3- a :2',3'- f ]naphthalenes. A New Extended Aromatic System", THE JOURNAL OF ORGANIC CHEMISTRY, Bd. 65, Nr. 25, 1. Dezember 2000 (2000-12-01), Seiten 8783-8785, XP55006875, ISSN: 0022-3263, DOI: 10.1021/jo005646o
- SAHU D ET AL: "Synthesis and applications of novel acceptor-donor-acceptor organic dyes with dithienopyrrole- and fluorene-cores for dye-sensitized solar cells", TETRAHEDRON, ELSEVIER SCIENCE PUBLISHERS, AMSTERDAM, NL, Bd. 67, Nr. 2, 14. Januar 2011 (2011-01-14), Seiten 303-311, XP027557087, ISSN: 0040-4020 [gefunden am 2010-12-11]

## Beschreibung

Die Erfindung betrifft ein organisches halbleitendes Material der allgemeinen Formeln I und II sowie lila.

Die Forschung und Entwicklung an organischen Solarzellen hat insbesondere in den letzten zehn Jahren stark zugenommen. Der maximale bisher für so genannte "kleine Moleküle" berichtete Wirkungsgrad liegt bei 5.7% [Jiangeng Xue, Soichi Uchida, Barry P. Rand, and Stephen R. Forrest, Appl. Phys. Lett. 85 (2004) 5757]. Unter kleinen Molekülen werden im Sinne der vorliegenden Erfindung nicht-polymere organische, monodisperse Moleküle im Massebereich zwischen 100 und 2000 Gramm/Mol verstanden. Damit konnten bisher die für anorganische Solarzellen typischen Effizienzen von 10-20% noch nicht erreicht werden. Organische Solarzellen unterliegen aber denselben physikalischen Limitierungen wie anorganische Solarzellen, weshalb nach entsprechender Entwicklungsarbeit zumindest theoretisch ähnliche Effizienzen zu erwarten sind.

Organische Solarzellen bestehen aus einer Folge dünner Schichten (die typischerweise jeweils 1nm bis 1µm dick sind) aus organischen Materialien, welche bevorzugt im Vakuum aufgedampft oder aus einer Lösung aufgeschleudert werden. Die elektrische Kontaktierung kann durch Metallschichten, transparente leitfähige Oxide (TCOs) und/oder transparente leitfähige Polymere (PEDOT-PSS, PANI) erfolgen.

Eine Solarzelle wandelt Lichtenergie in elektrische Energie um. In diesem Sinne wird der Begriff "photoaktiv" als Umwandlung von Lichtenergie in elektrische Energie verstanden. Im Gegensatz zu anorganischen Solarzellen werden bei organischen Solarzellen durch das Licht nicht direkt freie Ladungsträger erzeugt, sondern es bilden sich zunächst Exzitonen, also elektrisch neutrale Anregungszustände (gebundene Elektron-Loch-Paare). Erst in einem zweiten Schritt werden diese Exzitonen in freie Ladungsträger getrennt, die dann zum elektrischen Stromfluß beitragen.

Der Vorteil solcher Bauelemente auf organischer Basis gegenüber den konventionellen Bauelementen auf anorganischer Basis (Halbleiter wie Silizium, Galliumarsenid) sind die teilweise extrem hohen optischen Absorptionskoeffizienten (bis zu 2x10⁵ cm⁻¹), die es erlauben, effiziente Absorberschichten von nur wenigen Nanometern Dicke herzustellen, so dass sich die Möglichkeit bietet, mit geringem Material- und Energieaufwand sehr dünne Solarzellen herzustellen. Weitere technologische Aspekte sind die niedrigen Kosten, wobei die verwendeten organischen Halbleitermaterialien bei Herstellung in größeren Mengen sehr kostengünstig sind; die Möglichkeit, flexible großflächige Bauteile auf Plastikfolien herzustellen, und die nahezu unbegrenzten Variationsmöglichkeiten und die unbegrenzte Verfügbarkeit der organischen Chemie.

Da im Herstellungsprozess keine hohen Temperaturen benötigt werden, ist es möglich, organische Solarzellen als Bauteile sowohl flexibel als auch großflächig auf preiswerten Substraten, z.B. Metallfolie, Plastikfolie oder Kunststoffgewebe, herzustellen. Dies eröffnet neue Anwendungsgebiete, welche den konventionellen Solarzellen verschlossen bleiben. Auf Grund der nahezu unbegrenzten Anzahl verschiedener organischer Verbindungen können die Materialien für ihre jeweilige Aufgabe maßgeschneidert werden.

Eine in der Literatur bereits vorgeschlagene Realisierungsmöglichkeit einer organischen Solarzelle besteht in einer pin -Diode [Martin Pfeiffer, "Controlled doping of organic vacuum deposited dye layers: basics and applications", PhD thesis TU-Dresden, 1999.] mit folgendem Schichtaufbau:
0. Träger, Substrat,
1. Grundkontakt, meist transparent,
2. p- Schicht(en),
3. i- Schicht(en)
4. n- Schicht(en),
5. Deckkontakt.

Hierbei bedeutet n bzw. p eine n- bzw. p-Dotierung, die zu einer Erhöhung der Dichte freier Elektronen bzw. Löcher im thermischen Gleichgewichtszustand führt. In diesem Sinne sind derartige Schichten primär als Transportschichten zu verstehen. Die Bezeichnung i-Schicht bezeichnet demgegenüber eine undotierte Schicht (intrinsische Schicht). Eine oder mehrere i-Schicht(en) können hierbei Schichten sowohl aus einem Material, als auch eine Mischung aus zwei Materialien (sogenannte interpenetrierende Netzwerke) bestehen. Im Gegensatz zu anorganischen Solarzellen liegen die Ladungsträgerpaare in organischen Halbleitern nach Absorption jedoch nicht frei vor, sondern sie bilden wegen der weniger starken Abschwächung der gegenseitigen Anziehung ein Quasiteilchen, ein so genanntes Exziton. Um die im Exziton vorhandene Energie als elektrische Energie nutzbar zu machen, muss dieses Exziton in freie Ladungsträger getrennt werden. Da in organischen Solarzellen nicht ausreichend hohe Felder zur Trennung der Exzitonen zur Verfügung stehen, wird die Exzitonentrennung an photoaktiven Grenzflächen vollzogen. Die photoaktive Grenzfläche kann als eine organische Donator-Akzeptor-Grenzfläche [C.W. Tang, Appl. Phys. Lett. 48 (1986) 183] oder eine Grenzfläche zu einem anorganischen Halbleiter [B. O'Regan, M. Grätzel, Nature 1991, 353, 737])] ausgeprägt sein. Die Exzitonen gelangen durch Diffusion an eine derartige aktive Grenzfläche, wo Elektronen und Löcher voneinander getrennt werden. Diese kann zwischen der p- (n-) Schicht und der i-Schicht bzw. zwischen zwei i-Schichten liegen. Im eingebauten elektrischen Feld der Solarzelle werden die Elektronen nun zum n-Gebiet und die Löcher zum p-Gebiet abtransportiert. Vorzugsweise handelt es sich bei den Transportschichten um transparente oder weitgehend transparente Materialien mit großer Bandlücke (wide-gap). Als wide-gap Materialien werden hierbei Materialien bezeichnet, deren Absorptionsmaximum im Wellenlängenbereich <450nm liegt, bevorzugt bei <400nm.

Da durch das_ Licht immer erst Exzitonen erzeugt werden und noch keine freien Ladungsträger, spielt die rekombinationsarme Diffusion von Exzitonen an die aktive Grenzfläche eine kritische Rolle bei organischen Solarzellen. Um einen Beitrag zum Photostrom zu leisten, muss daher in einer guten organischen Solarzelle die Exzitonendiffusionslänge die typische Eindringtiefe des Lichts deutlich übersteigen, damit der überwiegende Teil des Lichts genutzt werden kann. Strukturell und bezüglich der chemischen Reinheit perfekte organische Kristalle oder Dünnschichten erfüllen durchaus dieses Kriterium. Für großflächige Anwendungen ist allerdings die Verwendung von monokristallinen organischen Materialien nicht möglich und die Herstellung von Mehrfachschichten mit ausreichender struktureller Perfektion ist bis jetzt noch sehr schwierig.

Ein wichtiger Faktor in der Verbesserung der oben genannten Solarzellen liegt in der Weiterentwicklung der organischen Schichten. Für die Absorberschichten, speziell auf dem Gebiet kleiner Moleküle, sind in den letzten 5 Jahren wenige neue Materialien bekannt geworden.

In der WO2006092134A1 werden Verbindungen offenbart, die über einen Akzeptor-Donor-Akzeptor-Aufbau verfügen, wobei der Donorblock ein ausgedehntes n-System besitzt.

In der DE60205824T2 werden Thienothiophenderivate offenbart, die mit weiteren Aromaten ein n-System bilden und an beiden Seiten von Alkylgruppen eingerahmt sind, und deren Verwendung in organischen Halbleitern.

In der WO2009051390 werden Thiophen basierte Akzeptor-Donator Farbstoffe für den Einsatz in farbstoffsensitiven Solarzellen offenbart.

In der WO 002008145172A1 werden neuartige Phthalocyanine zur Verwendung in Solarzellen vorgestellt.

In der US7655809B2 werden Verbindungen aus 5 kondensierten Kohlenstoffzyklen in Reihe und deren Verwendung als organische Halbleiter offenbart.

In der WO 2006111511A1 und WO2007116001A2 werden Rylentetracarbonsäurederivate zur Verwendung als aktive Schicht in Photovoltaik offenbart.

Dagegen sind verschiedene Polymere zur Verwendung als aktive Schichten in organischer Photovoltaik bekannt, beispielweise offenbart in WO 2008088595A2, EP2072557A1 oder US 20070112171A1. Diese sind im Allgemeinen nicht verdampfbar, sondern werden in flüssiger Form zu dünnen Schichten verarbeitet.

Aus der Publikation Qi et al. J. Mater. Chem. 2008, 18 Seiten 1131 bis 1138 sind n-halbleitende Fluoren- oder carbazol basierte organische Halbleitermaterialien bekannt. Jørgensen et al. J. Org. Chem. 2000, 65, Seiten 8783 bis 8785 beschreiben 3,8-Diaryldifurano[2,3-a:2', 3' -f] Naphtaline als neue aromatische Systeme.

In den letzten 3 Jahren konnten durch die verschiedenen Ansätze regelmäßig verbesserte Effizienzen für organische Solarzellen berichtet werden. Trotzdem sind die derzeitig erreichten Effizienzen noch nicht ausreichend für einen kommerziellen Einsatz.

Aufgabe der Erfindung ist es ein Material bereitzustellen, welches im Vakuum verdampfbar ist und als Lichtabsorber in einer organischen Solarzelle eingesetzt werden kann. Eine weitere Aufgabe der Erfindung besteht darin ein optoelektronsiches Bauelement anzugeben, welches ein organisches verdampfbares Halbleitermaterial enthält, welches die im Stand der Technik benannten Nachteile überwindet.

Erfindungsgemäß wir diese Aufgabe gelöst durch Verbindungen der allgemeinen Formel IIIa: 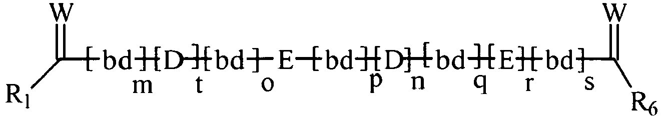
- mit W jeweils unabhängig voneinander ausgesucht aus C(CN)₂, CHCN, C(CN)COOR', mit R' jeweils ausgesucht aus C1-C10-Alkyl, C3-C10-Aryl oder C2-C8-Heteroaryl, besonders bevorzugt ausgesucht aus C(CN)₂, CHCN,
- R₁ und R₆ jeweils unabhängig voneinander ausgesucht aus H, C1-C30 Alkyl, C1-C30 Perfluoralkyl, C3-C10 Aryl, C2-C8-Heteroaryl, CN,
- wobei die Gruppen D ausgesucht sind aus: 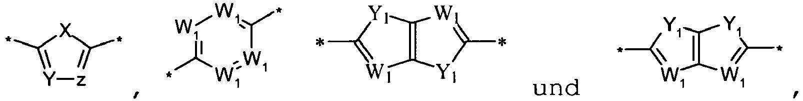
- wobei Y₁ ausgewählt ist aus: O, S, Se, P(R), P(O)R, Si(RR'), C(RR') und N(R) und
- W₁ unabhängig ausgewählt ist aus: N und C-R mit R und R' unabhängig voneinander ausgewählt aus substituiertem oder unsubstituiertem C1 bis C30-Alkyl, C3-C6-Aryl, oder C3-C8-Heteroaryl und
- wobei X jeweils unabhängig voneinander ausgesucht ist aus O, NR', S, Se, mit R' ausgesucht aus C1-C30-Alkyl, C1-C10-Aryl oder C1-C8-Heteroaryl,
- Y jeweils unabhängig voneinander ausgesucht ist aus N oder CR9 mit R9 = H, Halogen, C1-C30 Alkyl, C1-C30 Alkenyl, C1-C30 Alkinyl, jeweils linear oder verzweigt, substituiert oder unsubstituiert, OR', SR', SiR'3, NR'2 mit R' ausgesucht aus C1-C10-Alkyl, C3-C10-Aryl oder C1-C8-Heteroaryl,
- Z jeweils unabhängig voneinander ausgesucht ist aus N oder CR10 mit R10 = H, Halogen, C1-C30 Alkyl, C1-C30 Alkenyl, C1-C30 Alkinyl, jeweils linear oder verzweigt, substituiert oder unsubstituiert), OR', SR', SiR'3, NR'2 mit R' ausgesucht aus C1-C10-Alkyl, C3-C10-Aryl oder C1-C8-Heteroaryl,
- wobei R9 und R10 einen Ring bilden können, bevorzugt einem 5-Ring order 6 - Ring
- wobei die Gruppen E ausgewählt sind aus: 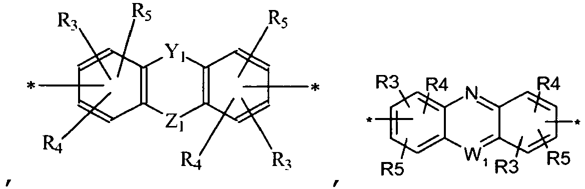 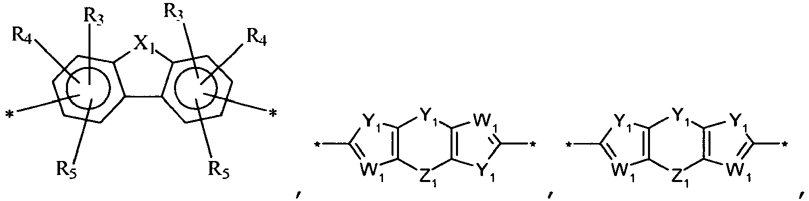 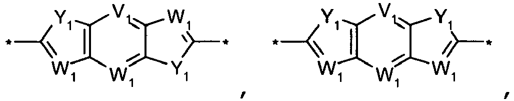 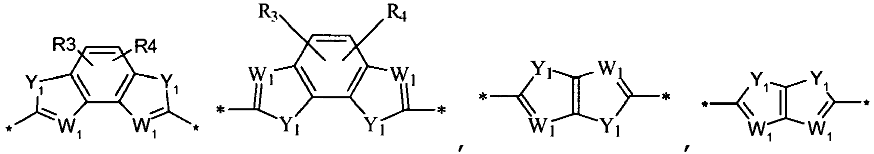 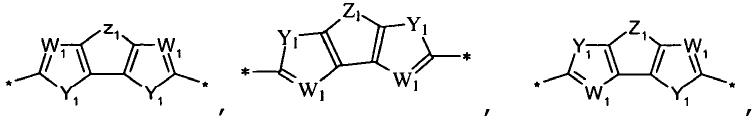 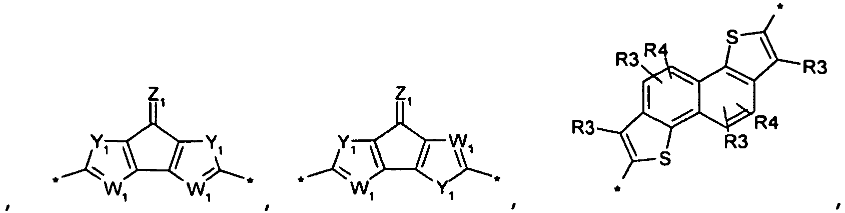 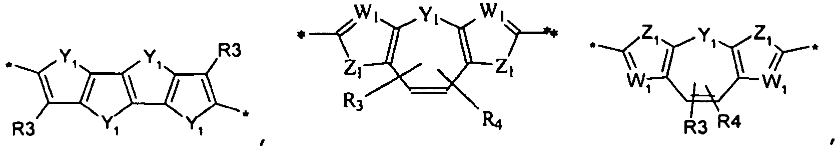
- wobei V₁ und W₁ unabhängig voneinander ausgewählt sind aus: N und C-R mit R = H, Halogen, C1-C30 Alkyl, C1-C30 Alkenyl, C1-C30 Alkinyl, jeweils linear oder verzweigt, substituiert oder unsubstituiert, C3-C10-Aryl oder C1-C8-Heteroaryl, substituiert oder unsubstituiert, OR', SR', SiR'₃, NR'₂ mit R' ausgesucht aus C1-C10-Alkyl, C1-C10-Aryl oder C1-C8-Heteroaryl, bevorzugt ist V₁ und W₁ unabhängig voneinander ausgewählt sind aus: N und C-R mit R = H, Halogen, C1-C30 Alkyl, C1-C30 Alkenyl, C1-C30 Alkinyl, jeweils linear oder verzweigt, substituiert oder unsubstituiert, C3-C10-Aryl oder C1-C8-Heteroaryl, substituiert oder unsubstituiert, OR', SR', SiR⁻₃, NR'₂ mit R' ausgesucht aus C1-C10-Alkyl, C1-C10-Aryl oder C1-C8-Heteroaryl
- Y1 und Z₁ ausgewählt ist aus: O, S, Se, P(R), P(O)R, Si(RR'), C(RR') und N(R) mit R und R' jeweils unabhängig voneinander ausgesucht aus H, linear oder verzweigtem, substituiert oder unsubstituiertem C1-C30 Alkyl, substituiert oder unsubstituiertem Aryl, substituiert oder unsubstituiertem Heteroaryl, Acyl (COR'), COOR' oder OR', mit R' ausgesucht aus C1-C30-Alkyl, C3-C10-Aryl oder C1-C8-Heteroaryl, bevorzugt sind Y1 uns Z1 ausgewählt aus: O, S, Se, Si(RR'), C(RR') und N(R) mit R und R' jeweils unabhängig voneinander ausgesucht aus H, linear oder verzweigtem, substituiert oder unsubstituiertem C1-C30 Alkyl, substituiert oder unsubstituiertem Aryl, substituiert oder unsubstituiertem Heteroaryl, Acyl (COR'), COOR' oder OR', mit R' ausgesucht aus C1-C30-Alkyl, C3-C10-Aryl oder C1-C8-Heteroaryl und
- X1 ausgewählt ist aus: O, S, Se, , und R und R' jeweils unabhängig voneinander ausgewählt ist aus H, C1 bis C20-Alkyl, linear oder verzweigt, substituiert oder unsubstituiert, C3 bis C6 Aryl oder C3 bis C8 Heteroaryl, OR', SR', SiR'3, NR'2 mit R' ausgesucht aus C1-C10-Alkyl, C3-C6-Aryl oder C3-C8-Heteroaryl, bevorzugt ist X1 ausgewählt aus O, S, Se und Si(RR'), und R und R' jeweils unabhängig voneinander ausgewählt ist aus H, C1 bis C20-Alkyl, linear oder verzweigt, substituiert oder unsubstituiert, C3 bis C6 Aryl oder C3 bis C8 Heteroaryl, OR', SR', SiR'3, NR'2 mit R' ausgesucht aus C1-C10-Alkyl, C3-C6-Aryl oder C3-C8-Heteroaryl,
- und wobei R3, R4 und R5 unabhängig voneinander H, C1 bis C20-Alkyl, linear oder verzweigt, substituiert oder unsubstituiert, C3 bis C6 Aryl oder C3 bis C8 Heteroaryl, OR', SR', SiR'3, NR'2 mit R' ausgesucht aus C1-C10-Alkyl, C3-C6-Aryl oder C3-C8-Heteroaryl sein können,
- bd unabhängig voneinander *-C=C-* oder *-C ≡ C-* bedeutet, n, m, o, p, q, und r, s, und t unabhängig voneinander 0 oder 1 sein können mit der Maßgabe, dass mindestens ein Parameter 1 ist und
- wobei eine aus den Gruppen bd, E und D aufgebaute Donoreinheit zumindest 10 konjugierte Elektronen aufweist und
- die mit dem Stern* gekennzeichneten Bindungen, die Bindungen zu weiteren Gruppen in den Verbindungen andeuten.

Bevorzugt sind die erfindungsgemäßen Verbindungen im Bezug auf die Hauptkette symmetrisch, weisen also gleiche Bausteine D und E, sowie falls vorhanden, gleiche Doppel- oder Dreifach-Bindungen bd auf, wobei Substituenten an der Hauptkette unterschiedlich sein können. Insbesondere weisen die erfindungsgemäßen Verbindungen entweder eine Punkt- oder Spiegelsymmetrie im Bezug auf die Hauptkette auf. Derartige symmetrische Verbindungen orientieren sich in geordneter Weise in Schichten in optoelektronischen Bauelementen und sind daher besonders als aktive Schichten in diesen Bauelementen geeignet.

Gemäß einer weiteren bevorzugten Ausführungsform sind die Verbindungen nicht nur bezüglich der Hauptkette sondern auch bezüglich der Substituenten an der Hauptkette, beispielsweise Alkylgruppen oder Ethergruppen symmetrisch.

Weiter bevorzugt weist die aus den Gruppen D, E und bd aufgebaute Donoreinheit zumindest 12 konjugierte Elektronen auf.

Die erfindungsgemäßen Verbindungen sind Oligomere und gut aus dem Vakuum verdampfbar. Diese Verbindungen weisen zwei Elektronenakzeptorblöcke 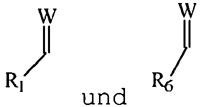 auf, die einen ausgedehnten Elektronendonorblock aufgebaut aus den Gruppen D, E und bd flankieren.

Gemäß einer weiteren Ausführungsform der Erfindung weisen die Verbindungen eine Gruppe D ausgewählt aus 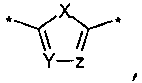 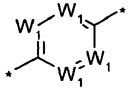 auf, wobei die Parameter r=s=0 sind.

Weiterhin kann die Gruppe E = 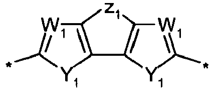 sein mit Y₁ = S und die Gruppen D = 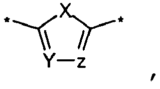 so dass Verbindungen mit der allgemeinen Formel: 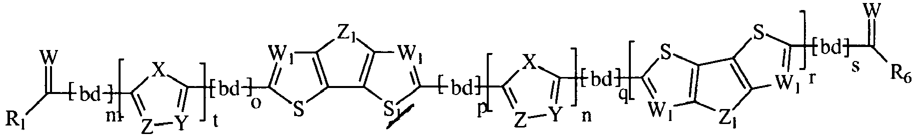 resultieren.

Gemäß einer weiteren Variante der oben genannten allgemeinen Formel weisen die Verbindungen die Parameter m, r, s und q = 0 auf mit der allgemeinen Formel:

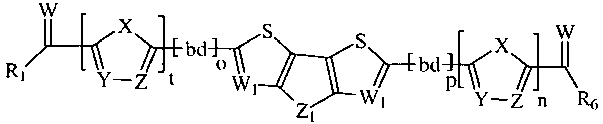

Weiterhin können diese Verbindungen mit o und p = 0 und t und n = 1 und W₁ = C-R durch die allgemeine Formel darstellbar sein:

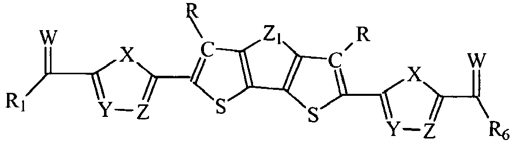

Die Verbindungen können mit den Parametern t, m, o und s =0 und r = 1 durch die allgemeine Formel: 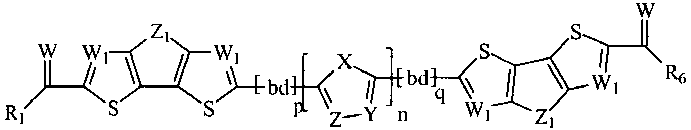 beschrieben werden.

Diese allgemeine Formel kann durch die Wahl der Parameter p und q = 0 und W₁ = C-R in die folgende speziellere Formel überführt werden:

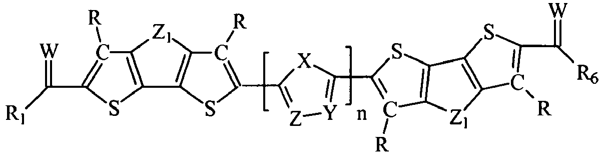

Alternativ können die erfindungsgemäßen Verbindungen die Gruppen D = 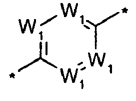 und E = 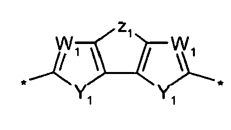 aufweisen.

Derartige Verbindungen können mit m, r, s und q =0 und t und n jeweils = 1 durch die allgemeinen Formel: 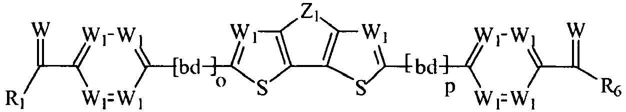 dargestellt werden, wobei bevorzugt die Parameter o und p = 0 und W₁ = C-R gewählt werden.

Weitere Verbindungen können die Gruppen D = 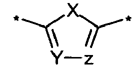 und E ausgewählt aus: 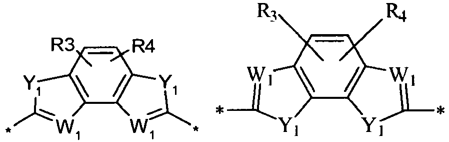 aufweisen.

Diese Verbindungen können die Parameter m, q, r, und s = 0 und t und n jeweils = 1 mit der allgemeinen Formel: 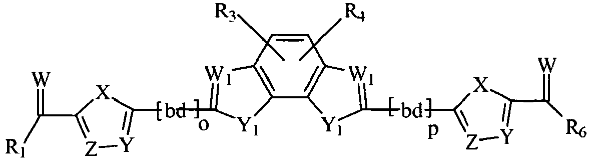 aufweisen, wobei insbesondere X = S, und Y₁ = S sowie W₁ = C-R sein kann.

Weitere alternative Verbindungen weisen die Parameter m, t, n, o, q, s = 0 und r = 1 auf, wobei E ausgewählt ist aus: 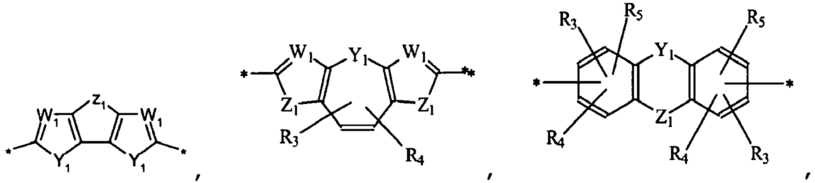 und 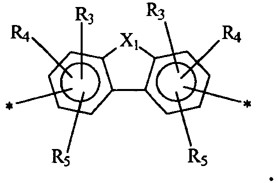 Bei diesen
Verbindungen kann zusätzlich p = 0 sein.

Bevorzugt weisen die Elektronenakzeptor-Gruppen Mono-, Di- oder Tricyanovinylengruppen auf, wobei W ausgewählt ist aus: C(CN)₂, CHCN und R1 und R6 ausgewählt sind aus: H und CN.

Gegenstand einer weiteren Ausführungsform der Erfindung sind Verbindungen der allgemeinen Formel I oder II 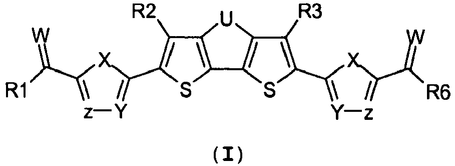 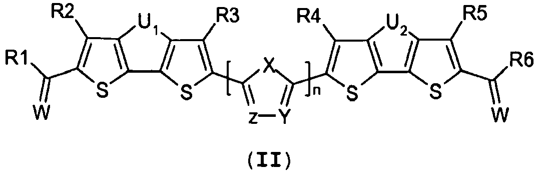 wobei U, U1 und U2 jeweils unabhängig voneinander ausgesucht ist aus N-R7, SiR7R8, CR7R8 mit R7 und R8 jeweils unabhängig voneinander ausgesucht aus H, linear oder verzweigtem, substituiert oder unsubstituiertem C1-C30 Alkyl, substituiert oder unsubstituiertem Aryl, substituiert oder unsubstituiertem Heteroaryl, Acyl (COR'), COOR' oder OR', mit R' ausgesucht aus C1-C30-Alkyl, C3-C10-Aryl oder C1-C8-Heteroaryl,
R2, R3, R4 und R5 jeweils unabhängig voneinander ausgesucht sind aus H, Halogen, C1-C30 Alkyl, C1-C30 Alkenyl, C1-C30 Alkinyl, jeweils linear oder verzweigt, substituiert oder unsubstituiert, C3-C10-Aryl oder C1-C8-Heteroaryl, substituiert oder unsubstituiert, OR', SR', SiR'₃, NR'₂ mit R' ausgesucht aus C1-C10-Alkyl, C1-C10-Aryl oder C1-C8-Heteroaryl,
X jeweils unabhängig voneinander ausgesucht ist aus O, NR', S, Se, mit R' ausgesucht aus C1-C30-Alkyl, C1-C10-Aryl oder C1-C8-Heteroaryl,
Y jeweils unabhängig voneinander ausgesucht ist aus N oder CR9 mit R9 = H, Halogen, C1-C30 Alkyl, C1-C30 Alkenyl, C1-C30 Alkinyl, jeweils linear oder verzweigt, substituiert oder unsubstituiert, OR', SR', SiR'₃, NR'₂ mit R' ausgesucht aus C1-C10-Alkyl, C1-C10-Aryl oder C1-C8-Heteroaryl,
Z jeweils unabhängig voneinander ausgesucht ist aus N oder CR10 mit R10 = H, Halogen, C1-C30 Alkyl, C1-C30 Alkenyl, C1-C30 Alkinyl, jeweils linear oder verzweigt, substituiert oder unsubstituiert), OR', SR', SiR'₃, NR'₂ mit R' ausgesucht aus C1-C10-Alkyl, C1-C10-Aryl oder C1-C8-Heteroaryl,
R1 und R6 jeweils unabhängig voneinander ausgesucht sind aus H, C1-C30 Alkyl, C1-C30 Perfluoralkyl, C3-C10 Aryl, CN, W jeweils unabhängig voneinander ausgesucht ist aus O,
C(CN)₂, C(CN)COOR' mit R' ausgesucht aus C1-C10-Alkyl, C1-C10-Aryl oder C1-C8-Heteroaryl und
n 0 oder 1 ist.

In einer Ausführungsform der Erfindung bilden R9 und R10 einen Ring, bevorzugt einen 5-Ring oder 6-Ring.

Im Rahmen der Formel I bevorzugte Verbindungen liegen vor wenn
U N-R7 ist mit R7 ausgesucht aus geradkettigem oder verzweigtem, substituierten oder unsubstituierten Alkyl oder Cycloalkyl mit 1-10 C-Atomen oder substituierten oder
unsubsubstituierten Aryl oder Hetereoaryl mit 3-10 Atomen, R1 bis R6 H sind,
Y C-R9 ist mit R9 ausgesucht aus H, Halogen, C1-C30 Alkyl, C1-C30 Alkenyl, C1-C30 Alkinyl, jeweils linear oder verzweigt, substituiert oder unsubstituiert, OR', SR', SiR'₃, NR'₂ mit R' ausgesucht aus C1-C10-Alkyl, C1-C10-Aryl oder C1-C8-Heteroaryl,
Z oder C-R10 ist mit R10 ausgesucht aus H, Halogen, C1-C30 Alkyl, C1-C30 Alkenyl, C1-C30 Alkinyl, jeweils linear oder verzweigt, substituiert oder unsubstituiert), OR', SR', SiR'₃, NR'₂ mit R' ausgesucht aus C1-C10-Alkyl, C1-C10-Aryl oder C1-C8-Heteroaryl,
X S ist und
W C(CN)₂ ist.

Im Rahmen von Formel II bevorzugte Verbindungen liegen vor wenn
n 0 oder 1 ist,
U N-R7 oder CR7R8 ist mit R7 und R8 unabhängig voneinander ausgesucht aus geradkettigem oder verzweigtem, substituierten oder unsubstituierten Alkyl oder Cycloalkyl mit 1-10 C-Atomen oder substituierten oder unsubsubstituierten Aryl oder Hetereoaryl mit 3-10 Atomen,
Y oder C-R9 ist mit R9 ausgesucht aus H, Halogen, C1-C30 Alkyl, C1-C30 Alkenyl, C1-C30 Alkinyl, jeweils linear oder verzweigt, substituiert oder unsubstituiert, OR', SR', SiR'₃, NR'₂ mit R' ausgesucht aus C1-C10-Alkyl, C1-C10-Aryl oder C1-C8-Heteroaryl,
Z oder C-R10 ist mit R10 ausgesucht aus H, Halogen, C1-C30 Alkyl, C1-C30 Alkenyl, C1-C30 Alkinyl, jeweils linear oder verzweigt, substituiert oder unsubstituiert), OR', SR', SiR'₃, NR'₂ mit R' ausgesucht aus C1-C10-Alkyl, C1-C10-Aryl oder C1-C8-Heteroaryl,
W C(CN)₂ ist und
R1 bis R6 H sind.

Im Einzelnen seien beispielweise die folgenden Verbindungen der allgemeinen Formel I a bis k genannt:

| N r | U | R7 | R8 | R2/R 3 | Y | Z | R1/R 6 | W | X |
|---|---|---|---|---|---|---|---|---|---|
| a | NR7 | Propy l | - | H | C-Butyl | CH | H | C (CN) 2 | S |
| b | NR7 | Propy l | - | H | C-Butyl | CH | H | O | S |
| c | NR7 | Pheny l | - | H | C-Butyl | CH | H | C (CN) 2 | S |
| d | NR7 | Pheny l | - | H | C-Butyl | CH | H | O | S |
| e | NR7 | Methy l | - | H | CH | CH | H | C (CN) 2 | S |
| f | NR7 | Propy l | - | H | CH | CH | H | C (CN) 2 | O |
| g | NR7 | Hexyl | - | H | CH | CH | H | C (CN) 2 | S |
| h | SiR7R 8 | Propyl | Propy l | H | C-Butyl | CH | H | C (CN) 2 | S |
| l | SiR7R 8 | Propy l | Propy l | H | C-Butyl | CH | H | O | S |
| j | CR7R8 | Propy l | Propy l | H | C-Propy l | C-Propy l | H | C (CN) 2 | S |
| k | CR7R8 | Propy l | Propy 1 | H | C-Propy l | C-Propy l | H | O | S |

Im Einzelnen seien beispielweise die folgenden Verbindungen der allgemeinen Formel II a bis f genannt:

| | | | | | | | | | | |
|---|---|---|---|---|---|---|---|---|---|---|
| N r | U1/U2 | R7 | R8 | R2-R5 | Y | Z | R1/ R6 | W | X | n |
| a | NR7 | Propy l | - | H | - | - | H | C (CN) 2 | - | 0 |
| b | NR7 | Propy l | - | H | - | - | H | O | - | 0 |
| c | NR7 | Propy l | - | H | C-Propyl | C-Propyl | H | C (CN) 2 | S | 1 |
| d | NR7 | Propy l | - | H | C-Propyl | C-Propyl | H | O | S | 1 |
| e | CR7R8 | Propy l | Prop yl | H | - | - | H | C (CN) 2 | - | 0 |
| f | CR7R8 | Propy l | Prop yl | H | - | - | H | O | - | 0 |

Die Herstellung der erfindungsgemäßen Verbindungen lässt sich über vielfältige dem Fachmann bekannte Reaktionsschritte realisieren. Der zentrale Baustein mit U, U1 oder U2 = NR lässt sich beispielsweise nach Zotti (Zotti et al, Makromol. Chem. 1992, 193, 399)

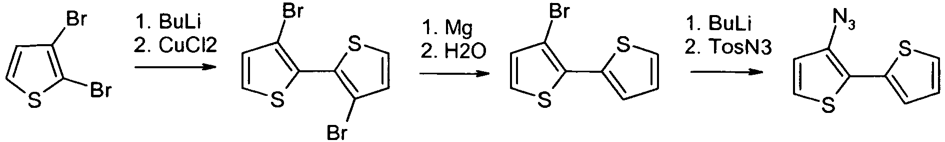

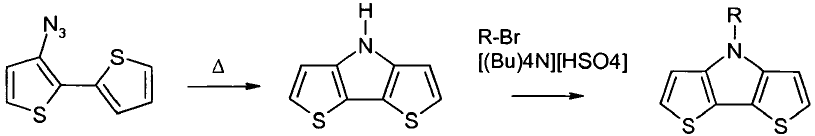

Oder nach Rasmussen (Rasmussen et al, Org. Chem. 2001, 66, 9067)

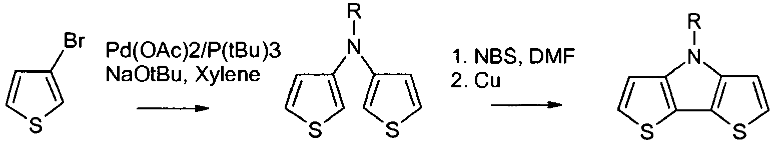

Oder nach Barlow (Barlow et al, Chem. Eur. J. 2007, 13, 9637) 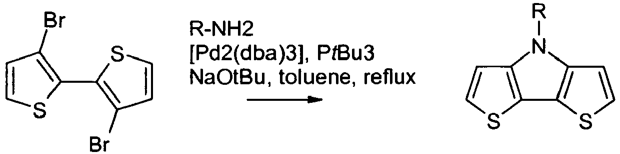 herstellen.

Ein zentraler Baustein mit U, U1 oder U2 = CR₂ lässt sich beispielsweise nach Jacek Z. Brzezinski, John R. Reynolds, Synthesis 2002 (8) 1053-1056 herstellen:

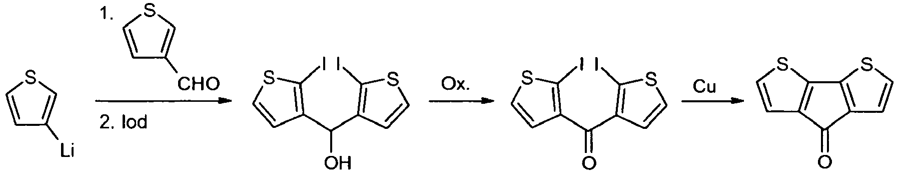

Ein zentraler Baustein mit U, U1 oder U2 = SiR2 lässt sich beispielsweise nach Hakan Usta, Gang Lu, Antonio Facchetti, Tobin J. Marks, J. Am. Chem. Soc. 2006 (128) 9034-9035 herstellen:

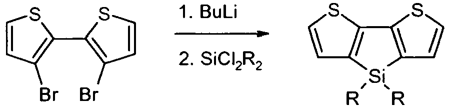

Die Kopplung der weiteren heterocyclischen 5-Ringe erfolgt über eine der üblichen Methoden zur unsymmetrischen Verknüpfung zweier (hetero-)Aromaten (z.B. Negishi, Stille, Suzuki, Kumada etc.) und ist dem Fachmann bekannt (Rasmussen et al, Org. Lett. 2005, 7, 23, 5253, McCullough et al, Adv. Funct. Mat.2009, 19, 3427, Gronowitz, Hörnfeldt, "Thiophenes", Elsevier 2004):

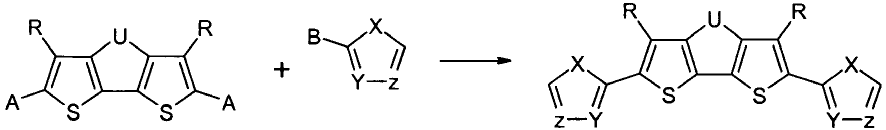

Dabei sind A und B jeweils Metall- oder Halogenkomponenten.

Die Kopplung zweier zentraler Bausteine zu einer Verbindung der allgemeinen Formel II mit n=0 kann nach den oben beschriebene Methoden unsymmetrisch stattfinden, wobei A'=B ist:

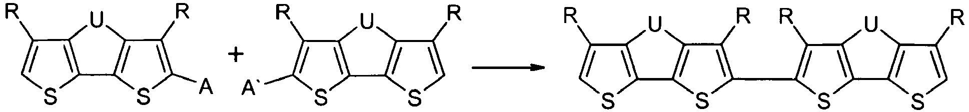

Darüber hinaus ist eine symmetrische homolytische Kopplung für den Fall A'= A = Halogen möglich, beispielsweise durch Umsetzung mit Magnesium gefolgt von Kupferchlorid oder BuLi gefolgt von Kupferchlorid (Allg. Beschreibung in Gronowitz, Hörnfeldt, "Thiophenes", Elsevier 2004).

Der Aufbau von Verbindungen der allgemeinen Formel II mit n=1 gelingt beispielsweise nach den bereits benannten Kopplungsmethoden: 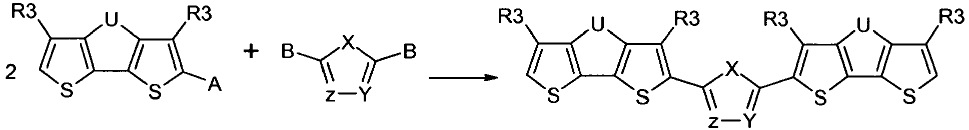 A und B sind jeweils Metall- oder Halogenkomponenten.

Die Einführung der endständigen Akzeptorgruppen kann beispielsweise erfolgen durch dem Fachmann bekannte Methoden, wie z.B. Gattermann, Gattermann-Koch, Houben-Hoesch, Vilsmeier/ Vilsmeier-Haack, Friedel-Crafts-Acylierung (Allg. Beschreibung in Organikum, Wiley-VCH), oder nach Lithiierung durch eine Umsetzung mit einem Säurederivat oder Carbonylierungsreagenz.

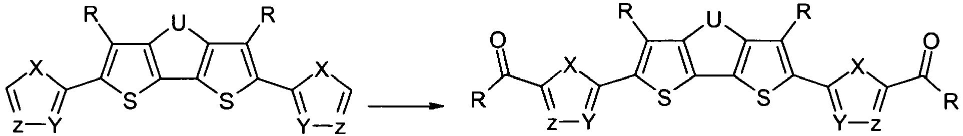

Weitere Akzeptorgruppen sind durch Umfunktionalisierung der zuvor beschriebenen Carbonylfunktion C(O)R beispielsweise durch Knoevenagel-Kondensation realisierbar(Allg. Beschreibung in Organikum, Wiley-VCH).

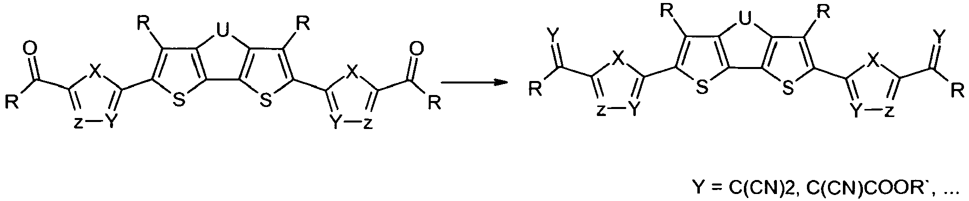

Im Fall von R1 = R6 = CN und W = C(CN)₂ kann die die Einführung der Akzeptorendgruppen beispielsweise mit BuLi und Tetracyanoethylen erfolgen (Cai et al, J. Phys. Chem. B 2006, 110, 14590):

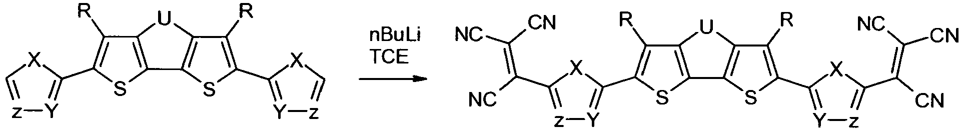

Alternativ kann die Umsetzung auch ohne BuLi in DMF durchgeführt werden (Pappenfus et al, Org. Lett. 2008, 10, 8, 1553)

Die Reihenfolge der Reaktionen kann variiert werden.

Weiterhin wird die Aufgabe durch ein optoelektronisches Bauelement gelöst.

In einer Ausführungsform der Erfindung ist ein optoelektronisches Bauelement mit einer Elektrode und einer Gegenelektrode und mindestens einer organischen lichtempfindlichen Schicht zwischen der Elektrode (2) und der Gegenelektrode angegeben, wobei die organische lichtempfindliche Schicht zumindest eine Verbindung gemäß Anspruch 1 und/oder 2 aufweist.

In einer weiteren Ausführungsform der Erfindung ist das optoelektronische Bauelement als organische Solarzelle ausgeführt.

In einer weiteren Ausführungsform der Erfindung ist das Bauelement als organische pin-Solarzelle bzw. organische pin-Tandemsolarzelle oder pin-Mehrfachsolarzelle ausgeführt. Als Tandemsolarzelle wird dabei eine Solarzelle bezeichnet, die aus einem vertikalen Stapel zweier in Serie verschalteter Solarzellen besteht. Als Mehrfachsolarzelle wird dabei eine Solarzelle bezeichnet, die aus einem vertikalen Stapel mehrerer in Serie verschalteter Solarzellen besteht, wobei maximal 10 Solarzellen in einem Stapel verschaltet sind.

In einer weiteren Ausführungsform der Erfindung sind in dem Bauelement noch eine oder mehrere undotierte, teilweise dotierte oder ganz dotierte Transportschichten vorhanden. Bevorzugt haben diese Transportschichten ein Maximum der Absorption bei < 450nm, besonders bevorzugt < 400nm.

In einer weiteren Ausführungsform der Erfindung sind die Schichten des Schichtsystems des Bauelements als eine den optischen Weg des einfallenden Lichts verlängernde Lichtfalle ausgebildet.

In einer weiteren Ausführungsform der Erfindung enthält mindestens eine der photoaktiven Mischschichten als Akzeptor ein Material aus der Gruppe der Fullerene bzw. Fullerenderivate (C₆₀, C₇₀, etc.).

In einer weiteren Ausführungsform der Erfindung bestehen die Kontakte aus Metall, einem leitfähigen Oxid, insbesondere ITO, ZnO:Al oder anderen TCOs oder einem leitfähigen Polymer, insbesondere PEDOT:PSS oder PANI.

In einer weiteren Ausführungsform ist zwischen der ersten elektronenleitenden Schicht (n-Schicht) und der auf dem Substrat befindlichen Elektrode noch eine p-dotierte Schicht vorhanden, so dass es sich um eine pnip oder pni-Struktur handelt, wobei vorzugsweise die Dotierung so hoch gewählt ist, dass der direkte pn-Kontakt keine sperrende Wirkung hat, sondern es zu verlustarmer Rekombination, bevorzugt durch einen Tunnelprozess kommt.

In einer weiteren Ausführungsform der oben beschriebenen Strukturen sind diese als organische Tandemsolarzelle oder Mehrfachsolarzelle ausgeführt. So kann es sich bei dem Bauelement um eine Tandemzelle aus einer Kombination aus nip, ni, ip, pnip, pni, pip, nipn, nin, ipn, pnipn, pnin oder pipn-Strukturen handeln, bei der mehrere unabhängige Kombinationen, die mindestens eine i-Schicht enthalten, übereinander gestapelt sind (Kreuzkombinationen).

In einer weiteren Ausführungsform der oben beschriebenen Strukturen ist diese als eine pnipnipn-Tandemzelle ausgeführt.

In einer Ausführungsform der Erfindung ist das Bauelement mit mindestens einer anorganischen Schicht beinhaltend eine oder mehrere anorganische Materialien aufgebaut.

In einer weiteren Ausführungsform der Erfindung wird das Bauelement auf ebenen, gekrümmten oder flexiblen Trägerflächen verwendet. Bevorzugt sind diese Trägerflächen Plastikfolien oder Metallfolien (z.B. Aluminium, Stahl), etc.

Nachfolgend soll die Erfindung anhand einiger Ausführungsbeispiele und dazugehöriger Figuren eingehender erläutert werden. Es zeigen die
Fig.1 ein Reaktionsschema zur Herstellung der Verbindung Ic und Id, in
Fig.2 eine schematische Darstellung eines Absorptionsspektrums der Verbindung Ic, in
Fig.3 eine schematische Darstellung einer Strom-Spannungs-Kurve einer Mip-Zelle mit einer Mischschicht von Verbindung 1c und in
Fig.4 die schematische Darstellung einer Struktur eines beispielhaften photoaktiven Bauelements.

Die Figuren 5A bis 9B zeigen die Absorptionsspektren weiterer erfindungsgemäßer Verbindungen sowie die Strom-Spannungs-Kurven der entsprechenden Mip-Zellen mit diesen Verbindungen.

In den aufgeführten Ausführungsbeispielen sind beispielhaft einige erfindungsgemäßen Bauelemente aufgezeigt. Zur Charakterisierung wichtige Kennzahlen sind der Füllfaktor, die Leerlaufspannung und Kurzschlussstrom aufgelistet, die aus der Strom-Spannungs-Kennlinie entnommen werden. Die Ausführungsbeispiele sollen die Erfindung beschreiben ohne sich auf diese zu beschränken.

### Ausführungsbeispiel 1:

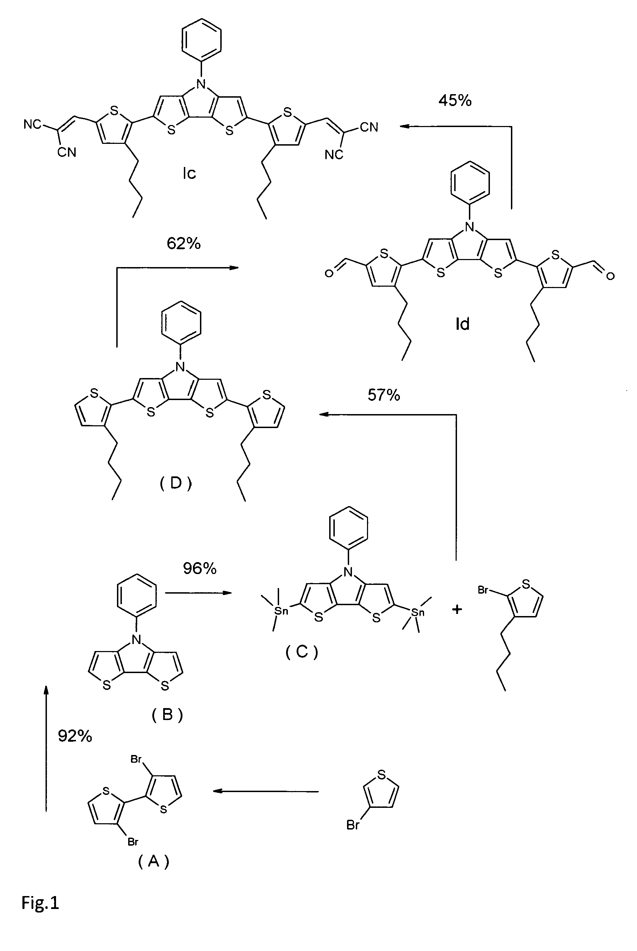

In einem ersten Ausführungsbeispiel ist in Fig. 1 schematisch die Darstellung der Verbindung Ic und Id gezeigt.

Nachfolgend sind die einzelnen Herstellungsschritte angegeben:

### Herstellung von Verbindung B:

In einem Autoklaven werden (A) (4.125 g, 12.75 mmol), t-BuONa (2.95 g, 30.7 mmol), Pd₂dba₃ (293 mg, 0.32 mmol), 2,2'-Bis(diphenylphosphino)-1,1'-binaphthyl) (793 mg, 1.27 mmol) vorgelegt, 25 ml Toluol zugegeben und das Reaktionsgemisch mit Argon belüftet. Phenylamin (752.5 mg, 12.725 mmol) wird zugegeben, und die Mischung 12 Sunden erhitzt (110°C). Nach dem Abkühlen werden 40 ml Wasser zugegeben. Die organische Phase wird abgetrennt, die wässrige mit Et₂O extrahiert. Die vereinigten organischen Phasen werden über Na₂SO₄ getrocknet und eingeengt. Der Rückstand wird chromatographisch gereinigt (Kieselgel, PE CH₂Cl₂ 10-1). Ausbeute: 2.59 g (92 %) von (B). NMR 1H: (CDCl₃) 7.16 (d, 2H), 7.03 (d, 2H), 4.04 (t, 2H), 1.93 (m, 2H), 0.97 (t, 3H)

### Herstellung von Verbindung C

In einem mit Ar belüfteten 250 ml 3-Halsrundkolben wird (B) (1g, 4.5 mmol) in 100 ml THF (abs.) gelöst und auf -78°C gekühlt. nBuLi (2.5 M in Hexan, 4.5 ml, 11.3 mmol) wird zugetropft, das Reaktionsgemisch wird ca.1 Stunde bei -78°C gehalten und dann langsam für 1h auf RT erwärmt, dann wieder auf -78°C gekühlt. Me3SnCl (1.0 M in Hexan, 11.3 ml, 11.3 mmol) wird zugegeben und nach ca. 1 Stunde wird die Reaktionsmischung aufgetaut. Nach 2 Stunden wird Wasser (100 ml) zugegeben, die wässrige Phase wird mit 50 ml Et2O extrahiert. Die vereinigten organischen Phasen werden getrocknet und eingeengt. Ausbeute 2.37 g (96%) von (C). NMR 1H: (CDCl3) 6.97 (s, 2H), 4.12 (m, 2H), 1.89 (m, 2H), 0.93 (m, 3H), 0.36 (s, 9H)

### Herstellung von Verbindung D

In einem Reaktionsgefäß werden unter Argon (C) (2.37 g, 4.3 mmol), 2-Brom-3-butyl-thiophen (2.3 g, 10.49 mmol) und 250 mg Pd[PPh3]4 vorgelegt, 30 ml Toluol zugegeben und 48 Stunden unter Rückfluss erhitzt. Das Reaktionsgemisch wird mit NH4Cl (gesät.) und Wasser gewaschen und eingeengt. Der Rückstand wird chromatographisch gereinigt (Kieselgel, PE CH2C12 10-1). Ausbeute: 1.217 g (57 %) von (D). NMR 1H: (CDC13) 7.21 (d, 2H), 7.04 (s, 2H), 6.97 (d, 2H), 4.20 (t, 2H), 2.85 (t, 4H), 1.96 (m, 2H), 1.67 (m, 4H), 1.44 (m, 4H), 0.98 (m, 6H), 0.88 (3H).

### Herstellung von Verbindung Id

In einem 100 ml Einhalsrundkolben werden DMF (2.27 ml, 29.3 mmol) und POCl3 (2.46 ml, 26.4 mmol) in 30 ml CH2Cl2 gelöst und 2 Stunden bei RT gerührt. Danach wird (D) (1.217 g, 2.44 mmol) in 50 ml CH2Cl2 zugetropft und 48 Stunden bei RT gerührt. 50 ml NaHCO3 (gesät.) Lösung werden dem Reaktionsgemisch zugegeben und 2 Stunden bei RT gerührt. Die organische Phase wird abgetrennt und 2x mit 50 ml Wasser gewaschen und getrocknet. Das Lösemittel wird abdestilliert, der Rückstand wird chromatographisch gereinigt (Kieselgel, CH2Cl2-MeOH 500-3). Ausbeute: 827 mg (62 %) von (Ib). NMR 1H: (CDCl3,) 9.85 (s, 2H), 7.63 (s, 2H), 7.23 (s, 2H), 4.25 (t, 2H), 2.91 (t, 4H), 1.98 (m, 2H), 1.72 (m, 4H), 1.49 (m, 4H), 0.98 (m, 9H)

### Herstellung von Verbindung Ic

In einem 250 ml Einhalsrundkolben werden (1d) (723 mg, 1.3 mmol) und Malonsäueredinitril (862 mg, 13 mmol) in 150 ml 1,2-DCE gelöst und mit Piperidin (11 mg, 0.13 mmol) versetzt. Nach 48h Erhitzen unter Rückfluss wird das Lösemittel entfernt und der Feststoff 2h unter Rückfluss mit Wasser digeriert. Der Niederschlag wird abgetrennt, mehrmals mit Wasser und dann mit MeOH gewaschen und getrocknet. Der Rückstand wird chromatographisch gereinigt (Kieselgel, CH2Cl2). Ausbeute: 370 mg (45 %) von (Ic). NMR 1H: (C2D2Cl4, 373 K) 7.73 (s, 2H), 7.63 (s, 2H), 7.36 (s, 2H), 4.27 (t, 2H), 2.96 (t, 4H), 2.05 (m, 2H), 1.79 (m, 4H), 1.53 (m, 4H), 1.07 (m, 9H)

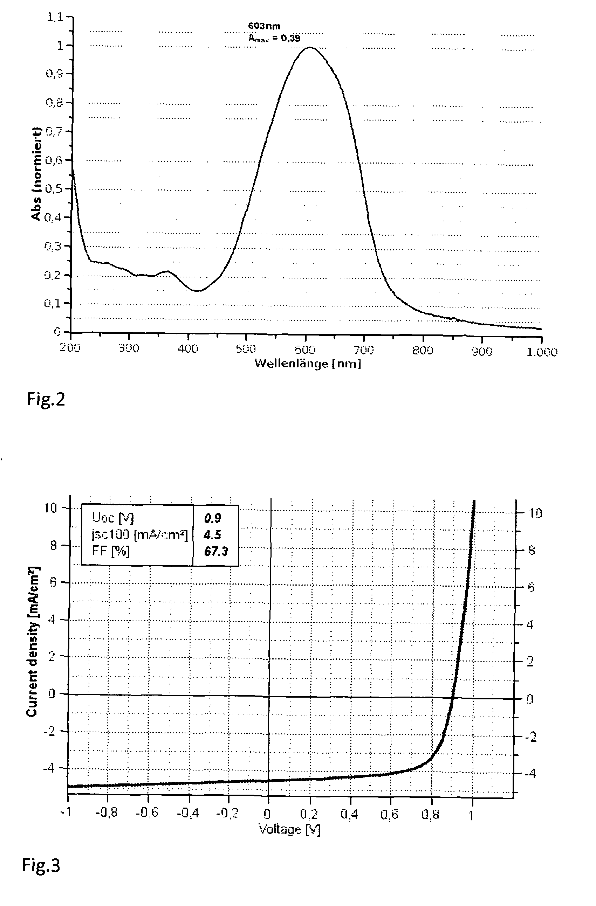

Durch Vakuumsublimation bei 10-6 bis 10-8 mbar wurde eine 30nm dünne Schicht hergestellt, ermittelt mittels Schwingquartzmonitor. Verbindung Ic lässt sich mit guter Ausbeute im Vakuum sublimieren. Das Absorptionsmaximum liegt, wie in Fig. 2 dargestellt, bei 603nm mit einer Absorption von 0,39.

In einem weiteren Ausführungsbeispiel wird ein MIP-Bauelement bestehend aus einer Probe auf Glas mit transparentem Deckkontakt ITO, einer Schicht Buckminster Fulleren C₆₀, Mischschicht von Verbindung 1c mit C₆₀ 2:1, einer p-dotierter Löchertransportschicht, einer Schicht Gold hergestellt. Fig.3 zeigt die Strom-Spannungs-Kurve dieser Mip-Zelle mit einer Mischschicht von Verbindung 1c mit C₆₀.

Die wichtigsten Kennzahlen wie der Füllfaktor FF, die Leerlaufspannung Uoc und der Kurzschlussstrom jsc zeigen eine gut funktionierende organische Solarzelle.

Die folgenden Verbindungen wurden bisher auf ähnliche Weise hergestellt:

| Nr | 1H-NMR | UV: λmax (Film) |
|---|---|---|
| Ia | (C2D2Cl4, 373 K) 7.73 (s, 2H), 7.63 (s, 2H), 7.36 (s, 2H), 4.27 (t, 2H), 2.96 (t, 4H), 2.05 (m, 2H), 1.79 (m, 4H), 1.53 (m, 4H), 1.07 (m, 9H) | 627 nm |
| Ib | (CDCl3) 9.85 (s, 2H), 7.63 (s, 2H), 7.23 (s, 2H), 4.25 (t, 2H), 2.91 (t, 4H), 1.98 (m, 2H), 1.72 (m, 4H), 1.49 (m, 4H), 0.98 (m, 9H) | |
| Ic | (C2D2Cl4, 373 K) 9.78 (s, 2H), 7.61 (s, 2H), 7.04 (s, 2H), 4.14 (t, 4H), 2.71 (t, 4H), 1.87 (m, 4H), 1.57 (m, 4H), 1.01 (t, 6H), 0.92 (t, 6H) | 603 nm |
| Id | (CDCl3) 9.85 (s, 2H), 7.63 (m, 7H), 7.37 (s, 2H), 2.91 (t, 4H), 1.73 (m, 4H), 1.46 (m, 4H), 1.00 (t, 6H) | |
| Ie | in TCE-d2 bei 100°C, ppm: 7,78 (s, 2H), 7,71 (d, 2H), 7,42 (s, 2H), 7,39 (d, 2H), 3,98 (s, 3H) | 638nm |
| If | in TCE-d2 bei 100 °C, ppm: 7,58 (s, 2H), 7,42 (s, 2H), 7,36 (d, 2H), 6,89 (d, 2H), 4,29 (t, 2H), 2,05 (qa, 2H), 1,07 (t, 3H) | 600nm |
| Ig | in TCE-d2 bei 100 °C, ppm: 7,77 (s, 2H), 7,71 (d, 2H), 7,40 (s, 2H), 7,39 (d, 2H), 4,28 (t, 3H), 2,00 (m, 2H), 1,43 (m, 6H), 0,97 (t, 3H) | 637nm |
| Ih | 1H-NMR (CDCl3, 293K): 0.98 ppm (m, 16H), 1.45 (m, 8H), 1.69 (m, 4H), 2.85 (m, 4H), 7.40 (s, 2H), 7.52 (s, 2H),7.70(s,2H). | 598 nm |
| Ii | 1H-NMR (CDCl3, 293K): 0.98 ppm (m, 16H), 1.46 (m, 8H), 1.70 (m, 4H), 2.84 (m, 4H), 7.28 (s, 2H), 7.60 (s, 2H),9.83(s,2H). | |
| Ij | 1H-NMR (CDCl3, 293K): 0.81 ppm (t, 6H), 0.98 (m, 4H), 1.05 (t, 6H), 1.10 (t, 6H), 1.58 (m, 4H), 1.61 (m, 4H), 1.93 (m, 4H), 2.70 (m, 4H), 2.80 (m, 4H), 7.30 (s, 2H), 7.81 (s, 2H). | 586 nm |
| Ik | 1H-NMR (CDCl3, 293K): 0.80 ppm (t, 6H), 1.05 (m, 10H), 1.07 (t, 6H), 1.63 (m, 4H), 1.68 (m, 4H), 1.87 (m, 4H), 2.75 (m, 4H), 2.89 (m, 4H), 7.17 (s, 2H), 10.00 (s, 2H). | |
| | | |
| | | |

| Nr | | |
|---|---|---|
| IIa | Unlöslich | 616 nm |
| IIb | (C2D2Cl4, 373 K) 9.68 (s, 2H), 7.69 (s, 2H), 7.22 (s, 2H), 4.23 (t, 4H), 1.97 (m, 4H), 1.02 (t, 6H) | |
| IIc | (C2D2Cl4, 373 K) 7.77 (s, 2H), 7.74 (s, 2H), 7.17 (s, 2H), 4.28 (t, 4H), 2.90 (t, 4H), 2.06 (m, 4H), 1.76 (m, 4H), 1.15 (t, 6H), 1.08 (t, 6H) | 597 nm |
| IId | (C2D2Cl4, 373 K) 9.78 (s, 2H), 7.61 (s, 2H), 7.04 (s, 2H), 4.14 (t, 4H), 2.71 (t, 4H), 1.87 (m, 4H), 1.57 (m, 4H), 1.01 (t, 6H), 0.92 (t, 6H) | |
| IIe | 1H-NMR (CDCl3, 293K): 0.81 ppm (t, 12H), 0.99 (m, 8H), 1.91 (m, 8H), 7.16 (s, 2H), 7.53 (s, 2H), 7.73 (s, 2H). | 601 nm |
| IIf | 1H-NMR (CDCl3, 293K): 0.80 ppm (t, 12H), 1.01 (m, 8H), 1.88 (m, 8H), 7.12 (s, 2H), 7.57 (s, 2H), 9.83 (s, 2H). | |

### Ausführungsbeispiel 2:

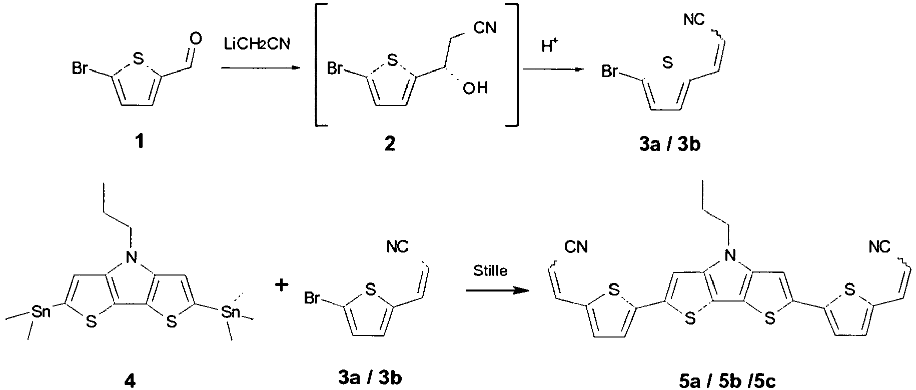

### Allgemeine Arbeitsvorschrift für Stille-Kupplung (AAV1)

Die Bisstannyl-Verbindung und 3 äquivalente der Brom-Verbindung werden in trockenem THF gelöst. Pd(PPh₃)₄ wird hinzugegeben und die Reaktionsmischung wird auf Rückflußtemperatur erhitzt. Nach dem Abkühlen auf Raumtemperatur wird filtriert und der Rückstand wird mit THF und Methanol gewaschen. Das Rohprodukt wird entweder mittels Chromatographie oder durch umkristallisieren gereinigt, um das Produkt sauber zu isolieren.

### Allgemeine Arbeitsvorschrift für Knoevenagel-Reaktion (AAV2)

Der Aldehyd und Malodinitril werden in Ethanol gelöst, man gibt Piperidine hinzu und erhitzt 2 h unter Rückfluß. Nach dem Abkühlen wird der Niederschlag abfiltriert und mit Ethanol und Hexan gewaschen. Das Rohprodukt wird umkristallisiert um das Produkt sauber zu isolieren.

### 4-Propyl-2,6-bistrimethylstannyl-4H-dithieno[3,2-b;2',3'-d]pyrrol (4)

Zu einer Lösung von 1,1 g (5,0 mmol) 4-Propyl-4*H*-dithieno[3, 2-b; 2',3'-d]pyrrol (**32**) in 17 ml trockenem THF werden 6,9 ml (11,0 mmol) einer 1,6 M n-Butyllithium-hexane-Lösung bei -78°C hinzugegeben. Nachdem die Mischung für 1 h bei -78°C gerührt wurde, wird das Kühlbad entfernt und eine weitere Stunde bei Raumtemperatur gerührt. Ein Niederschlag entsteht während dieser Zeit. Die Suspension wird -78°C gekühlt und eine Lösung von 2,2 g (11,0 mmol) Trimethylstannylchlorid gelöst in 3 ml THF wird hinzugegeben. Man rührt die Mischung für 1 h -78°C und entfernt anschließend das Kühlbad, um weitere 3 h bei Raumtemperatur zu rühren. 50 ml n-Hexan werden hinzugegeben und man hydrolisiert mit Wasser. Die organische Phase wird dreimal mit 50 ml Wasser gewaschen und über Natriumsulfat getrocknet. Nach dem Abdestillieren der Lösemittel wird der Rückstand im Vakuum getrocknet und das Rohprodukt (2,26g, 88%) wird in der nächsten Stufe ohne weitere Reinigung eingesetzt.. ¹H-NMR (CDCl₃) : 7,01 ppm (s, 2H), 4,16 (t, 2H), 1,94 (qa, 2H), 0,98 (t, 3H), 0,40 (s, 18H) .

### 3-(5-Brom-2-thienyl)prop-2-ennitril (3a/b)¹

Zu einer Lösung von 6,0 ml (10,8 mmol) 1,8 M LDA-THF-Lösung in 10 ml THF werden 0,56 ml (10,8 mmol) Acetonitril bei - 78°C getropft. Die Mischung wird 30 min gerührt. Man gibt 1,91 g (10,0 mmol) 5-Bromthiophen-2-carbaldehyd (**1**) hinzu und rührt 1 h bei -78°C. Die Reaktionsmischung wird auf-20°C erwärmt und gesättigte NH₄Cl-Lösung (2 ml) wird hinzugegeben. Man trennt die Phasen und extrahiert die wässrige Phase mit Ether (30 ml). Die vereinigten organischen Phasen werden über Natriumsulfat getrocknet. Man destilliert die Lösemittel ab und erhält ein braunes Öl. Das braune Öl wird in 5 ml Essigsäureanhydrid und 0,1 ml konz. Phosphorsäure gelöst. Diese Reaktionsmischung wird für 2 h auf 100°C erhitzt. 30 ml Wasser werden hinzugegeben und die wässrige Phase wird mit 50 ml Ether extrahiert. Die organische Phase wird portionsweise mit Natriumhydrogencarbonat-Lösung bis zur Neutralität gewaschen und über Ntriumsulfat getrocknet. Nach dem Abdestillieren der Lösemittel wird der Rückstand mittels Chromatographie gereinigt (SiO₂, DCM : Hexan (1:1)). Man erhält 0,78 g (36%) Produkt als E/Z-Isomere. ¹H-NMR (CDCl₃) E-Isomer: 7,25 ppm (d, 1H), 7,11 (d, 1H), 7,08 (d, 1H), 5,26 (d, 1H). Z-Isomer: 7, 33 (d, 1H), 7,04 (d, 1H), 6,98 (d, 1H), 5,54 (d, 1H).

### 3-(5-{6-[5-(2-cyano-vinyl)-thiophen-2-yl]-4-propyl-4H-dithieno[3,2-b;2',3'-d]pyrrol-2-yl}-thiophen-2-yl)prop-2-ennitril (5a/b/c)

Nach AAV1, 3-(5-Brom-2-thienyl)prop-2-ennitril (3a/b) (578 mg, 2,70 mmol) und 4-Propyl-2,6-bistrimethylstannyl-4*H-*dithieno[3,2-b;2',3'-d]pyrrol (**4**) (490 mg, 0,90 mmol) werden in 5 ml THF gelöst, Pd(PPh₃)₄ (104 mg, 0,09 mmol) wird hinzugegeben und die Mischung wird für 16 h auf 70°C erhitzt. Nach der Aufarbeitung wird das Rohprodukt mittels Chromatographie gereinigt (SiO₂, DCM). Man erhält drei Produkte (60 mg (14%) Z,Z-Isomer, 60 mg (14%) E,E-Isomer and 100 mg (23%) E,Z-Iisomer) in einer Gesamtausbeute von 50%. ¹H-NMR (TCE-d2, 375 K) Z,Z-Isomer: 7,36 ppm (d, 2H), 7,18 (s, 2H), 7, 14 (d, 2H), 7, 11 (d, 2H), 5,16 (d, 2H), 4,12 (dd, 2H), 1,93 (qa, 2H), 0,98 (t, 3H). UV (Film): 500 nm. E,E-Isomer: 7,35 (d, 2H), 7,11 (s, 2H), 7,10 (d, 4H), 5,54 (d, 2H), 4,11 (dd, 2H), 2,92 (qa, 2H), 0,97 (t, 3H). UV (Film): 521 nm. E,Z-Isomer: 7,35 (m, 2H), 7,18 (s, 1H), 7,11 (m, 5H), 5,53 (d, 1H), 5,17 (d, 1H), 4,11 (dd, 2H), 1,92 (qa, 2H), 0,98 (t, 3H). UV (Film) : 514 nm.

### Ausführungsbeispiel 3:

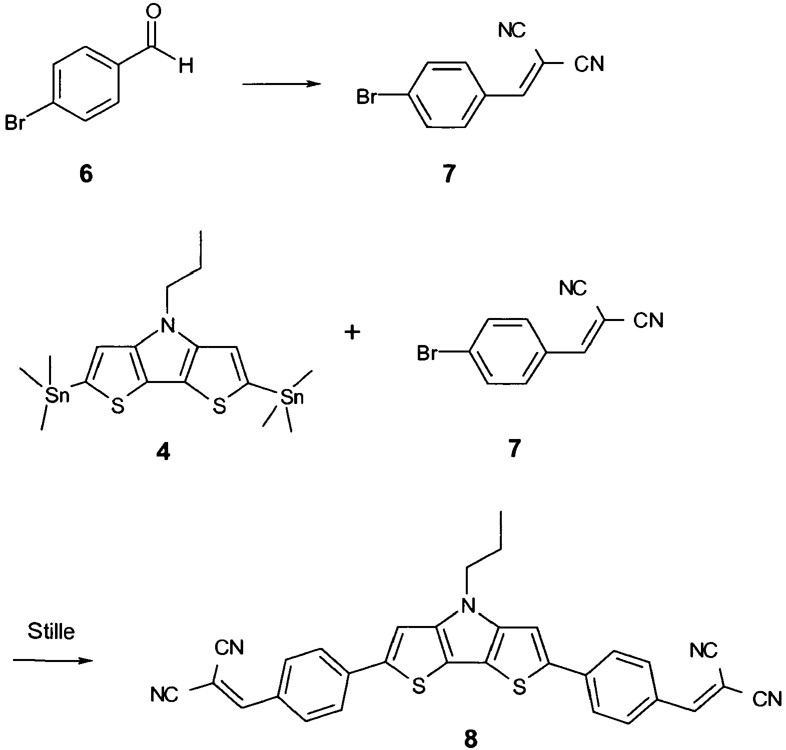

### 2-[(4-Bromphenyl)methylen]propandinitril (7)

Nach AAV2, 4-Brom-benzaldehyd (**6**) (1,0 g, 5,40 mmol) und Malodinitril (429 mg, 6,48 mmol) werden in 15 ml Ethanol gelöst, Piperidine (50 µl, 0,05 mmol) wird hinzugegeben und die Mischung wird für 2 h erhitzt. Nach der Aufarbeitung wird das Rohprodukt aus Ethanol umkristallisiert und man erhält 775 mg (62%) Produkt. ¹H-NMR (CDCl₃): 7,77 ppm (d, 2H), 7,72 (s, 1H), 7,69 (d, 2H).

### Dicyanovinyl-Verbindung 8

Nach AAV1, 2-[(4-bromphenyl)methylen]propandinitril (7) (699 mg, 3,00 mmol) und 4-Propyl-2,6-bistrimethylstannyl-4*H-*dithieno[3,2-b;2',3'-d]pyrrol (**4**) (547 mg, 1,00 mmol) werden in 3,8 ml THF gelöst, Pd(PPh₃)₄ (59 mg, 0,05 mmol) wird hinzugegeben und die Mischung wird für 36 h auf 70°C erhitzt. Nach der Aufarbeitung wird das Rohprodukt aus Chlorbenzol umkristallisiert und man erhält 120 mg (23%) Produkt. ¹H-NMR (TCE-d2, 375 K): 7,98 ppm (d, 4H), 7,85 (d, 4H), 7,76 (s, 2H), 7, 49 (s, 2H), 4,29 (dd, 2H), 2,07 (qa, 2H), 1,11 (t, 3H). UV (Film): 526 nm.

### Ausführungsbeispiel 4:

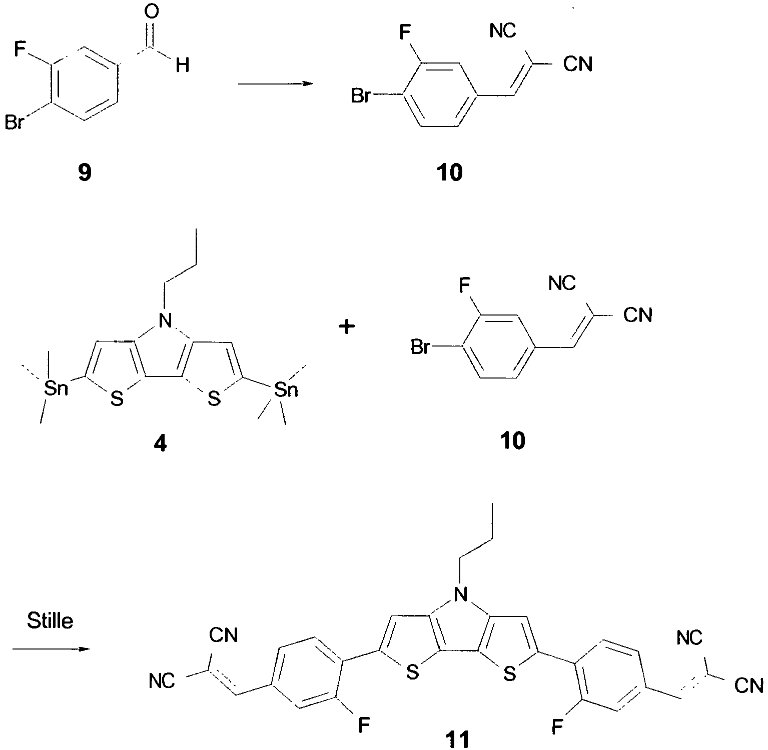

### 2-[(4-Brom-3-fluor-phenyl)methylen]propandinitril (10)

Nach AAV2, 4-Brom-3-fluorbenzaldehyd (**9**) (820 mg, 4,04 mmol) und Malodinitril (320 mg, 4,85 mmol) werden in 15 ml Ethanol gelöst, Piperidin (40 µl, 0,04 mmol) wird hinzugegeben und die Mischung wird für 2 h erhitzt. Nach der Aufarbeitung wird das Rohprodukt aus Ethanol umkristallisiert und man erhält 847 mg (84%) Produkt. ¹H-NMR (CDCl₃) : 7,75 ppm (dd, 1H), 7,70 (s, 1H), 7,68 (dd, 1H), 7,56 (dd, 1H).

### Dicyanovinyl-Verbindung 11

Nach AAV1, 2-[(4-Brom-3-fluor-phenyl)methylen]propandinitril (**10**) (840 mg, 3,35 mmol) and 4-Propyl-2,6-bistrimethylstannyl-4H-dithieno[3,2-b;2',3'-d]pyrrol (**4**) (610 mg, 1,12 mmol) werden in 4,5 ml THF gelöst, Pd(PPh₃)₄ (127 mg, 0,11 mmol) wird hinzugegeben und die Mischung wird für 20 h auf 70°C erhitzt. Nach der Aufarbeitung wird das Rohprodukt aus Chlorbenzol umkristallisiert und man erhält 20 mg (3%) Produkt. ¹H-NMR (TCE-d2, 375 K): 7,78 ppm (dd, 2H), 7,69 (d, 2H), 7,65 (d, 2H), 7,61 (s, 2H), 7,59 (s, 2H), 4,21 (dd, 2H), 1,96 (qa, 2H), 0,99 (t, 3H).

### Ausführungsbeispiel 5:

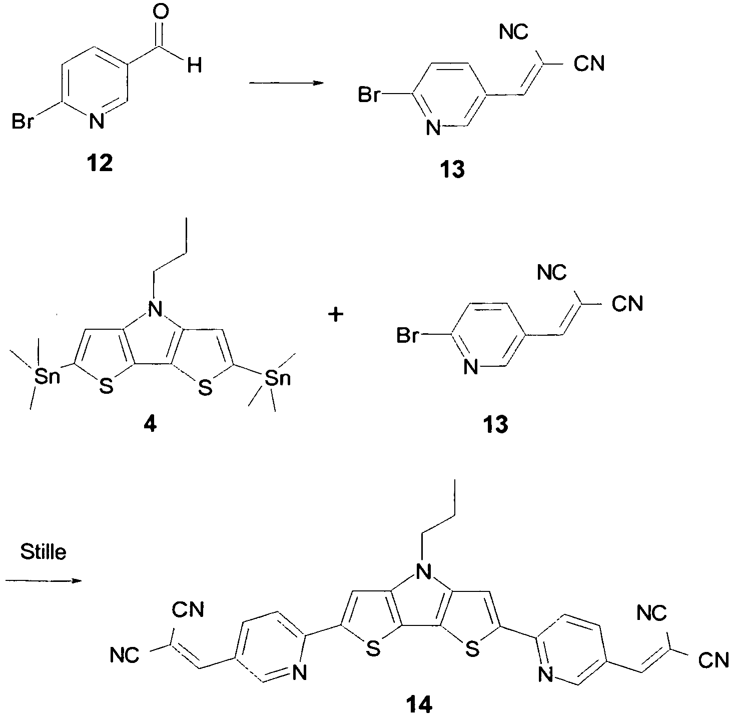

### 2-[(6-Brom-3-pyridyl)methylen]propandinitril (13)

Nach AAV2, 6-Brompyridin-3-carbaldehyd (**12**) (1,0 g, 5,40 mmol) and malodinitril (429 mg, 6,48 mmol) werden in 15 ml Ethanol gelöst, Piperidin (50 µl, 0,05 mmol) wird hinzugegeben und die Mischung wird für 2 h erhitzt. Nach der Aufarbeitung wird das Rohprodukt aus Ethanol umkristallisiert und man erhält 690 mg (55%) Produkt. ¹H-NMR (CDCl₃): 8, 63 ppm (d, 1H), 8,31(dd, 1H), 7, 7 6 (s, 1H), 7,71 (d, 1H).

### Dicyanovinyl-Verbindung 14

Nach AAV1, 2-[(6-Brom-3-pyridyl)methylen]propandinitril (13) (690 mg, 2,95 mmol) and 4-Propyl-2,6-bistrimethylstannyl-4*H-*dithieno[3,2-b;2',3'-d]pyrrol (4) (547 mg, 1,00 mmol) werden in 4,0 ml THF gelöst, Pd(PPh₃)₄ (69 mg, 0,06 mmol) wird hinzugegeben und die Mischung wird für 36 h auf 70°C erhitzt. Nach der Aufarbeitung wird das Rohprodukt aus Chlorbenzol umkristallisiert und man erhält 320 mg (61%) Produkt. MALDI (m/z): 527,1. UV (Film): 581 nm.

### Ausführungsbeispiel 6:

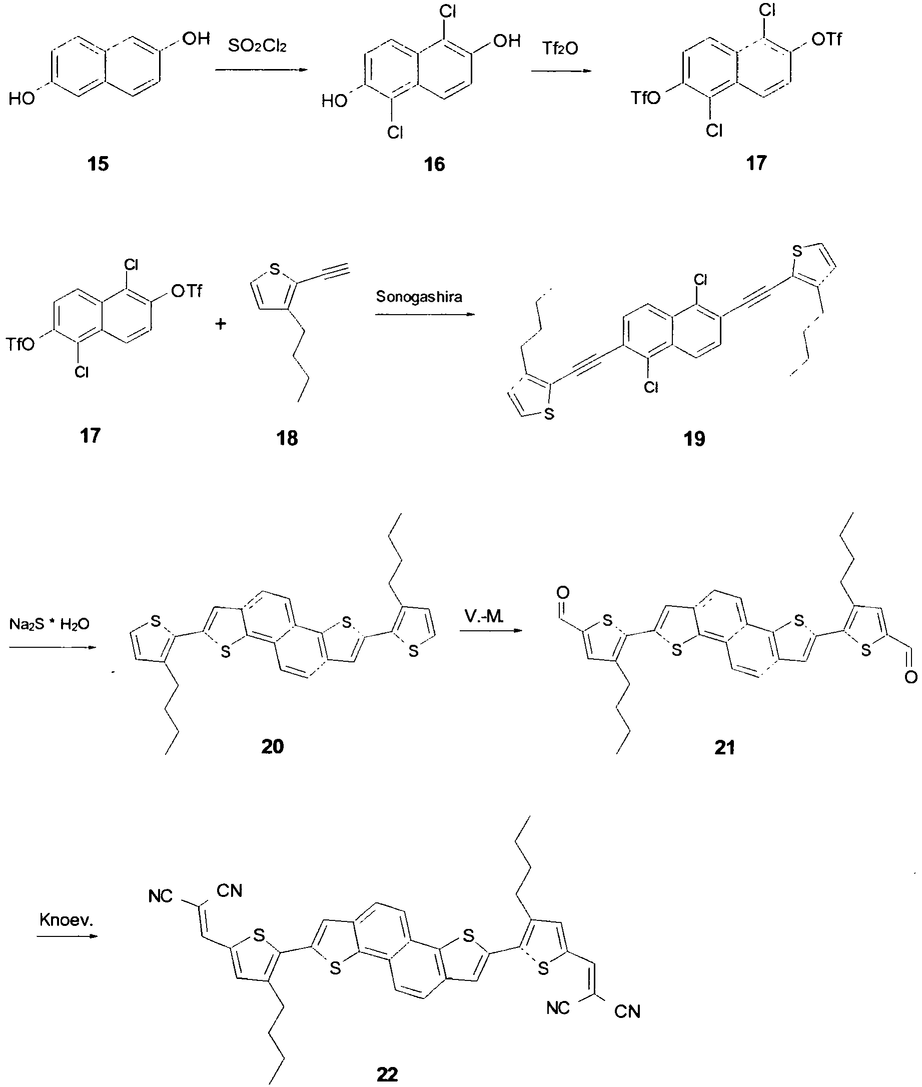

### 3-Butyl-2-[2-[6-[2-(3-butyl-2-thienyl)ethinyl]-1,5-dichlor-2-naphthyl]ethinyl]thiophen (19)²

1,46 g (2,96 mmol) [1,5-Dichlor-6-(trifluormethylsulfonyloxy)-2-naphthyl] trifluormethanesulfonat (**17**) und 1,46 g (8,89 mmol) 3-Butyl-2-ethinyl-thiophen (**18**) werden in 1,25 ml trockenem Triethylamin und 30 ml trockenem THF gelöst. Zu der Reaktionsmischung gibt man 208 mg (0,30 mmol) Pd(PPh₃)₂Cl₂ und 113 mg (0,59 mmol) Kupfer(I)-Iodid. Die Mischung wird 20 h unter Rückfluß erhitzt. Man gibt 3 ml Wasser und 3 ml 1N HCl-Lösung hinzu, die Phasen werden getrennt und die wässrige Phase wird dreimal mit 25 ml DCM extrahiert. Die vereinigten organischen Phasen werden über Natriumsulfat getrocknet. Nach dem Abdestillieren der Lösemittel wird der Rückstand aus Hexan / Chloroform umkristallisiert und man erhält 505 mg (33%) Produkt. ¹H-NMR (CDCl₃): 8,22 ppm (d, 2H), 7,68 (d, 2H), 7,27 (d, 2H), 6,94 (d, 2H), 2,88 (dd, 4H), 1,70 (m, 4H), 1,42 (qa, 4H), 0,96 (t, 6H).

### 2,7-Bis(3-butyl-2-thienyl)benzthiopheno[7,6-g]benzthiophen (20)

Zu einer Suspension von 932 mg (3,88 mmol) Natriumsulfidnona-hydrat in 23 ml NMP werden 505 mg (0,97 mmol) von naphthylethin **19** gegeben. Die Reaktionsmischung wird für 16 h unter Rückfluß erhitzt. Nach dem Abkühlen auf Raumtemperatur werden 76 ml Ammoniumchlorid-Lösung hinzugegeben und man extrahiert dreimal mit 30 ml Chloroform. The organics were combined and dried over anhydrous sodium sulphate. Die vereinigten organischen Phasen werden über Natriumsulfat getrocknet. Nach dem Abdestillieren der Lösemittel wird der Rückstand mittels Chromatographie (SiO₂, n-hexane) gereinigt und man erhält 300 mg (60%) Produkt. ¹H-NMR (CDCl₃) : 8, 01 ppm (d, 2H), 7,88 (d, 2H), 7, 47 (s, 2H), 7, 27 (d, 2H), 7,01 (d, 2H), 2, 91 (dd, 4H), 1,71 (m, 4H), 1,45 (qa, 4H), 0,96 (t, 6H).

### 4-Butyl-5-[2-(3-butyl-5-formyl-2-thienyl)benzthiopheno[7,6-g]benzthiophen-7-yl]thiophen-2-carbaldehyd (21)

Zu einer Lösung von Benzthioheno-benzthiophen **20** (300 mg, 0,58 mmol) in 7 ml Dichlormethan wird eine Lösung von 1,1 ml (11,4 mmol) Phosphoroxychlorid and 0, 94 ml (12,2 mmol) DMF in 7 ml DCM gegeben. Die Reaktionsmischung wird 16 h refluxiert, anschließend gibt man ges. Natriumhydrogencarbonat-Lösung (50 ml) hinzu und rührt 2 h bei Raumtemperatur. Nach Phasentrennung wird die organische Phase über Natriumsulfat getrocknet. Nach dem Abdestillieren der Lösemittel wird der Rückstand aus Toluol umkristallisiert und man erhält 172 mg (52%) Produkt. ¹H-NMR (TCE-d2, 375 K): 9,83 ppm (s, 2H), 8,01 (d, 2H), 7,90 (s, 2H), 7,61 (s, 4H), 2,92 (dd, 4H), 1,72 (m, 4H), 1,44 (qa, 4H), 0,95 (t, 6H).

### 2-[[4-Butyl-5-[7-[3-butyl-5-(2,2-dicyanovinyl)-2-thienyl]benzthiopheno[7,6-g]benzthiophen-2-yl]-2-thienyl]methylen]propandinitril (22)

Eine Lösung von Dialdehyd **21** (167 mg, 0,29 mmol), Malodinitril (214 mg, 3,24 mmol) und Piperidin (4 mg 0,04 mmol) in 50 ml 1,2-Dichlorethan wird für 48 h refluxiert. Nach dem Abdestillieren des Lösemittels wird der Rückstand in Wasser suspendiert und für 2 h refluxiert. Das Rohprodukt wird filtriert, gewaschen mit Wasser und Methanol und im Vakuum getrocknet. Der Rückstand wird aus Chlorbenzol umkristallisiert und man erhält 158 mg (81 %) Produkt. ¹H-NMR (TCE-d2, 375 K): 8,03 ppm (d, 2H), 7,92 (d, 2H), 7,69 (s, 2H), 7,68 (s, 2H), 7,61 (s, 2H), 2,94 (dd, 4H), 1,72 (m, 4H), 1,45 (qa, 4H), 0,96 (t, 6H).

### Ausführungsbeispiel 7:

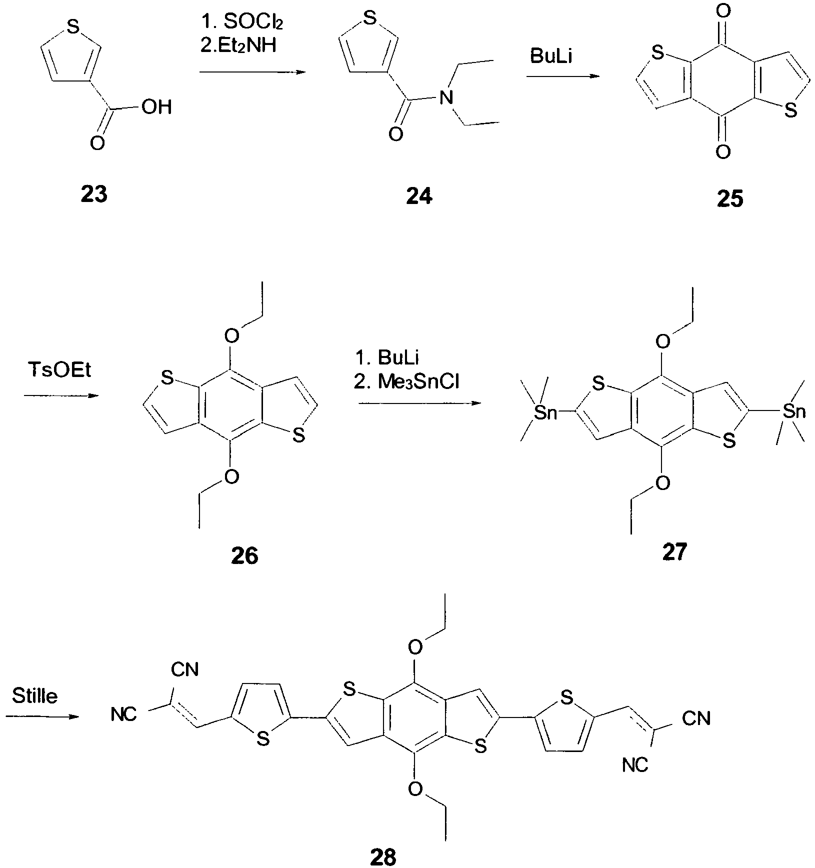

### (4,8-Diethoxy-2-trimethylstannyl-thieno[2,3-f]benzthiophen-6-yl)-trimethyl-stannan (27)³

Zu einer Lösung von 556 mg (2,0 mmol) 4,8-Diethoxythieno[2,3-f]benzthiophen (**26**) in 8 ml trockenem THF werden 1,6 ml (4,1 mmol) 2,5 M n-Butyllithium-Hexan-Lösung bei -78°C getropft. Nachdem die Mischung 1h bei -78°C gerührt wurde, wird das Kühlbad entfernt und für eine weitere Stunde bei Raumtemperatur gerührt. Es wird auf -78°C gekühlt und man gibt eine Lösung von 4,2 ml (4,2 mmol) 1,0 M Trimethylstannylchlorid in n-Hexane hinzu. Die Reaktionsmischung wird noch 10 min bei -78°C gerührt und anschließend 16 h bei Raumtemperatur. 20 ml Ether und 20 ml Wasser werden hinzugegeben und die organische Phase wird separiert. Die wässrige Phase wird extrahiert mit 20 ml Ether, man vereinigt die organischen Phasen und trocknet über Natriumsulfat. Nach dem Abdestillieren der Lösemittel wird der Rückstand im Vakuum getrocknet. Das Rohprodukt (1,13 g, 94%) wird ohne weitere Reinigung in der nächsten Stufe eingesetzt. ¹H-NMR (CDCl₃) : 7,51 ppm (s, 2H), 4,38 (qa, 4H), 1,50 (t, 6H), 0,45 (s, 18H).

### Dicyanovinyl-Verbindung 28

Nach AAV1, 2-[(5-Brom-2-thienyl)methylen]propandinitril (621 mg, 2,6 mmol) and (4,8-Diethoxy-2-trimethylstannyl-thieno[2,3-f]benzthiophen-6-yl)-trimethyl-stannan (**27**) (604 mg, 1,0 mmol) werden in 4 ml THF gelöst, Pd(PPh₃)₄ (59 mg, 0,05 mmol) wird hinzugegeben und die Mischung wird für 16 h auf 70°C erhitzt. Nach der Aufarbeitung wird das Rohprodukt aus Chlorbenzol umkristallisiert und man erhält 290 mg (49%) Produkt. ¹H-NMR (TCE-d2, 375 K): 7,84 ppm (s, 2H), 7,83 (s, 2H), 7,78 (d, 2H), 7,53 (d, 2H), 4,53 (qa, 4H), 1,62 (t, 6H). UV (Film): 589 nm (max), 466 nm.

### Ausführungsbeispiel 8:

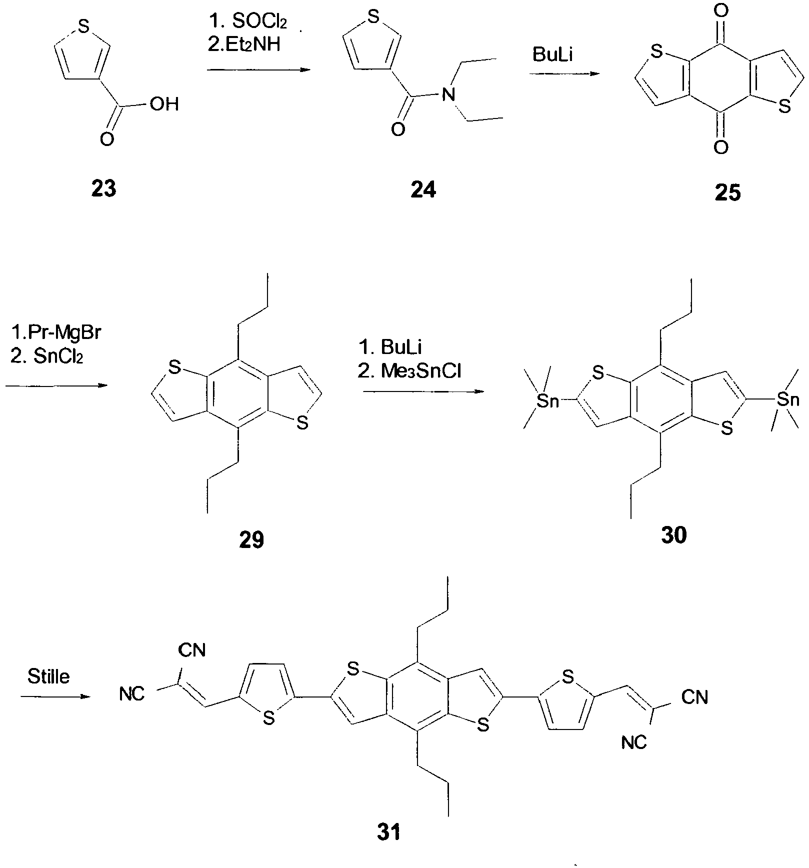

### (4,8-Dipropyl-2-trimethylstannyl-thieno[2,3-f]benzthiophen-6-yl)-trimethyl-stannan (30)⁴

Zu einer Lösung von 400 mg (1,47 mmol) 4,8-Dipropylthieno[2,3-f]benzthiophen (29) in 6 ml THF werden 1,2 ml (3,02 mmol) 2,5 M n-Butyllithium-Hexan-Lösung bei-78°C getropft. Nachdem die Mischung 1h bei -78°C gerührt wurde, wird das Kühlbad entfernt und für eine weitere Stunde bei Raumtemperatur gerührt. Es wird auf -78°C gekühlt und man gibt eine Lösung von 3,2 ml (3,2 mmol) 1,0 M Trimethylstannylchlorid in n-Hexane hinzu. Die Reaktionsmischung wird noch 10 min bei -78°C gerührt und anschließend 16 h bei Raumtemperatur. 20 ml Ether und 20 ml Wasser werden hinzugegeben und die organische Phase wird separiert. Die wässrige Phase wird extrahiert mit 20 ml Ether, man vereinigt die organischen Phasen und trocknet über Natriumsulfat. Nach dem Abdestillieren der Lösemittel wird der Rückstand im Vakuum getrocknet. Das Rohprodukt (890 mg, 100%) wird ohne weitere Reinigung in der nächsten Stufe eingesetzt. ¹H-NMR (CDCl₃): 7,50 ppm (s, 2H), 3,21 (dd, 4H), 1,88 (qa, 4H), 1,07 (t, 6H), 0,45 (s, 18H).

### Dicyanovinyl-Verbindung 31

Nach AAV1, 2-[(5-Brom-2-thienyl)methylen]propandinitril (538 mg, 2,25 mmol) und (4,8-Diethoxy-2-trimethylstannyl-thieno[2,3-f]benzthiophen-6-yl)-trimethyl-stannan (**30**) (450 mg, 0,75 mmol) werden in 5 ml THF gelöst, Pd(PPh₃)₄ (43 mg, 0,04 mmol) wird hinzugegeben und die Mischung wird für 16 h auf 70°C erhitzt. Nach der Aufarbeitung wird das Rohprodukt aus Chlorbenzol umkristallisiert und man erhält 301 mg (68%) Produkt. ¹H-NMR (TCE-d2, 375K): 7, 73 ppm (s, 2H), 7,72 (s, 2H), 7,67 (d, 2H), 7,44 (d, 2H), 3,14 (dd, 4H), 1,89 (qa, 4H), 1,07 (t, 6H). UV (Film): 586 nm (max), 545 nm, 481 nm.

### Ausführungsbeispiel 9:

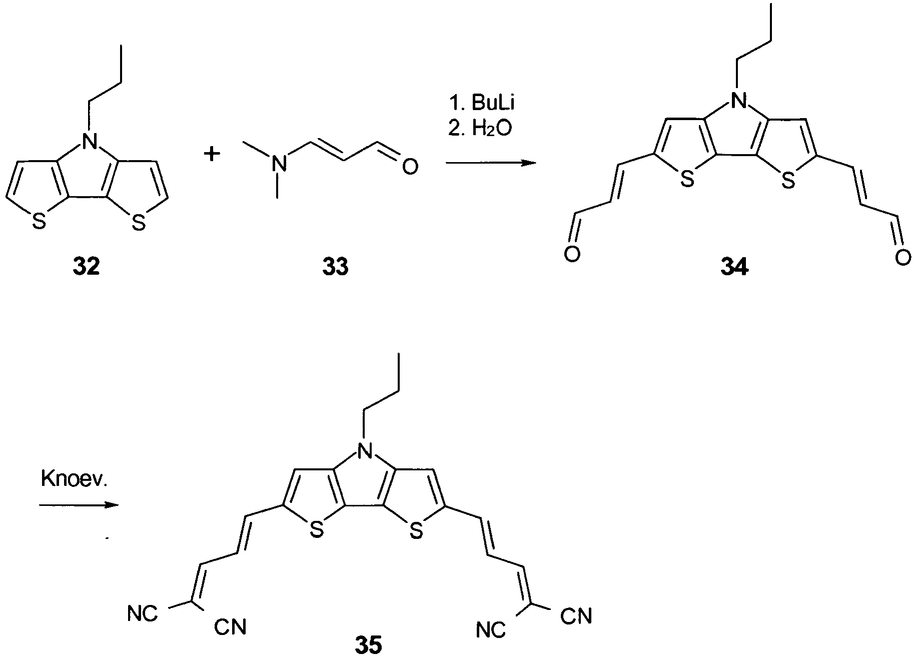

### Diacrolein-dithienopyrrol 34⁵

Zu einer Lösung von 490 mg (2,22 mmol) 4-Propyl-4*H-*dithieno[3, 2-b; 2',3'-d]pyrrol (**32**) in 9 ml THF werden 1,9 ml (4,66 mmol) 2,5 M n-Butyllithium-Hexan-Lösung bei -78°C getropft. Es wird für 5 min bei -78°C gerührt, anschließend rührt man 1 h bei -10°C. 770 mg (7,77 mmol) 3-Dimethylaminoacrolein (**33**) werden zu der Mischung hinzugegeben und es wird bei -5°C für 30 min gerührt. Die Kühlung wird entfernt und es wird für weitere 4 h bei Raumtemperatur gerührt. 20 ml ges. NH₄Cl-Lösung werden hinzugegeben und die Reaktionsmischung wird für 1 h gerührt. Man gibt 100 ml DCM hinzu, die organische Phase wird separiert und über Natriumsulfat getrocknet. Nach dem Abdestillieren der Lösemittel wird der Rückstand chromatographisch (SiO₂, DCM : Ethylacetat (10:1)) gereinigt und man erhält 152 mg (21%) Produkt. ¹H-NMR (CDCl₃): 9,64 ppm (d, 2H), 7,58 (d, 2H), 7,24 (s, 2H), 6,54 (dd, 2H), 5,14 (dd, 2H), 1,93 (qa, 2H), 0,97 (t, 3H).

### Dicyanovinyl-Verbindung 35

Eine Lösung von Dialdehyd **34** (150 mg, 0,46 mmol), Malodinitril (241 mg, 3,65 mmol) und Piperidin (4 mg 0,04 mmol) in 40 ml 1,2-Dichlorethan wird für 5 Tage refluxiert. Nach dem Abdestillieren des Lösemittels wird der Rückstand in Wasser suspendiert und für 2 h refluxiert. Das Rohprodukt wird filtriert, gewaschen mit Wasser und Methanol und im Vakuum getrocknet. Der Rückstand wird aus Chlorbenzol umkristallisiert und man erhält 77 mg (39 %) Produkt. ¹H-NMR (TCE-d2, 375 K): 7,42 ppm (d, 2H), 7,32 (d, 2H), 7,23 (s, 2H), 7,01 (dd, 2H), 4,11 (dd, 2H), 1,90 (qa, 2H), 0,96 (t, 3H). UV (Film): 611 nm (max), 663 nm.

### Literatur

1. T. J. Dietsche, D. B. Gorman, J. A. Orvik, G. A. Roth, W. R. Shiang, Organic Process Research & Development 2000, 4, 4, 275-285.
2. S. Shinamura, E. Miyazaki,K. Takimiya, J. Org. Chem. 2010, 75, 4, 1228-1234.
3. J. Hou, M.-H. Park, S. Zhang, Y. Yao, L.-M. Chen, J.-H. Li, Y. Yang, Macromolecules 2008, 41, 16, 6012-6018.
4. A. K. Mishra, S. Vaidyanathan, H. Noguchi, F. Dötz, S. Köhler, M. Kastler, PCT Int. Appl. (2009), 8 pp. WO 2010079064.
5. P. Frère, J.-M. Raimundo, P. Blanchard, J. Delaunay, P. Richomme, J.-L. Sauvajol, J. Orduna, J. Garin, J. Roncali, J. Org. Chem. 2003, 68, 19, 7254-7265.

### Ausführungsbeispiel 10:

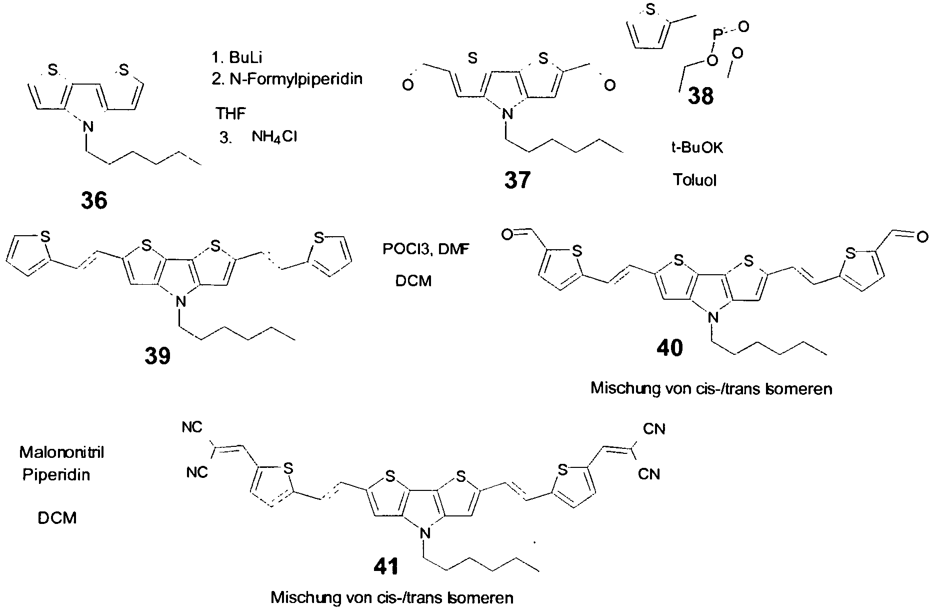

### 4-Hexyl-4H-dithieno[3,2-b;2',3'-d]pyrrol-2,6-dicarbaldehyd (37) :

Zu einer Lösung von 4-Hexyl-4H-dithieno[3,2-b;2',3'-d]pyrrol (**36**) (1,100 g, 4,18 mmol) in 126 ml trockenem THF wurde bei -78°C unter Argon über einen Zeitraum von 20 Minuten n-BuLi (1,6 M in Hexanes; 5,5 ml, 8.79 mmol) zugegeben. Nach der Zugabe wurde das Gemisch für 90 Minuten bei -78 °C weiter gerührt und danach auf Raumtemperatur erwärmt. Nach weiterem Rühren für 1 Stunde bei RT weiter wurde wieder auf -78 °C abgekühlt und N-Formylpiperidin (1,045 g, 9,24 mmol) schnell über eine Spritze zugegeben. Das Gemisch wurde für 30

Minuten bei -78 °C weiter gerührt und dann innerhalb von 4 Stunden auf RT gebracht. Es wurde über Nacht weiter unter Argon gerührt. Die Reaktion wurde durch Zugabe einer gesättigten NH₄Cl-Lösung (47 ml) abgebrochen. Nach fortgesetztem Rühren für 15 Minuten bei RT wurde in einen Scheidetrichter überführt und mit Dichlormethan ( 3 x 80 ml) extrahiert. Die vereinigten organischen Phasen wurden mit Wasser (2 x 30ml) gewaschen und über Na₂SO₄ getrocknet. Nach Filtration und Entfernung des Lösungsmittels wurde das Rohprodukt mittels Flashchromatographie (Silicagel, DCM als Eluent, R_{f} = 0.25) gereinigt. Nach Vereinigung der Produktfraktionen und Entfernen des Lösungsmittels wurde ein gelber Feststoff isoliert (533 mg, 1,67 mmol; 40%). ¹H-NMR (CDCl₃; δ = 9.88 (s, 2H, CHO), 7.63 (s, 2H, Th-H), 4.21 (t, 2H, CH₂), 1.85 (m, 2H, CH₂), 1.12 (m, 6H, 3xCH₂), 0.80 (t, 3H, CH₃).

### 4-Hexyl-2,6-bis(E)-2-thiophen-2-yl-vinyl)-4H-dithieno[3,2-b;2',3'-d]pyrrol (39) :

Dialdehyd (**37**) (0,320 g, 1,00 mmol) und Diethyl(2-thienylmethyl)phosphonat (**38**) (1,000g, 4,27 mmol) (bezogen von metina (Scweden)) wurden in 100 ml wasserfreiem und entgastem Toluol gelöst. Nachdem die Mischung auf 110°C erwärmt worden ist, wurde t-BuOK (0,900 g, 8,00 mmol) in 4 Portionen (jede 0,250 g) innerhalb von 15 Minuten unter fortgesetztem Rühren und Argon zugegeben. Die Mischung wurde für weitere 10 Stunden unter Rühren refluxiert. Nach dem Abkühlen auf RT wurde die Reaktion durch Zugabe von 50 ml 0.2 M HCl (10 mmol) und fortgesetztem Rühren für 15 Minuten gequencht. Die Mischung wurde in ein 11 Becherglas transferriert und durch Zugabe von gesättigter NaHCO₃-Lösung neutralisiert. Schließlich wurde in einen Scheidetrichter überführt und das Produkt mit Toluol (3 x 80 ml) extrahiert. Die vereinigten organischen Phasen wurden mit Wasser (2 x 20 ml) gewaschen und über Na₂SO₄ getrocknet. Nach Entfernen des Lösungsmittels und Trocknung wurde ein roter Feststoff (0,480 mg, 1,00 mmol; 100%) erhalten. ¹H-NMR (C₂D₂Cl₄, 100 °C; δ = 7.14-6.87 (m, 12H, Th-H & Vinyl-H), 4.14-4.04 (m, 2H, CH₂), 1.87-1.81 (m, 2H, CH₂), 1.30-1.20 (m, 6H, 3xCH₂), 0.84 (m, 3H, CH₃) ; MALDI-MS (m/z): 479.2 (M⁺) . Das Rohprodukt wurde ohne weitere Reinigung für den nächsten Schritt verwendet.

### Dialdehyd (40):

Phosphoroxychlorid (1,1 ml, 12,2 mmol) wurde zu einer Lösung von Dimethylformamid (1,04 ml, 13,4 mmol) in Dichlormethan (DCM, 13,8 ml) gegeben und die Mischung für 2 Stunden unter Argon gerührt. Zu einer Lösung von 39 (479,8 mg, 1,00 mmol) in DCM (18,3 ml) wurde ein Teil des oben beschriebenen Vilsmeierreagens (11,6 ml, 10,0 mmol) zugetropft. Das Gemisch wurde für 48 Stunden unter Argon refluxiert. Nach dem Abkühlen auf RT wurde die Lösung mit einer gesättigten NaHCO₃-Lösung ( 61ml) versetzt und für 2 Stunden gerührt. Nach Überführung in einen Scheidetrichter und Zugabe von DCM (80 ml) wurde die organische Phase abgetrennt und die wässrige dreimal mit DCM (30 ml) extrahiert. Die vereinigten organischen Phasen wurden mit Wasser (2 x 20 ml) gewaschen und über Na₂SO₄ getrocknet. Nach Abrotieren des Lösungsmittels und Trocknung wurde das Produkt als Gemisch von cis- und trans-Isomeren als roter Feststoff erhalten (528 mg, 0,987 mmol; 99 %). ¹H-NMR (C₂D₂Cl₄, 100 °C; δ = 9.93-9.58 (m, 2H, CHO), 7.80-7.60 (m, 4H, Th-H), 7.40-7.05 (m, 6H, Th-H & Vinyl-H), 4.30-4.10 (m, 2H, CH₂-N), 2.05-1.90 (m, 2H, CH₂), 1.50-1.30 (m, 6H, 3xCH₂), 0.84 (m, 3H, CH₃); MALDI-MS (m/z): 535.1 (M⁺); UV-Vis(DCM): 518 nm.

### 2-Cyano-3-{5-[(E)-2-(6-{(E)-2-[5-(2,2-dicyano-vinyl)-thiophen-2-yl]-vinyl}-4-hexyl-4H-dithieno[3,2-b;2',3'-d]pyrrol-2-yl)-vinyl]-thiophen-2-yl}-acrylnitril (41):

Eine Mischung von Dialdehyd **40** (0,520 g, 0,97 mmol), Malodinitril (0,513 mg, 7,76 mmol) und Piperidin (0,083 mg, 0,1 ml, 0,97 mmol) in 1,2-Dichlorethane (95 ml) wurde für 46 Stunden unter Argon refluxiert. Der Fortgang der Reaktion wurde mittels UV-Vis Spektroskopie (DCM, □ₘₐₓ = 577nm, Schulter brei □ = 545 nm) verfolgt. Eine Fortsetzung der Knoevenagel-Reaktion für weitere 24 Stunden nach erneuter Zugabe von Malononitril (0,133g, 2,00 mmol) und Piperidin (0,01 ml, 0,01 mmol) ergab keine Veränderung im UV-Vis-Spektrum. Deswegen wurde die Reaktion abgebrochen und das Lösungsmittels mittels Rotationsverdampfer abgezogen. Der Rückstand wurde mit Wasser (50ml) versetzt und für 2 Stunden refluxiert. Nach Heißfiltration mittels Filternutsche wurde der Filterrückstand getrocknet und danach einer Soxhletextraktion mit Methanol unterzogen. Nach der Trocknung wurde nacheinander mit Toluol und Chlorbenzol extrahiert. Die ausgefallenen Feststoffe beider Extraktionen (Toluol: 0,170 g, bronzeglänzender Feststoff; Chlorbenzol: 0,400g, dunkelbronzeglänzender Feststoff) lieferten das gleiche UV-Vis Spektrum aufgenommen in DCM. Wegen der schlechten Löslichkeit in allen organischen Lösungsmitteln war eine korrekte Zuordnung der ¹H-NMR Signale nicht möglich. MALDI-MS (m/z): 631.2 (M⁺) ; UV-Vis(DCM): 577 nm, 545 nm(sh).

### Ausführungsbeispiel 11:

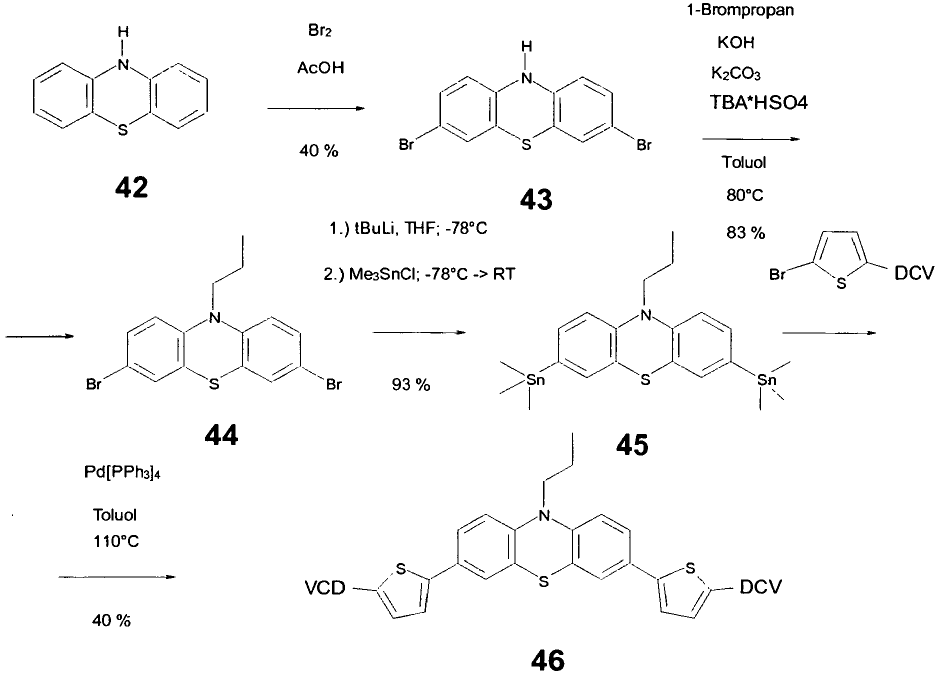

3,7-Dibrom-10H-phenothiazine(43): hergestellt aus 10H-Phenothiazine (**42**) in 40% Ausbeute nach einer Literaturvorschrift ( C.Bodea, M.Raileanu, Ann.Chim.(Paris), 1960, 631, 194-197).

NMR ¹H (Aceton-d6): 8.22(br.s, 1H), 7.24(d, 2H), 7.22 (s, 2H), 6.76 (d, 2H).

**3,7-Dibromo-10-propyl-10H-phenothiazin (44):** synthetisiert in 83% Ausbeute aus 3,7-Dibrom-10H-phenothiazine (**43**) nach einem modifiziertem Literaturprotokoll ( H.Oka, J.Mater.Chem., 2008, 18, 1927-1934).

NMR ¹H (CDCl₃) : 7.26(d, 2H), 7.25(s, 2H), 6.69(d, 2H), 3.75(t, 2H), 1.80 (m, 2H), 1.00 (t, 3H).

**10-Propyl-3,7-bis-trimethylstannanyl-10H-phenothiazin (45):** hergestellt aus (**44**) nach der oben beschriebenen allgemeinen Vorschrift zur Darstellung von Bistrimethylstannnyl-Verbindungen.

NMR ¹H (CDCl₃) : 7. 60 (m, 4H), 7.20(d, 2H), 4.15(m, 2H), 2.19(m, 2H), 1.35(t, 3H), 0.60(s, 18H).

**2-Cyano-3-(5-{7-[5-(2,2-dicyano-vinyl)-thiophen-2-yl]-10-propyl-10H-phenothiazin-3-yl}-thiophen-2-yl)-acrylonitril (46):** hergestellt in 40% Ausbeute via Stille-Kupplung aus (**45**) gemäß der oben beschriebenen allgemeinen Vorschrift.

Schmp.: 290°C

NMR ¹H (TCE-d2 at 375°C) : 7.79(s, 2H), 7.77(d, 2H), 7.54(d, 2H), 7.47(s, 2H), 7.40(d, 2H), 6.96(d, 2H), 3.94(t, 2H), 1.93 (m, 2H), 1.13(t, 3H).

UV-VIS: 574 nm (Film 30.0 nm, A ₘₐₓ 0,28); 495 nm (THF, A ₘₐₓ 0,61).

### Ausführungsbeispiel 12:

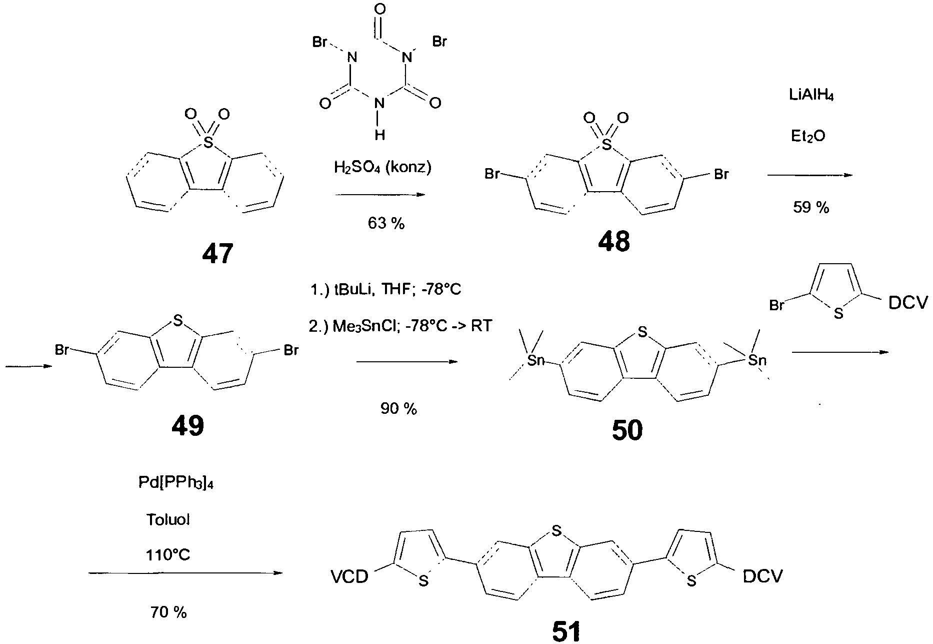

### DCV-T-PhTPh-T (51) :

**3,7-Dibrom-dibenzothiophene 5,5-dioxid (48):** hergestellt aus Dibenzothiophenedioxid (**47**) in 63% Ausbeute nach einer Literaturvorschrift (s. H. Sirringhaus, R.H. Friend, C. Wang et al., J.Mater.Chem., 1999, 9, 2095-2101).

NMR ¹H (DMSO-d6): 8.71(s, 2H), 8.50(d, 2H), 8.38(d, 2H).

**3,7-Dibrom-dibenzothiophen (49):** hergestellt aus 3,7-Dibromdibenzothiophendioxid (**48**) in 59%iger Ausbeute nach einem Literaturprotokoll (s. H. Sirringhaus, R.H. Friend, C. Wang et al., J.Mater.Chem., 1999, 9, 2095-2101).

NMR ¹H (CDCl₃): 7.97 (d, 2H), 7.96(d, 2H), 7.56(dd, 2H).

**3,7-Bis-trimethylstannnyl-dibenzothiophen (50):** hergestellt aus 49 in 90%iger Ausbeute nach dem oben aufgeführten allgemeinen Protokoll zur Synthese von Bistrimethylstannnyl-Verbindungen.

NMR ¹H (CDCl₃): 8.12 (d, 2H), 7.98(s, 2H), 7.56(d, 2H), 0.35(s, 18H).

**2-Cyano-3-(5-(7-[5-(2,2-dicyano-vinyl)-thiophen-2-yl]-dibenzothiophen-3-yl}-thiophen-2-yl)-acrylonitril (51):** synthetisiert in 70%iger Ausbeute nach dem oben aufgeführten allgemeinen Protokoll für Knoevenagel-Kondensationen.

Schmp.: 353°C.

NMR ¹H (DMSO-d6 at 375 K): 8.57(d, 4H), 8.48(d, 2H), 8.02(s, 2H), 7.94(m, 4H)

UV-VIS: 476 nm (Film 30.0 nm, A ₘₐₓ 0,55)

### Ausführungsbeispiel 13:

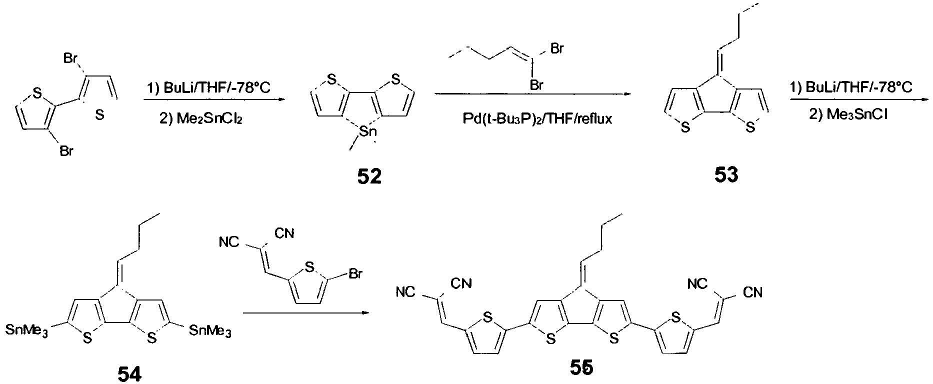

### 7,7-Dimethyl-7H-3,4-dithia-7-stanna-cyclopenta[a]pentalen (52) :

2.0 g (6.17 mmol) Dibrombithiophen wurden in 120 ml wasserfreiem Diethylether gelöst und unter Argon auf -78°C abgekühlt. N-Butyllithium (8 ml, 12.34 mmol, 1.6 molar in Hexan) wurde langsam zugegeben. Das Reaktionsgemisch wurde für 2 Stunden bei -78°C gerührt und danach auf RT erwärmt. Eine Lösung von Dimethylstannyldichlorid (1.42 g, 6.46 mmol) in 30 ml trockenem Diethylether wurde zugegeben und die Mischung für 8 Stunden unter Argon gerührt. Alle Lösungsmittel wurden abrotiert und der Rückstand in Hexan aufgenommen. Die so erhaltene Suspension wurde über Celite gefiltert und das Lösemittel entfernt um 1.27 g von Verbindung **1** (66% Ausbeute) zu erhalten. GC-MS m/z 314 (11.09 Min).

### 4-Butylidene-4H-cyclopenta[2,1-b;3,4-b']dithiophen (53):

500 mg (1.6 mmol) von **52** and 364 mg (1.6 mmol) Dibrompenten wurden in 32 ml frisch destilliertem THF gelöst und 42 mg (80 µmol) Bis(tri-tert-butylphosphine)palladium(0) wurden zugegeben. Die Reaktionsmischung wurde für 8 Stunden unter Rühren refluxiert. THF wurde abrotiert und der Rückstand in n-Hexan aufgenommen. Das Rohprodukt wurde mittels Säulenchromatographie gereinigt (n-Hexan als Eluent (Rf = 0.4) um 115 mg der Zielverbindung **53** (37% Ausbeute) zu erhalten. GC-MS m/z 232 (18.10 Min).

### 4-Butylidene-2,6-bis-trimethylstannyl-4H-cyclopenta[2,1-b;3,4-b']dithiophen (54):

Verbindung **54** wurde nach obiger allgemeiner Vorschrift in 69%iger Ausbeute synthetisiert.

### 3-(5-(4-Butylidene-6-[5-(2,2-dicyano-vinyl)-thiophen-2-yl]-4H-cyclopenta[2,1-b;3,4-b']dithiophen-2-yl}-thiophen-2-yl)-2-cyano-acrylonitril (55):

Verbindung **55** wurde nach obigem allgemeinen Protokoll für Stille-Kupplungen hergestellt. UV/Vis: λmax (Dichlormethan) 525 nm.

### Ausführungsbeispiel 14:

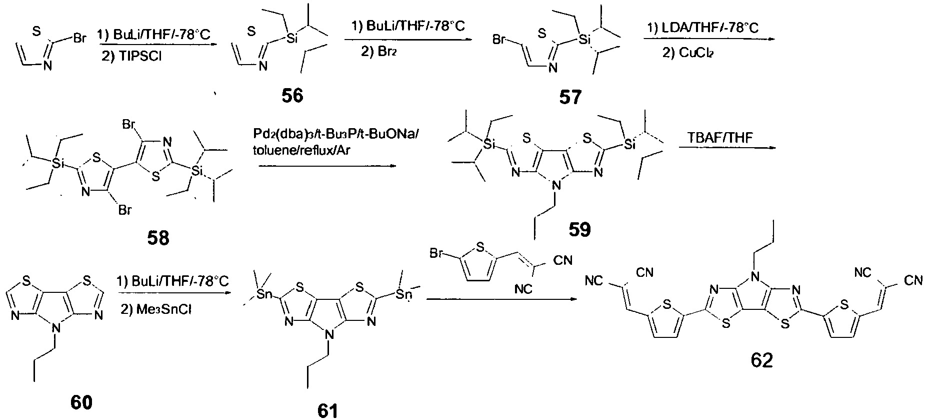

### 2-Triisopropylsilanylthiazol (56):

Verbindung **56** wurde nach einer Literaturvorschrift (E. L. Stangeland, T. Sammakia, J. Org. Chem., 2004, 69, 2381-2385) hergestellt.

### 5-Bromo-2-triisopropylsilanylthiazol (57):

Verbindung **57** wurde nach einer Literaturvorschrift (E. L. Stangeland, T. Sammakia, J. Org. Chem., 2004, 69, 2381-2385) hergestellt.

**4,4'-Dibromo-2,2'-bis-triisopropylsilanyl-[5,5']bithiazolyl (58), 7-Propyl-2,5-bis-triisopropyl-silanyl-7H-3,4-dithia-1,6,7-triaza-cyclopenta[a]pentalen (59) und 7-Propyl-7H-3,4-dithia-1,6,7-triaza-cyclopenta[a]pentalen (60): Verbindungen** 58, 59 und 60 wurden ebenfalls nach einem Lieraturprotokoll (Y. A. Getmanenko, P. Tongwa, T. V. Timofeeva, S. R. Marder, Org. Lett., 2010, 12 (9), 2136-2139) synthetisiert.

**7-Propyl-2,5-bis-trimethylstannyl-7H-3,4-dithia-1,6,7-triaza-cyclopenta[a]pentalene (61) und 2-Cyano-3-(5-{5-[5-(2,2-dicyano-vinyl)-thiophen-2-yl]-7-propyl-7H-3,4-dithia-1,6,7-triaza-cyclopenta[a]pentalen-2-yl}-thiophen-2-yl)-acrylonitril (62):** Verbindungen **61** (Ausbeute 87%) and **62** (20.0 mg; Ausbeute 8%) wurden nach unserer obigen allgemeinen Vorschrift für Stille-Kupplungen hergestellt. Verbindung **62**: MALDI (m/z): 539.1; UV (film, 30nm): 601 nm, Aₘₐₓ = 0.49.

### Ausführungsbeispiel 15:

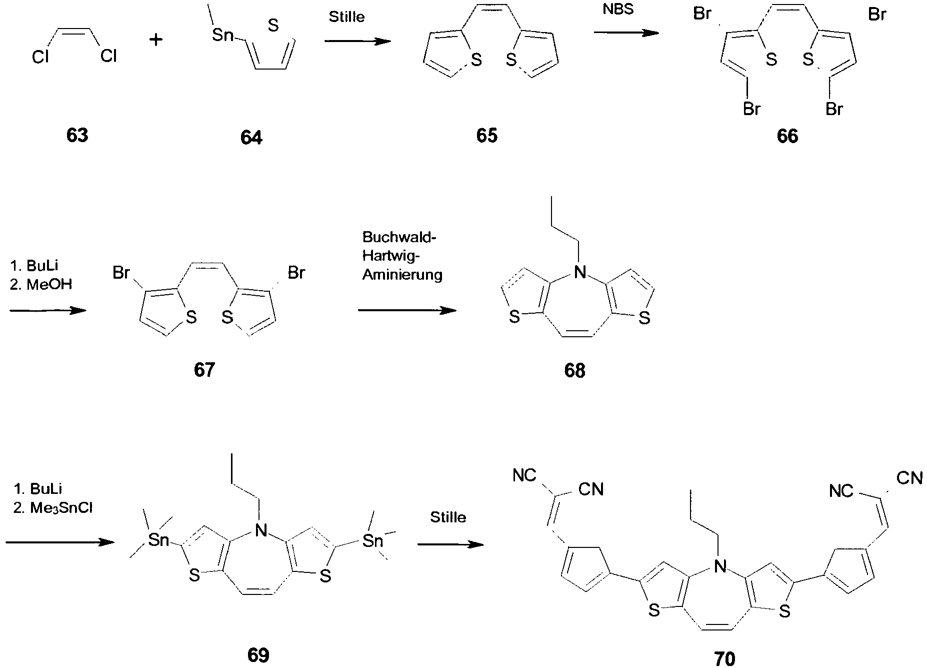

### 8-Propyl-8H-dithieno[3,2-b:2',3'-f]azepin (68)

Verbindungen **65**, 6**6**, **67**, und 68 werden nach einem Lieraturprotokoll (C. Song, D. B. Walker, T. M. Swager, Macromolecules 2010, 43, 12, 5233-5237) synthetisiert.

### Dicyanovinyl-Verbindung (70)

Verbindungen **69** und **70** werden nach der AAV1 für Stille-Kupplungen hergestellt.

### Ausführungsbeispiel 16:

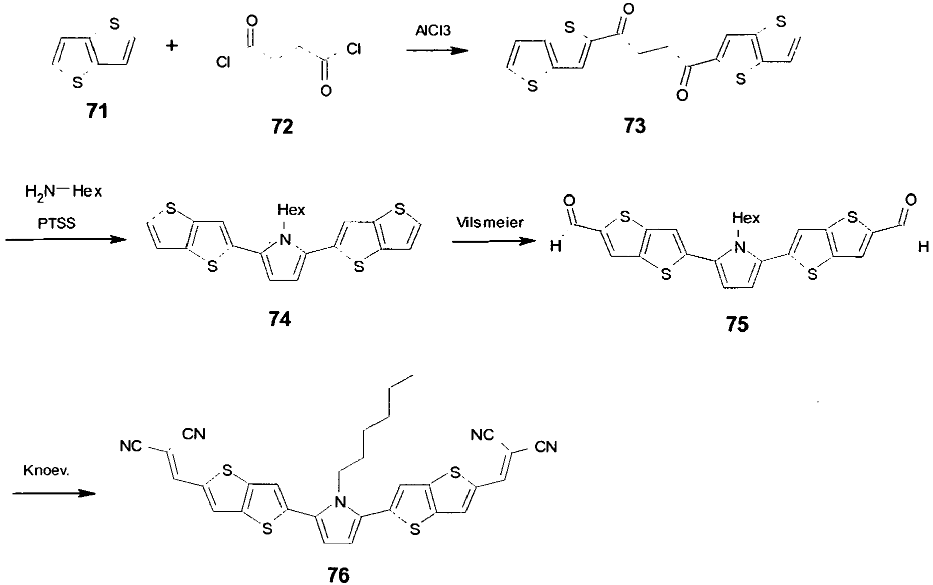

### 1-Hexyl-2,5-bis(thieno[3,2-b]thiophen-5-yl)pyrrol (74)

Verbindungen **73**, und **74** werden nach einem Lieraturprotokoll (L. I. Belen'kii, V. Z. Shirinyan, G. P. Gromova, A. V. Kolotaev, Y. A. Strelenko, S. N. Tandura, A. N. Shumskii, M. M. Krayushkin, Chem. Heterocycl. Comp. 2003, 39, 1570) synthetisiert.

### 5-[5-(5-Formylthieno[3,2-b]thiophen-2-yl)-1-hexyl-pyrrol-2-yl]thieno[3,2-b]thiophen-2-carbaldehyd (75)

Zu einer Lösung von 1-Hexyl-2,5-bis(thieno[3,2-b]thiophen-5-yl)pyrrol (**74**) in Dichlormethan wird eine Lösung von Phosphoroxychlorid and DMF in DCM gegeben. Die Reaktionsmischung wird 16 h refluxiert, anschließend gibt man ges. Natriumhydrogencarbonat-Lösung hinzu und rührt 2 h bei Raumtemperatur. Nach Phasentrennung wird die organische Phase über Natriumsulfat getrocknet. Nach dem Abdestillieren der Lösemittel wird der Rückstand aus Toluol umkristallisiert und man erhält das Produkt.

### 2-[[2-[5-[5-(2,2-Dicyanovinyl)thieno[3,2-b]thiophen-2-yl]-1-hexyl-pyrrol-2-yl]thieno[3,2-b]thiophen-5-yl]methylen]propandinitril (76)

Eine Lösung von Dialdehyd 75, Malodinitril und Piperidin in 1,2-Dichlorethan wird für 48 h refluxiert. Nach dem Abdestillieren des Lösemittels wird der Rückstand in Wasser suspendiert und für 2 h refluxiert. Das Rohprodukt wird filtriert, gewaschen mit Wasser und Methanol und im Vakuum getrocknet. Der Rückstand wird aus Chlorbenzol umkristallisiert und man erhält das Produkt.

### Ausführungsbeispiel 17:

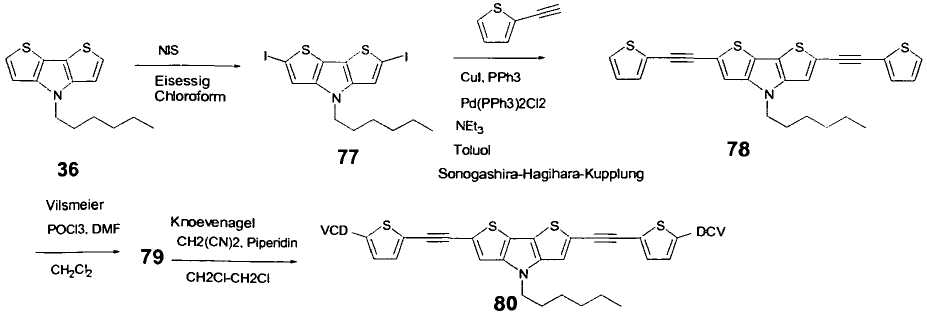

### 4-Hexyl-2,6-diiod-4H-dithieno[3,2-b;2',3'-d]pyrrol (77):

Zu einer Lösung von 4-Hexyl-4H-dithieno[3,2-b;2',3'-d]pyrrol (**36**) (0,500 g, 1,90 mmol) in einem Gemisch bestehend aus Chloroform (12 ml) und Eisessig (12 ml) wurde unter Rühren, Lichtausschluß und Argon bei O°C (Eiskühlung) N-Iodsuccinimid (1,068 g, 4,75 mmol) in 5 Portionen zu jeweils 214 mg zugegeben. Die Zeitspanne zwischen den Zugaben betrug jeweils 7 Minuten. Es wurde über Nacht bei 0-4 °C unter Argon und Lichtausschluß weiter gerührt und dann auf RT erwärmt. Nach 2 Stunden fortgesetztem Rühren wurde in einen Scheidetrichter überführt und mit weiterem Wasser (50 ml) und Chloroform (100 ml) versetzt. Nach dem Schütteln wurde abgetrennt und die wässrige Phase noch dreimal mit je 50 ml Chloroform extrahiert. Die vereinigten organischen Phasen wurden nacheinander mit gesättigten wässrigen Lösungen von NaHCO₃und Na₂S₂O₃ (jeweils 30 ml) sowie mit Wasser (2 x 30 ml) gewaschen. Nach dem Trocknen über Na₂SO₄ wurde abfiltriert und das Lösungsmittel abrotiert. Der Rückstand wurde mittels Flashsäulenchromatographie gereinigt (Kieselgel, n-Hexan als Eluent (R_{f} = 0.37). Nach Abrotieren des Lösungsmittels wurden 0.718 g (74% Ausbeute) gelbe Kristalle von 2 erhalten. ¹H-NMR (CDCl₂-CDCl₂) : δ =7.21 (s, 2H, Th-H), 4.07 (t, 2H, CH₂-N), 1.80 (t, 2H, CH₂), 1.29 (m, 6H, 3 x CH₂), 0.88 (t, 3H, CH₃).

**4-Hexyl-2,6-bis-thiophen-2-ylethynyl-4H-dithieno[3,2-b;2',3'-d]pyrrol (78):** Die Verbindung wurde in modifizierter Vorgehensweise nach der oben beschriebenen allgemeinen Vorschrift für Sonogashira-Hagihara-Kupplungen synthetisiert.

In einem sorgfältig getrockneten Schraubdeckelüberdruckgefäß wurden nacheinander absolutes Toluol (25 ml) und abs. Triethylamin (3,359 g, 4,6 ml, 33,2 mmol) vorgelegt. Es wurde für 30 Minuten Argon durchgeblubbert (Entgasen). Im leichten Argongegenstrom wurden nacheinander 2-Ethinylthiopen (1,000g, 9,25 mmol), Verbindung 77 (0,4764 g, 0,92 mmol), Pd (PPh₃)₂.Cl₂ (0.0324 g, 0,046 mmol), CuI (0,0176 g, 0,09 mmol) und PPh₃ (0,0242 g, 0,09 mmol) zugegeben. Nach 3 maligem Digerieren (Entgasen und Argonflutung) und weiterem Durchblubbern von Argon für 10 Minuten wurde mit dem Schraubdeckel verschlossen und 48 Stunden bei 50 °C gerührt. Nach dem Abkühlen wurde in einen Scheidetrichter überführt und nach Zugabe von weiteren 20 ml Toluol nacheinander mit Wasser (15 ml), jeweils 30 ml ges. wässr. NH₄Cl-Lösung, ges. wässr. NaHCO₃-Lösung, ges. wässr. NaCl-Lösung und Wasser gewaschen. Nach dem Trocknen über Na₂SO₄ wurde das Lösungsmittel abrotiert und 1,5743 g eines roten Feststoffes isoliert. MALDI-MS (m/z): 475.3 (M⁺). Das Zielprodukt war zu ca. 30% im Rohprodukt vorhanden.

Die Synthesen zum Bisaldehyd **79** über die Vilsmeier-Reaktion und weiter zu 2-Cyano-3-(5-{6-[5-(2,2-dicyano-vinyl)-thiophen-2-ylethynyl]-4-hexyl-4H-dithieno[3,2-b;2',3'-d]pyrrol-2-ylethynyl}-thiophen-2-yl)-acrylonitril **80** über Knoevenagel-Kondensation laufen momentan nach obigem Protokoll.

### Ausführungsbeispiel 18:

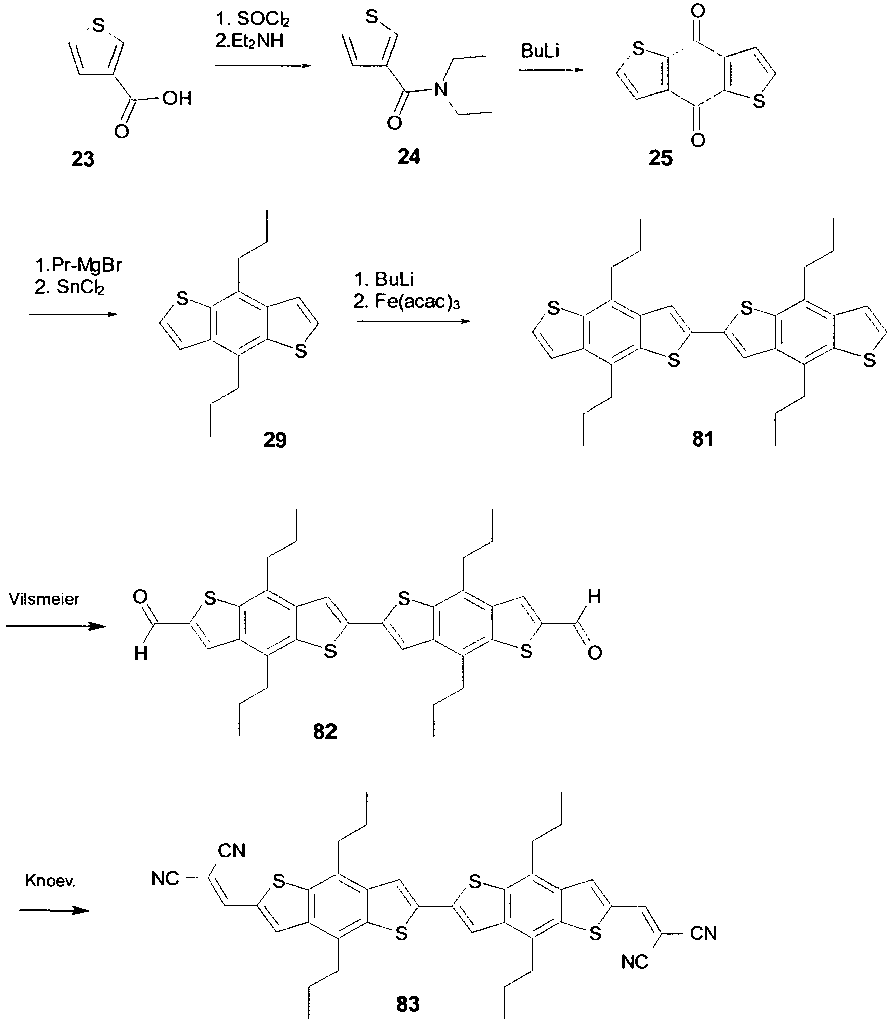

### Bis-benzodithiophen 81

Verbindungen **81** wird nach einem Lieraturprotokoll (J. G. Laquindanum, H. E. Katz, A. J. Lovinger, A. Dodabalapur, Adv. Mater. 1997, 9, 1, 36-39) synthetisiert.

### Dialdehyd-Verbindung 82

Zu einer Lösung von Bis-benzodithiophen **81** in Dichlormethan wird eine Lösung von Phosphoroxychlorid and DMF in DCM gegeben. Die Reaktionsmischung wird 16 h refluxiert, anschließend gibt man ges. Natriumhydrogencarbonat-Lösung hinzu und rührt 2 h bei Raumtemperatur. Nach Phasentrennung wird die organische Phase über Natriumsulfat getrocknet. Nach dem Abdestillieren der Lösemittel wird der Rückstand aus Toluol umkristallisiert und man erhält das Produkt.

### Dicyanovinyl-Verbindung 83

Eine Lösung von Dialdehyd **82**, Malodinitril und Piperidin in 1,2-Dichlorethan wird für 48 h refluxiert. Nach dem Abdestillieren des Lösemittels wird der Rückstand in Wasser suspendiert und für 2 h refluxiert. Das Rohprodukt wird filtriert, gewaschen mit Wasser und Methanol und im Vakuum getrocknet. Der Rückstand wird aus Chlorbenzol umkristallisiert und man erhält das Produkt.

### Ausführungsbeispiel 19:

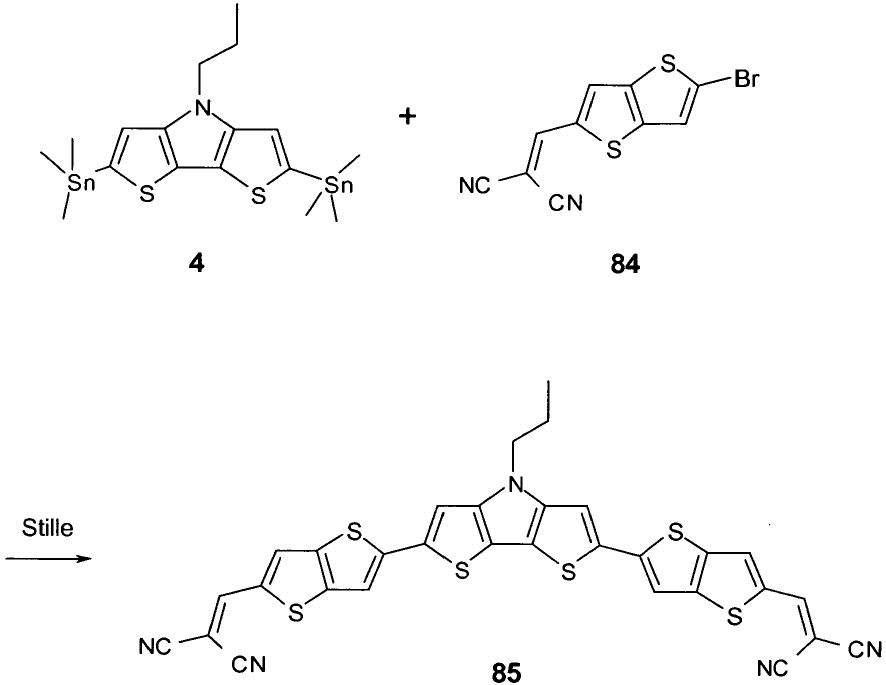

### Dicyanovinyl-Verbindung 85

Verbindung **85** wird nach der AAV1 für Stille-Kupplungen hergestellt.

Die oben beschriebenen Synthesevorschriften können auch zur Synthese weiterer erfindungsgemäßer Verbindungen verwendet werden. Beispielsweise können die Stille Kupplung und die Knoevenagel-Reaktion mit anderen Bausteinen D oder E zur Synthese weiterer erfindungsgemäßer Oligomere eingesetzt werden. So können weitere Organozinnverbindungen R-SnR'3 und organische Halogenide R"-X (X = Halogenid) gemäß der folgenden allgemeinen Formel:

R-SnR'3 + R"-X R-R" + X-SnR'3

wobei R und R" organische Reste zur Verknüpfung der Gruppen D, E und bd sind, über die Stille-Kupplung miteinander gekoppelt werden. Wie bereits weiter oben beschrieben, können die Akzeptorgruppen über dem Fachmann bekannte Reaktionen wie Gattermann, Gattermann-Koch, Houben-Hoesch, Vilsmeier/ Vilsmeier-Haack, oder Friedel-Crafts-Acylierung eingeführt werden

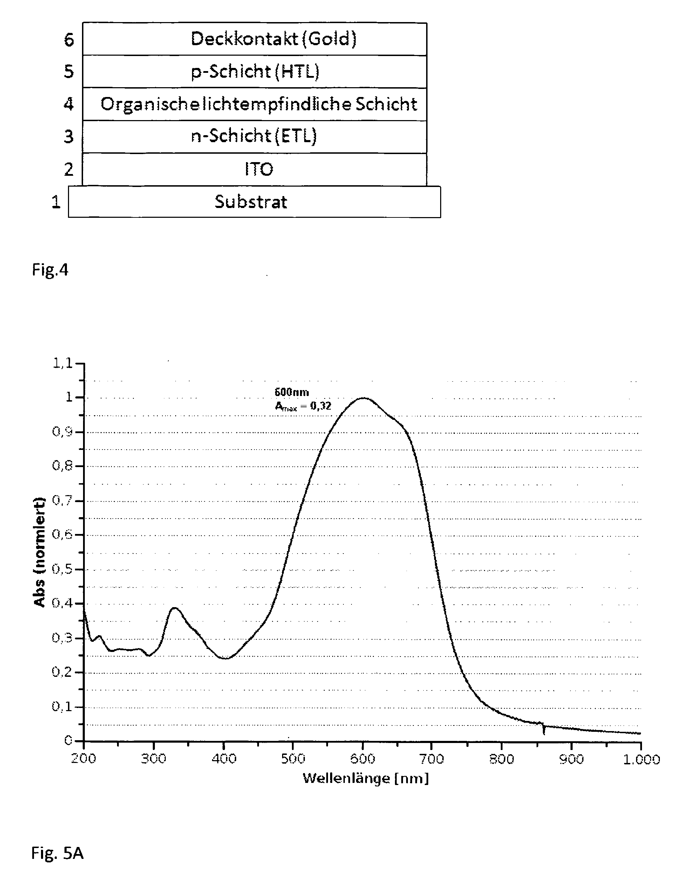

In einem weiteren Ausführungsbeispiel ist eine Verbindung der allgemeinen Formel IIIa als Bestandteil eines organischen lichtempfindlichen Schichtsystems in einem erfindungsgemäßen optoelektronischen Bauelement enthalten. In Fig.4 ist schematisch ein solches erfindungsgemäßes Bauelement dargestellt. Diese weist folgende Schichtreihenfolge auf:
1.) Glas-Substrat **1**,
2.) ITO Grundkontakt **2**,
3.) Elektronentransportschicht (ETL) **3**,
4.) organisches lichtempfindliches Schichtsystem (10-200nm) **4**,
5.) Löchertransportschicht (HTL) **5**,
6.) Deckkontakt (z.B. Gold) **6**.

### Ausführungsbeispiel 20 (Bauelement mit der erfindungsgemäßen

### Verbindung DCV-Fu-TPyT-Fu-Pr(3)):

In einem weiteren Ausführungsbeispiel wird ein MIP-Bauelement bestehend aus einer Probe auf Glas mit transparentem Deckkontakt ITO, einer Schicht Buckminster Fulleren C₆₀, Mischschicht von Verbindung DCV-Fu-TPyT-Fu-Pr(3) der Formel: 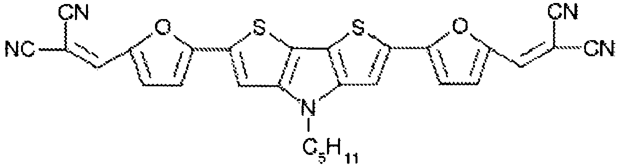 mit C₆₀ 2:1,
einer p-dotierten Löchertransportschicht, und einer Schicht Gold hergestellt.

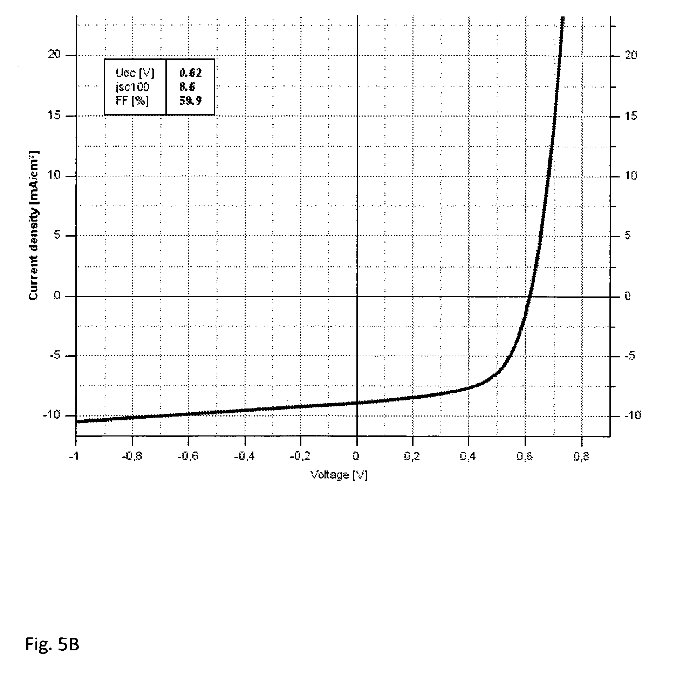

Fig. 5A und 5B zeigen eine schematische Darstellung eines Absorptionsspektrums der Verbindung sowie die Strom - Spannungskurve dieser Mip-Zelle mit einer Mischschicht von Verbindung DCV-Fu-TPyT-Fu-Pr(3) mit C₆₀. Die wichtigsten Kennzahlen wie der Füllfaktor FF, die Leerlaufspannung Uoc und der Kurzschlussstrom jsc zeigen eine gut funktionierende organische Solarzelle.

### Ausführungsbeispiel 21 (Bauelement mit der erfindungsgemäßen Verbindung DCV-Ph-TPyT-Ph-Pr(3)):

In einem weiteren Ausführungsbeispiel wird ein MIP-Bauelement bestehend aus einer Probe auf Glas mit transparentem Deckkontakt ITO, einer Schicht Buckminster Fulleren C₆₀, Mischschicht von Verbindung DCV-Ph-TPyT-Ph-Pr(3) der folgenden Formel mit C₆₀ 1:1, 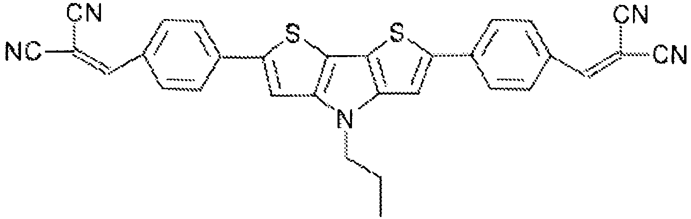 einer p-dotierten Löchertransportschicht, und einer Schicht Gold hergestellt.

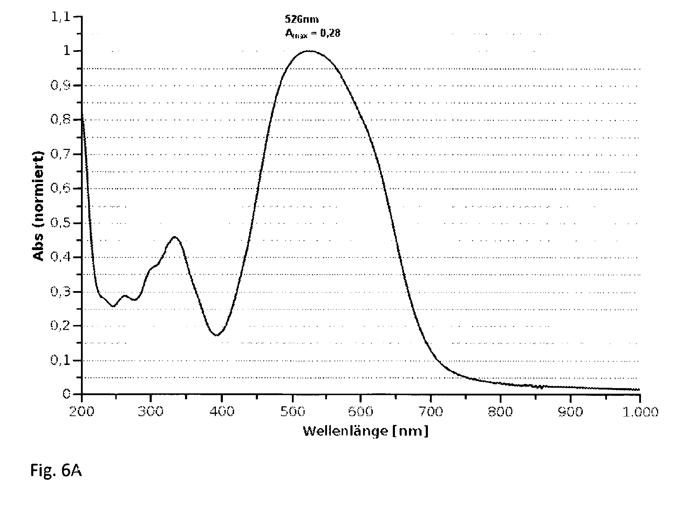

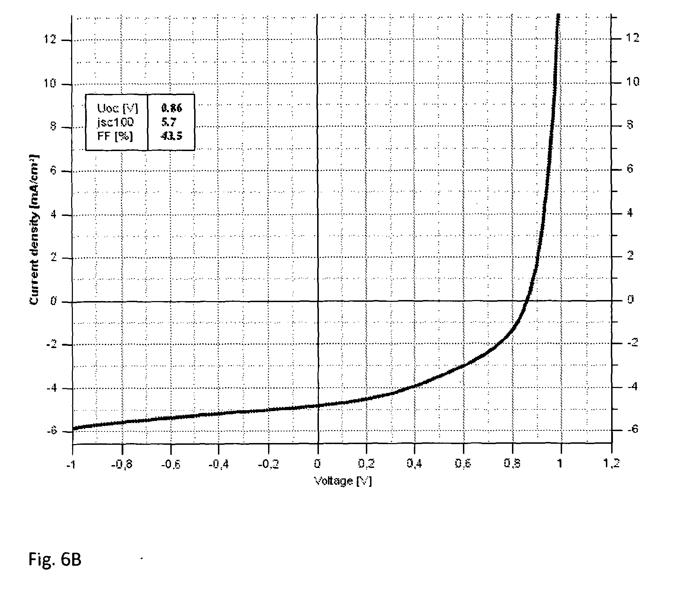

Fig. 6A und 6B zeigen eine schematische Darstellung eines Absorptionsspektrums der Verbindung sowie die Strom - Spannungskurve dieser Mip-Zelle mit einer Mischschicht von Verbindung DCV-Ph-TPyT-Ph-Pr(3) mit C₆₀. Die wichtigsten Kennzahlen wie der Füllfaktor FF, die Leerlaufspannung Uoc und der Kurzschlussstrom jsc zeigen eine funktionierende organische Solarzelle.

### Ausführungsbeispiel 22 (Bauelement mit der erfindungsgemäßen Verbindung DCV-Pyr-TPyT-Pyr-Pr(3)):

In einem weiteren Ausführungsbeispiel wird ein MIP-Bauelement bestehend aus einer Probe auf Glas mit transparentem Deckkontakt ITO, einer Schicht Buckminster Fulleren C₆₀, Mischschicht von Verbindung DCV-Pyr-TPyT-Pyr-Pr(3)mit der folgenden Formel mit C₆₀ 2:1, 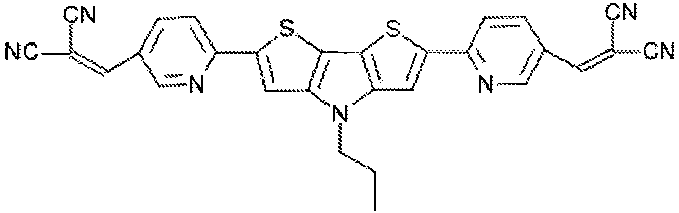 einer p-dotierten Löchertransportschicht, und einer Schicht Gold hergestellt.

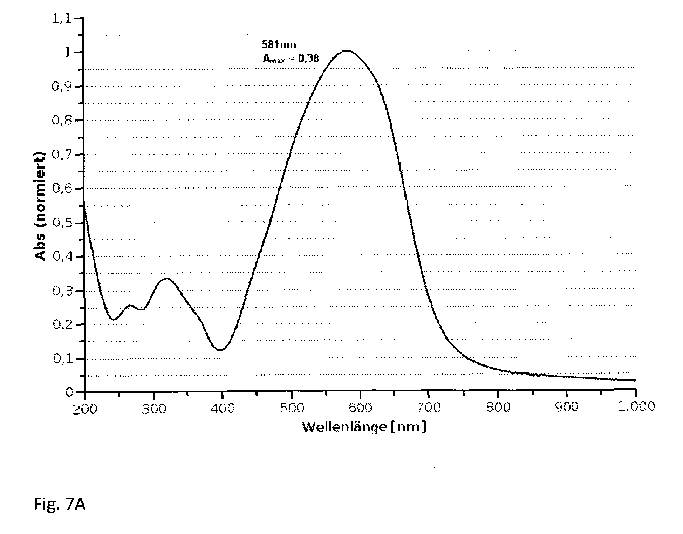

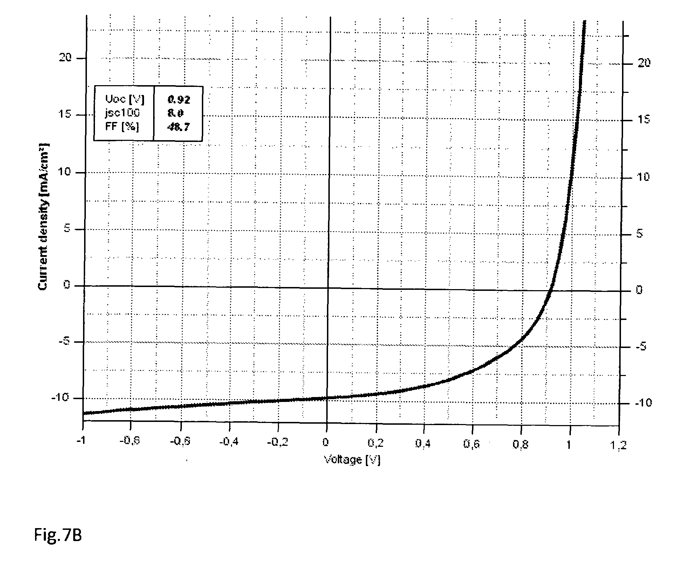

Fig. 7A und 7B zeigen eine schematische Darstellung eines Absorptionsspektrums der Verbindung sowie die Strom - Spannungskurve dieser Mip-Zelle mit einer Mischschicht von Verbindung DCV-Pyr-TPyT-Pyr-Pr(3) mit C₆₀. Die wichtigsten Kennzahlen wie der Füllfaktor FF, die Leerlaufspannung Uoc und der Kurzschlussstrom jsc zeigen eine gut funktionierende organische Solarzelle.

### Ausführungsbeispiel 23 (Bauelement mit der erfindungsgemäßen Verbindung DCV-T-PhTaPh-T-Pr(3)):

In einem weiteren Ausführungsbeispiel wird ein MIP-Bauelement bestehend aus einer Probe auf Glas mit transparentem Deckkontakt ITO, einer Schicht Buckminster Fulleren C₆₀, einer Schicht DCV-T-PhTaPh-T-Pr(3), 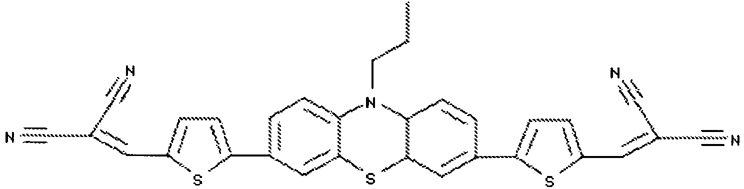 einer p-dotierten Löchertransportschicht, einer Schicht Gold hergestellt.

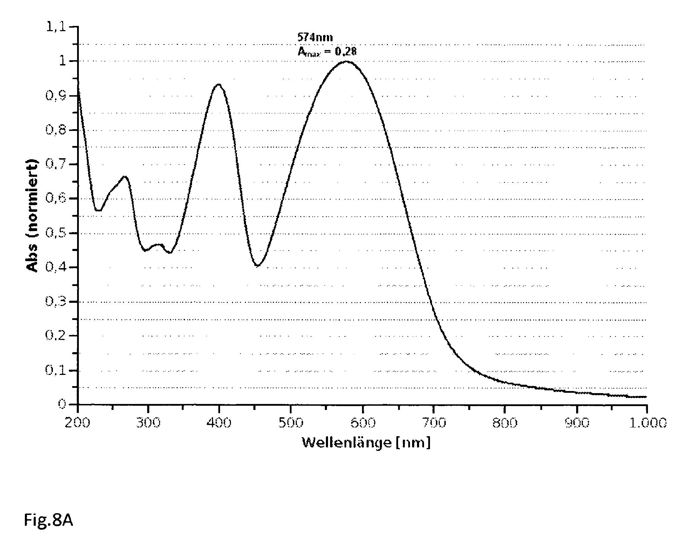

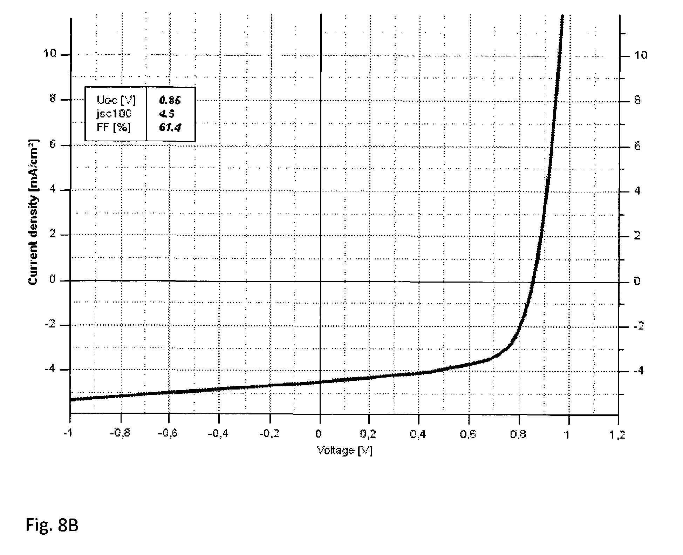

Fig. 8A und 8B zeigen eine schematische Darstellung eines Absorptionsspektrums der Verbindung sowie die Strom - Spannungskurve dieser Mip-Zelle mit einer Schicht DCV-T-PhTaPh-T-Pr(3). Die wichtigsten Kennzahlen wie der Füllfaktor FF, die Leerlaufspannung Uoc und der Kurzschlussstrom jsc zeigen eine gut funktionierende organische Solarzelle.

### Ausführungsbeispiel 24 (Bauelement mit der erfindungsgemäßen Verbindung DCV-TzPyTz-Pr2(3,3)):

In einem weiteren Ausführungsbeispiel wird ein MIP-Bauelement bestehend aus einer Probe auf Glas mit transparentem Deckkontakt ITO, einer Schicht Buckminster Fulleren C₆₀, Mischschicht von Verbindung DCV-TzPyTz-Pr2(3,3): mit C₆₀ 1:1, 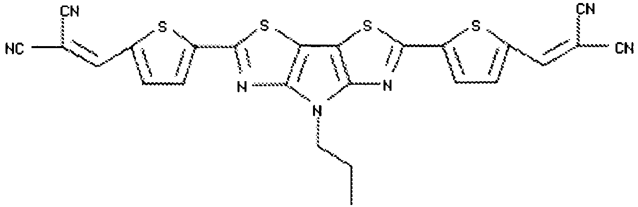 einer p-dotierten Löchertransportschicht, einer Schicht Gold hergestellt.

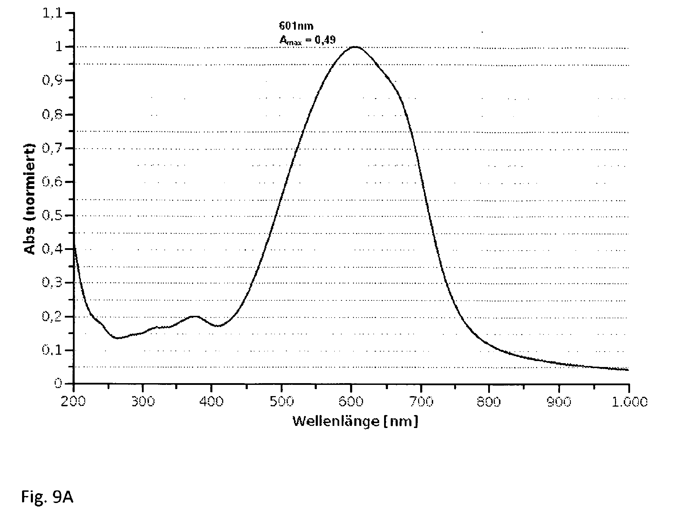

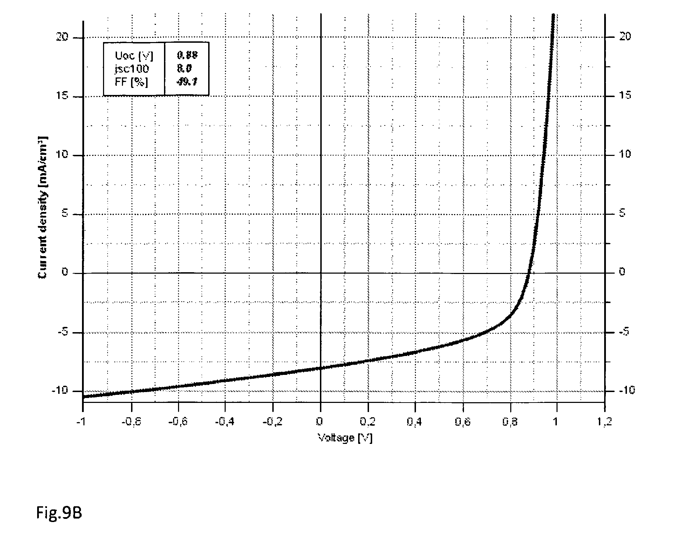

Fig. 9A und 9B zeigen eine schematische Darstellung eines Absorptionsspektrums der Verbindung sowie die Strom - Spannungskurve dieser Mip-Zelle mit einer Mischschicht von Verbindung DCV-TzPyTz-Pr2(3,3) mit C₆₀. Die wichtigsten Kennzahlen wie der Füllfaktor FF, die Leerlaufspannung Uoc und der Kurzschlussstrom jsc zeigen eine funktionierende organische Solarzelle.

### Bezugszeichenliste

1 Substrat
2 Elektrode
3 Transportschichtsystem (ETL bzw. HTL)
4 organisches lichtempfindliches Schichtsystem
5 Transportschichtsystem (ETL bzw. HTL)
6 Gegenelektrode

## Patentansprüche

1. Verbindungen der allgemeinen Formel IIIa: 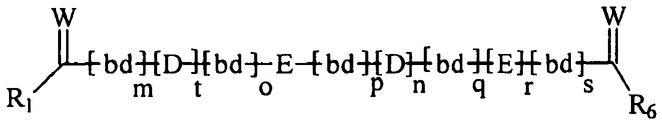
- mit W jeweils unabhängig voneinander ausgesucht aus C(CN)2, CHCN, C(CN)COOR', mit R' jeweils ausgesucht aus C1-C10-Alkyl, C3-C10-Aryl oder C2-C8-Heteroaryl, besonders bevorzugt ausgesucht aus C(CN)2, CHCN,
- R1 und R6 jeweils unabhängig voneinander ausgesucht aus H, C1-C30 Alkyl, C1-C30 Perfluoralkyl, C3-C10 Aryl, C2-C8-Heteroaryl, CN,
- wobei die Gruppen D ausgesucht sind aus: 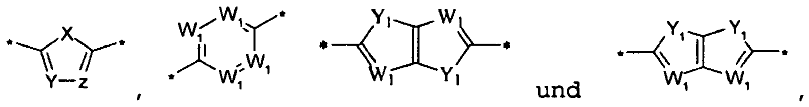
- wobei Y1 ausgewählt ist aus: O, S, Se, P(R), P(O)R, Si(RR'), C(RR') und N(R) und
- W1 unabhängig ausgewählt ist aus: N und C-R mit R und R' unabhängig voneinander ausgewählt aus substituiertem oder unsubstituiertem C1 bis C30-Alkyl, C3-C6-Aryl, oder C3-C8-Heteroaryl und
- wobei X jeweils unabhängig voneinander ausgesucht ist aus O, NR', S, Se, mit R' ausgesucht aus C1-C30-Alkyl, C1-C10-Aryl oder C1-C8-Heteroaryl,
- Y jeweils unabhängig voneinander ausgesucht ist aus N oder CR9 mit R9 = H, Halogen, C1-C30 Alkyl, C1-C30 Alkenyl, C1-C30 Alkinyl, jeweils linear oder verzweigt, substituiert oder unsubstituiert, OR', SR', SiR'3, NR'2 mit R' ausgesucht aus C1-C10-Alkyl, C3-C10-Aryl oder C1-C8-Heteroaryl,
- Z jeweils unabhängig voneinander ausgesucht ist aus N oder CR10 mit R10 = H, Halogen, C1-C30 Alkyl, C1-C30 Alkenyl, C1-C30 Alkinyl, jeweils linear oder verzweigt, substituiert oder unsubstituiert, OR', SR', SiR'3, NR'2 mit R' ausgesucht aus C1-C10-Alkyl, C3-C10-Aryl oder C1-C8-Heteroaryl,
wobei R9 und R10 einen Ring bilden können, bevorzugt einen 5-Ring oder 6-Ring,
- wobei die Gruppen E ausgewählt sind aus: 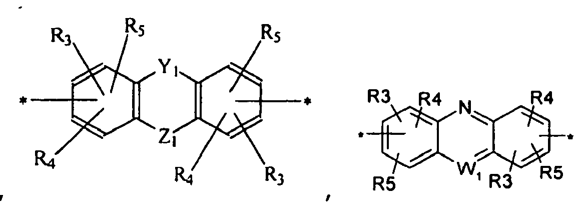 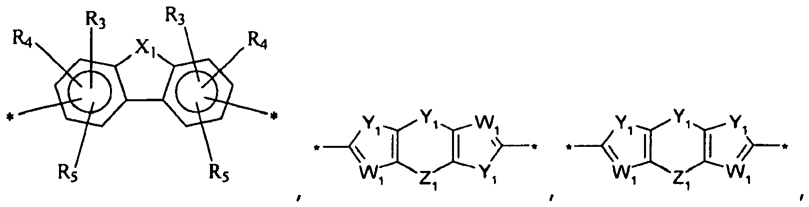 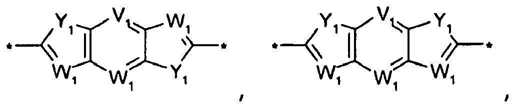 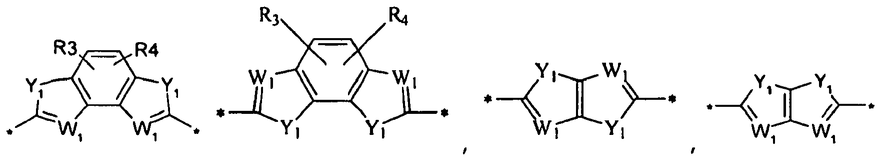 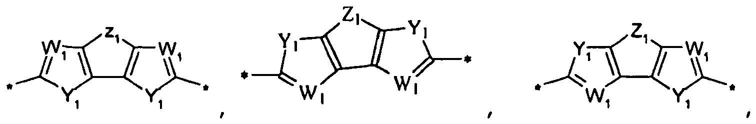 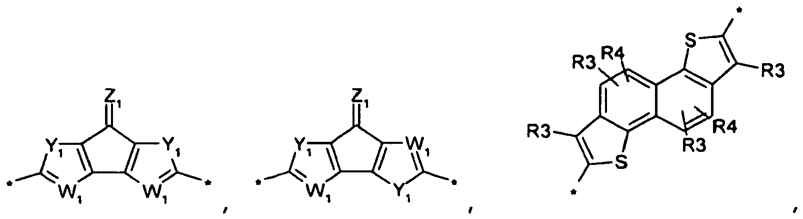 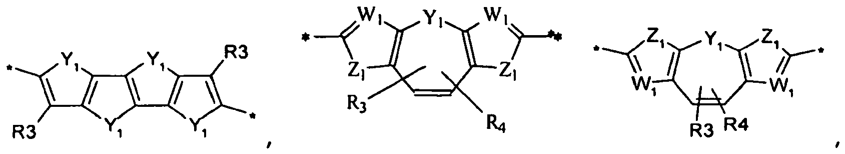
- wobei V1 und W1 unabhängig voneinander ausgewählt sind aus: N und C-R mit R = H, Halogen, C1-C30 Alkyl, C1-C30 Alkenyl, C1-C30 Alkinyl, jeweils linear oder verzweigt, substituiert oder unsubstituiert, C3-C10-Aryl oder C1-C8-Heteroaryl, substituiert oder unsubstituiert, OR', SR', SiR'3, NR'2 mit R' ausgesucht aus C1-C10-Alkyl, C1-C10-Aryl oder C1-C8-Heteroaryl,
- Y1 und Z1 ausgewählt ist aus: O, S, Se, P(R), P(O)R, Si(RR'), C(RR') und N(R) mit R und R' jeweils unabhängig voneinander ausgesucht aus H, linear oder verzweigtem, substituiert oder unsubstituiertem C1-C30 Alkyl, substituiert oder unsubstituiertem Aryl, substituiert oder unsubstituiertem Heteroaryl, Acyl (COR'), COOR' oder OR', mit R' ausgesucht aus C1-C30-Alkyl, C3-C10-Aryl oder C1-C8-Heteroaryl und
- X1 ausgewählt ist aus: O, S, Se, und R und R' jeweils unabhängig voneinander ausgewählt ist aus H, C1 bis C20-Alkyl, linear oder verzweigt, substituiert oder unsubstituiert, C3 bis C6 Aryl oder C3 bis C8 Heteroaryl, OR', SR', SiR'3, NR'2 mit R' ausgesucht aus C1-C10-Alkyl, C3-C6-Aryl oder C3-C8-Heteroaryl,
- und wobei R3, R4 und R5 unabhängig voneinander H, C1 bis C20-Alkyl, linear oder verzweigt, substituiert oder unsubstituiert, C3 bis C6 Aryl oder C3 bis C8 Heteroaryl, OR', SR', SiR'3, NR'2 mit R' ausgesucht aus C1-C10-Alkyl, C3-C6-Aryl oder C3-C8-Heteroaryl sein können,
- bd unabhängig voneinander *-C=C-* oder *-C ≡C-* bedeutet,
n, m, o, p, q, und r, s, und t unabhängig voneinander 0 oder 1 sein können mit der Maßgabe, dass mindestens ein Parameter 1 ist,
- wobei eine aus den Gruppen bd, E und D aufgebaute Donoreinheit zumindest 10 konjugierte Elektronen aufweist und
- die mit dem Stern* gekennzeichneten Bindungen, die Bindungen zu weiteren Gruppen in den Verbindungen andeuten.

2. Verbindung nach Anspruch 1 mit r=s=0 und D ausgesucht aus

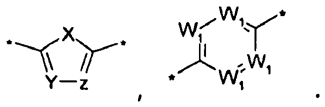

3. Verbindungen nach Anspruch 1, wobei die Gruppe 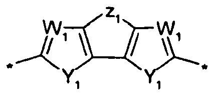 mit Y1 = S und die Gruppen D = 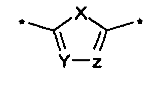 mit der allgemeinen Formel:

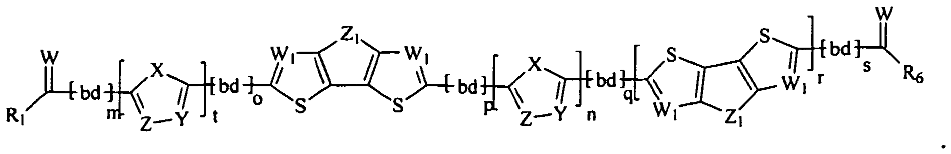

4. Verbindungen nach dem vorhergehenden Anspruch mit m, r, s und q = 0 mit der allgemeinen Formel: 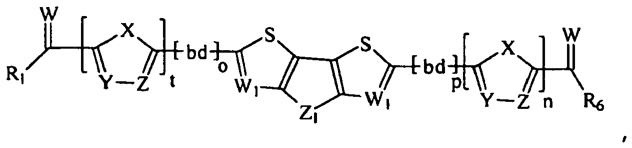 wobei bevorzugt o und p = 0 und t und n = 1 und W1 = C-R mit der allgemeinen Formel: 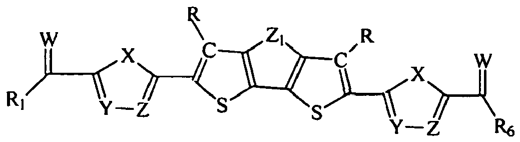 ist.

5. Verbindungen nach Anspruch 3 mit t, m, o und s =0 und r = 1 mit der allgemeinen Formel: 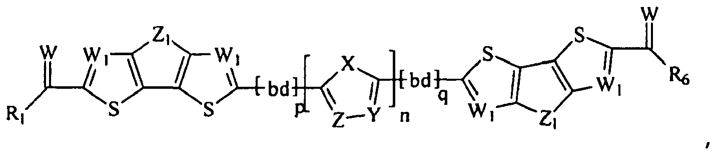 wobei bevorzugt p und q = 0 und W1 = C-R mit der allgemeinen Formel:

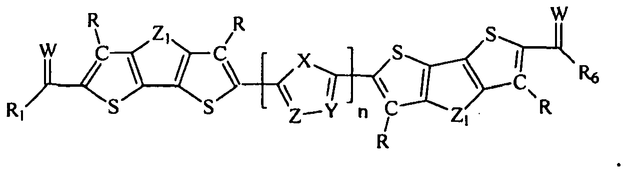

6. Verbindungen nach Anspruch 1 mit D = 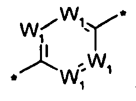 und E = 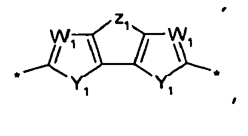 wobei bevorzugt m, r, s und q =0 und t und n jeweils = 1 mit der allgemeinen Formel: 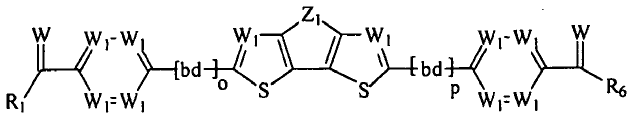 ist.

7. Verbindungen nach Anspruch 6 mit o und p = 0 und W1 = C-R.

8. Verbindungen nach Anspruch 1 mit D = 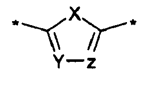 und E
ausgewählt aus:

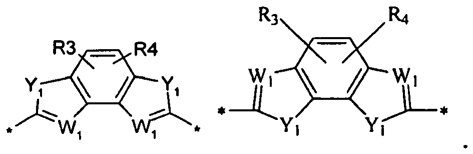

9. Verbindungen nach dem vorherigen Anspruch 8 mit m, q, r, und s = 0 und t und n jeweils = 1 mit der allgemeinen Formel:

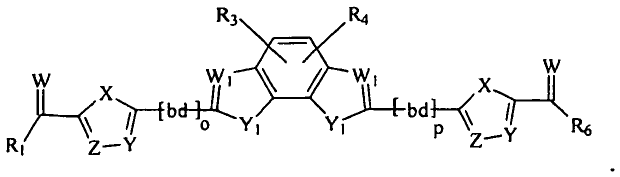

10. Verbindungen nach dem vorherigen Anspruch mit X = S, und Y1 = S sowie W1 = C-R.

11. Verbindungen nach Anspruch 1 mit m, t, n, o, q und s = 0 und r = 1, wobei E ausgewählt ist aus: 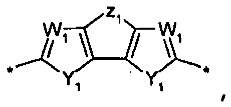 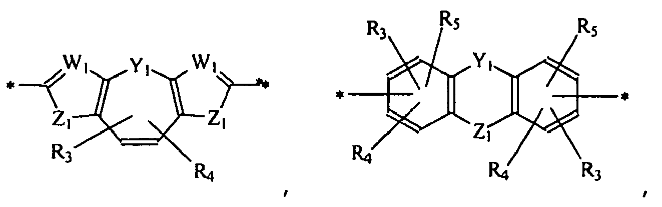 und

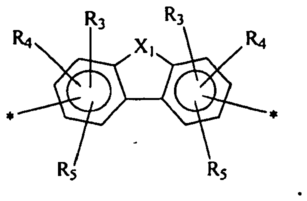

12. Verbindungen nach dem vorherigen Anspruch, wobei zusätzlich p = 0.

13. Verbindungen nach einem der vorherigen Ansprüche, bei denen W ausgewählt ist aus: C(CN)2, CHCN und R1 und R6 ausgewählt sind aus: H und CN.

14. Optoelektronisches Bauelement mit einer Elektrode (2) und einem Gegenelektrode (6) und mindestens einer organischen lichtempfindlichen Schicht (4) zwischen der Elektrode (2) und der Gegenelektrode (6), **dadurch gekennzeichnet, dass** die organische lichtempfindliche Schicht (4) zumindest eine Verbindung gemäß den vorherigen Ansprüchen aufweist.

15. Verwendung einer Verbindung nach einem der Ansprüche 1 bis 13 in einem optoelektronischen Bauelement.

## Claims

1. Compounds of the general formula IIIa: 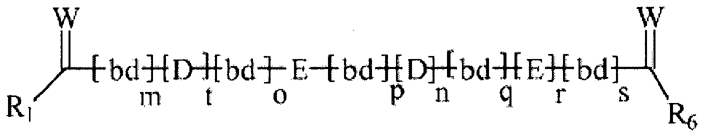
- where each W is independently selected from C(CN)₂, CHCN, C(CN)COOR' where R' is in each case selected from C1-C10 alkyl, C3-C10 aryl and C2-C8 heteroaryl, more preferably selected from C(CN)₂, CHCN,
- R₁ and R₆ are each independently selected from H, C1-C30 alkyl, C1-C30 perfluoroalkyl, C3-C10 aryl, C2-C8-heteroaryl, CN,
- where the D groups are selected from: 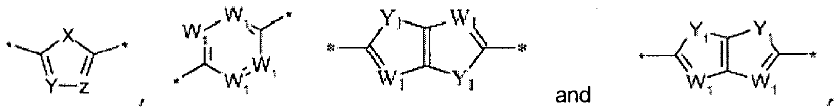
- where Y₁ is selected from: O, S, Se, P(R), P(O)R, Si(RR'), C(RR') and N(R) and
- W₁ is independently selected from: N and C-R where R and R' are each independently selected from substituted and unsubstituted C1 to C30 alkyl, C3-C6 aryl and C3-C8 heteroaryl
and
- where each X is selected independently from O, NR', S, Se, where R' is selected from C1-C30 alkyl, C1-C10 aryl and C1-C8 heteroaryl,
- each Y is independently selected from N and CR9 where R9 = H, halogen, C1-C30 alkyl, C1-C30 alkenyl, C1-C30 alkynyl, each linear or branched, substituted or unsubstituted, OR', SR', SiR'₃, NR'₂, where R' is selected from C1-C10 alkyl, C3-C10 aryl and C1-C8 heteroaryl,
- each Z is independently selected from N and CR10 where R10 = H, halogen, C1-C30 alkyl, C1-C30 alkenyl, C1-C30 alkynyl, each linear or branched, substituted or unsubstituted, OR', SR', SiR'₃, NR'₂, where R' is selected from C1-C10 alkyl, C3-C10 aryl and C1-C8 heteroaryl,
where R9 and R10 may form a ring, preferably a 5-membered or 6-membered ring,
- where the E groups are selected from: 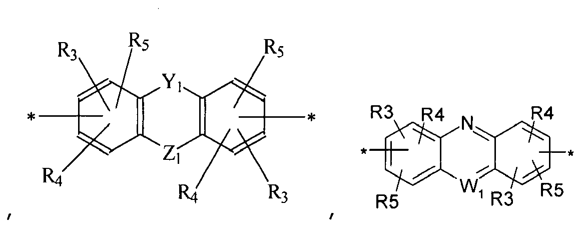 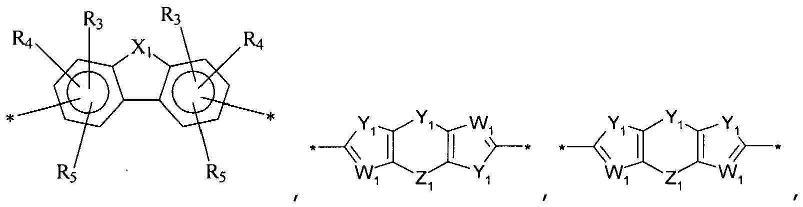 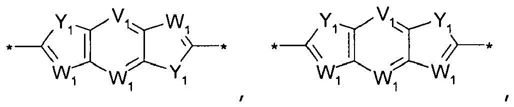 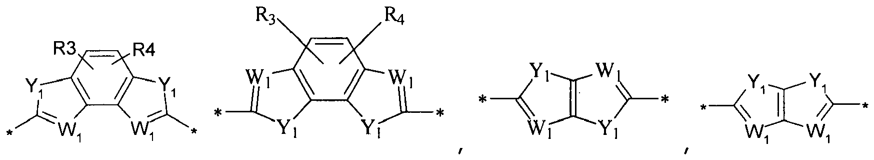 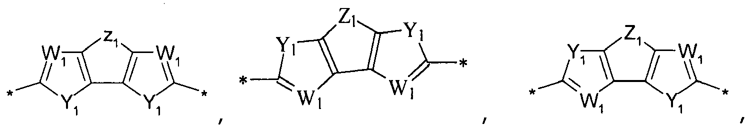 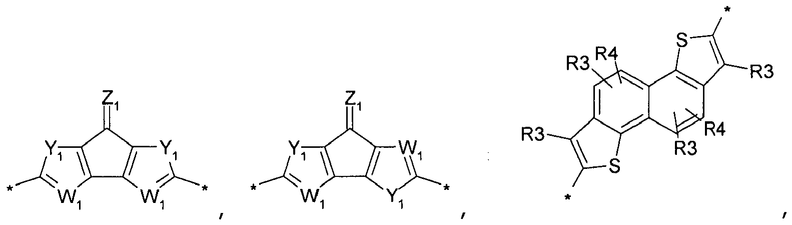 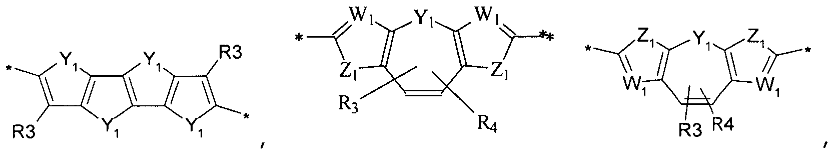
- where V₁ and W₁ are each independently selected from: N and C-R where R = H, halogen, C1-C30 alkyl, C1-C30 alkenyl, C1-C30 alkynyl, each linear or branched, substituted or unsubstituted, C3-C10 aryl or C1-C8 heteroaryl, substituted or unsubstituted, OR', SR', SiR'₃, NR'₂, where R' is selected from C1-C10 alkyl, C1-C10 aryl and C1-C8 heteroaryl,
- Y₁ and Z₁ are each selected from: O, S, Se, P (R), P(O)R, Si(RR'), C(RR') and N(R), where R and R' are each independently selected from H, linear or branched, substituted or unsubstituted C1-C30 alkyl, substituted or unsubstituted aryl, substituted or unsubstituted heteroaryl, acyl (COR'), COOR' and OR', where R' is selected from C1-C30 alkyl, C3-C10 aryl and C1-C8 heteroaryl, and
- X₁ is selected from: O, S, Se, and R and R' are each independently selected from H, C1 to C20 alkyl, linear or branched, substituted or unsubstituted, C3 to C6 aryl or C3 to C8 heteroaryl, OR', SR', SiR'₃, NR'₂, where R' is selected from C1-C10 alkyl, C3-C6 aryl and C3-C8 heteroaryl,
- and where R3, R4 and R5 are each independently H, C1 to C20 alkyl, linear or branched, substituted or substituted, C3 to C6 aryl or C3 to C8 heteroaryl, OR', SR', SiR'₃, NR'₂, where R' may be selected from C1-C10 alkyl, C3-C6 aryl and C3-C8 heteroaryl,
- bd is independently *-C≡C-* or *-C ≡ C-*,
n, m, o, p, q, and r, s and t may each independently be 0 or 1, with the proviso that at least one parameter is 1,
- where one donor unit formed from the groups bd, E and D has at least 10 conjugated electrons and
- the bonds identified with the asterisk* indicate the bonds to further groups in the compounds.

2. Compound according to Claim 1 where r=s=0 and D is
selected from

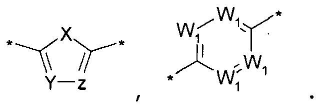

3. Compounds according to Claim 1, where the E group = 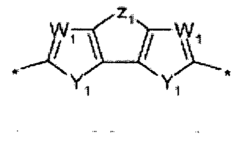 where Y₁ = S and the D groups = 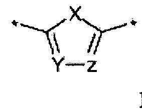 having the general formula:

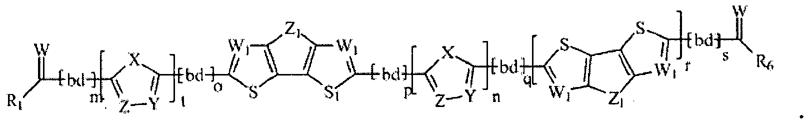

4. Compounds according to the preceding claim where m, r, s and q = 0 having the general formula: 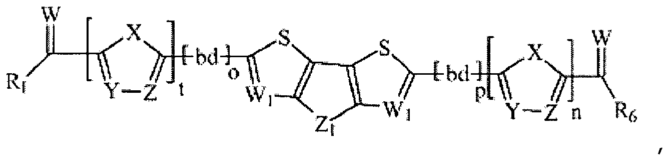 where preferably o and p = 0 and t and n = 1 and W₁ = C-R having the general formula:

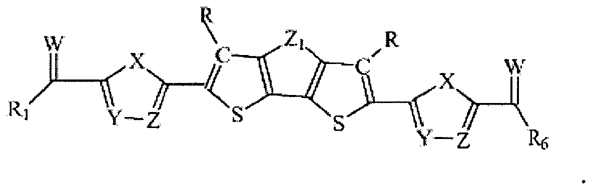

5. Compounds according to Claim 3 where t, m, o and s = 0 and r = 1 having the general formula: 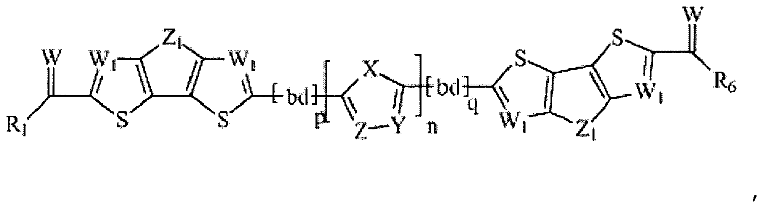 where preferably p and q = 0 and W₁ = C-R having the general formula:

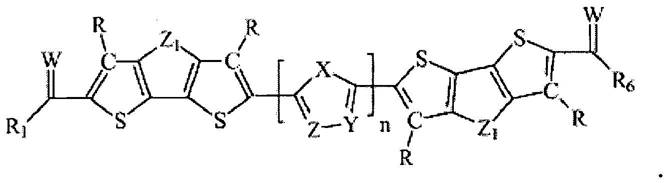

6. Compounds according to claim 1 where D = 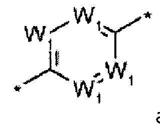 and E = 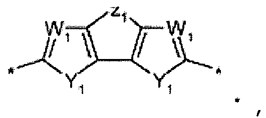 where preferably m, r, s and q = 0 and t and n each = 1 having the general formula:

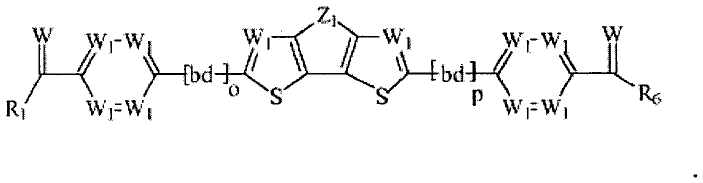

7. Compounds according to Claim 6 where o and p = 0 and W₁ = C-R.

8. Compounds according to Claim 1 where D = 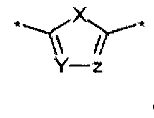 and E is selected from:

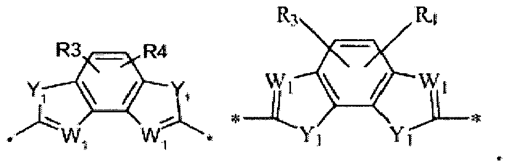

9. Compounds according to the preceding Claim 8 where m, q, r and s = 0 and t and n each = 1 having the general formula:

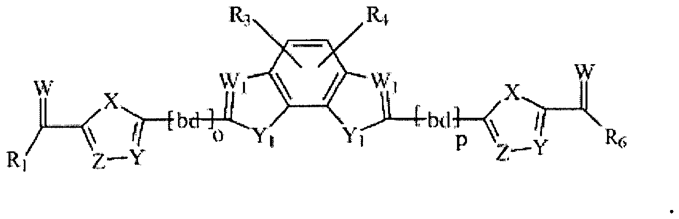

10. Compounds according to the preceding claim where X = S, and Y₁ = S and W₁ = C-R.

11. Compounds according to Claim 1 where m, t, n, o, q and s = 0 and r = 1, where E is selected from: 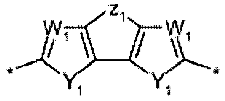 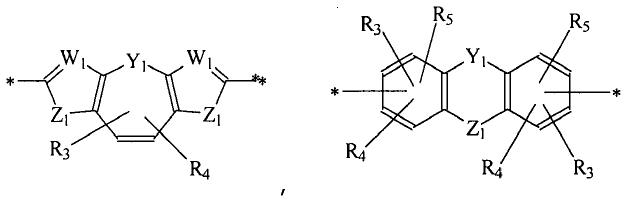 and

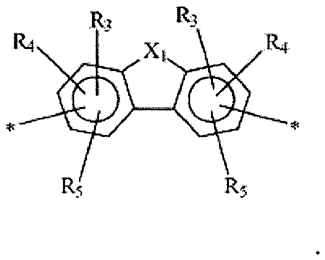

12. Compounds according to the preceding claim, where additionally p = 0.

13. Compounds according to any of the preceding claims, in which W is selected from: C(CN)₂, CHCN, and R₁ and R₆ are each selected from: H and CN.

14. Optoelectronic component having an electrode (2) and a counterelectrode (6) and at least one organic light-sensitive layer (4) between the electrode (2) and the counterelectrode (6), **characterized in that** the organic light-sensitive layer (4) comprises at least one compound according to the preceding claims.

15. Use of a compound according to any of Claims 1 to 13 in an optoelectronic component.

## Revendications

1. Composés de formule générale IIIa : 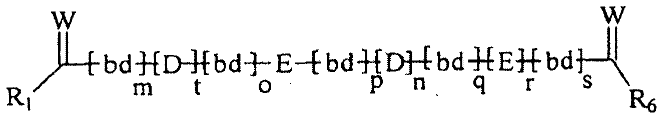
- les W étant chacun choisis indépendamment l'un de l'autre parmi C(CN)₂, CHCN, C(CN)COOR', R' étant à chaque fois choisi parmi alkyle en C1-C10, aryle en C3-C10 ou hétéroaryle en C2-C8, de manière particulièrement préférée parmi C(CN)₂, CHCN,
- R1 et R6 étant chacun choisis indépendamment l'un de l'autre parmi H, alkyle en C1-C30, perfluoroalkyle en C1-C30, aryle en C3-C10, hétéroaryle en C2-C8, CN,
- les groupes D étant choisis parmi : 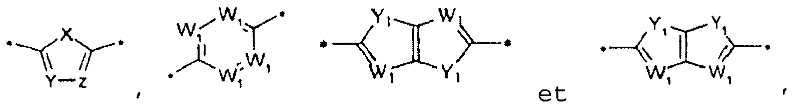
- les Y1 étant choisis parmi : O, S, Se, P(R), P(O)R, Si(RR'), C(RR') et N(R), et
- les W1 étant choisis indépendamment parmi : N et C-R, avec R et R' choisis indépendamment l'un de l'autre parmi alkyle en C1 à C30 substitué ou non substitué, aryle en C3-C6 ou hétéroaryle en C3-C8,
et
- les X étant chacun choisis indépendamment les uns des autres parmi O, NR', S, Se, avec R' choisi parmi alkyle en C1-C30, aryle en C1-C10 ou hétéroaryle en C1-C8,
- les Y étant chacun choisis indépendamment les uns des autres parmi N ou CR9, avec R9 = H, halogène, alkyle en C1-C30, alcényle en C1-C30, alcynyle en C1-C30, à chaque fois linéaire ou ramifié, substitué ou non substitué, OR', SR', SiR'₃, NR'₂ avec R' choisi parmi alkyle en C1-C10, aryle en C3-C10 ou hétéroaryle en C1-C8,
- les Z étant chacun choisis indépendamment les uns des autres parmi N ou CR10, avec R10 = H, halogène, alkyle en C1-C30, alcényle en C1-C30, alcynyle en C1-C30, à chaque fois linéaire ou ramifié, substitué ou non substitué, OR', SR', SiR'₃, NR'₂ avec R' choisi parmi alkyle en C1-C10, aryle en C3-C10 ou hétéroaryle en C1-C8,
R9 et R10 pouvant former un cycle, de préférence un cycle à 5 éléments ou un cycle à 6 éléments,
- les groupes E étant choisis parmi : 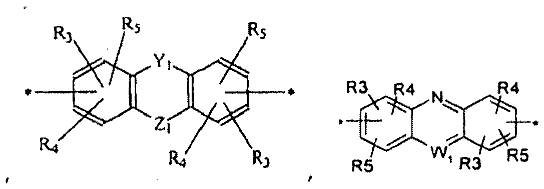 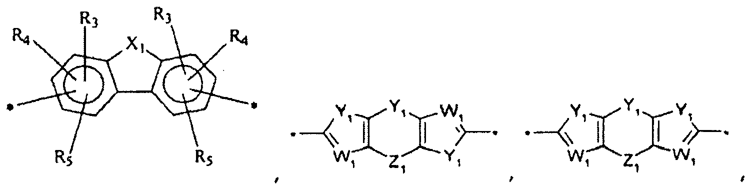 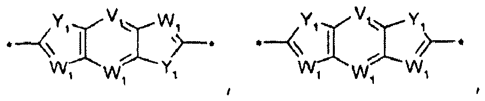 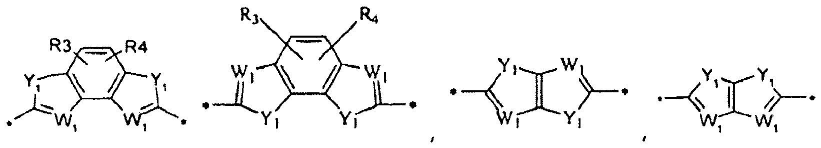 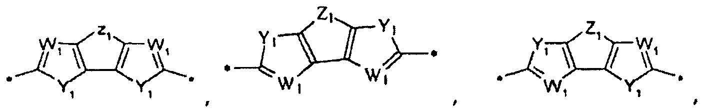 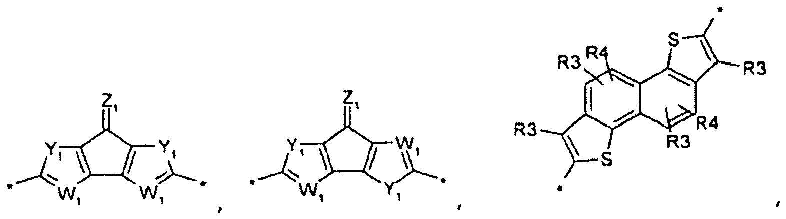 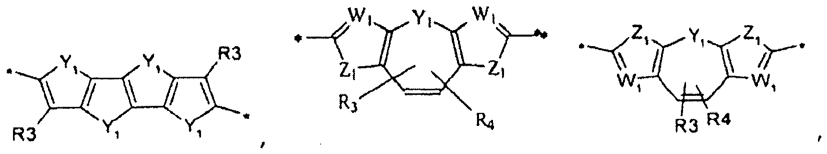
- V1 et W1 étant choisis indépendamment l'un de l'autre parmi : N et C-R, avec R = H, halogène, alkyle en C1-C30, alcényle en C1-C30, alcynyle en C1-C30, à chaque fois linéaire ou ramifié, substitué ou non substitué, aryle en C3-C10 ou hétéroaryle en C1-C8, substitué ou non substitué, OR', SR', SiR'₃, NR'₂ avec R' choisi parmi alkyle en C1-C10, aryle en C1-C10 ou hétéroaryle en C1-C8,
- Y1 et Z1 étant choisis parmi : O, S, Se, P(R), P(O)R, Si(RR'), C(RR') et N(R) avec R et R' chacun choisis indépendamment l'un de l'autre parmi H, alkyle en C1-C30 linéaire ou ramifié, substitué ou non substitué, aryle substitué ou non substitué, hétéroaryle substitué ou non substitué, acyle (COR'), COOR' ou OR' avec R' choisi parmi alkyle en C1-C30, aryle en C3-C10 ou hétéroaryle en C1-C8, et
- X1 étant choisi parmi : O, S, Se, et R et R' étant chacun choisis indépendamment l'un de l'autre parmi H, alkyle en C1 à C20, linéaire ou ramifié, substitué ou non substitué, aryle en C3 à C6 ou hétéroaryle en C3 à C8, OR', SR', SiR'₃, NR'₂ avec R' choisi parmi alkyle en C1-C10, aryle en C3-C6 ou hétéroaryle en C3-C8,
- et R3, R4 et R5 pouvant représenter indépendamment les uns des autres H, alkyle en C1 à C20, linéaire ou ramifié, substitué ou non substitué, aryle en C3 à C6 ou hétéroaryle en C3 à C8, OR', SR', SiR'₃, NR'₂ avec R' choisi parmi alkyle en C1-C10, aryle en C3-C6 ou hétéroaryle en C3-C8,
- les bd signifiant indépendamment les uns des autres *-C=C-* ou *-C=C-*,
n, m, o, p, q, et r, s et t pouvant représenter indépendamment les uns des autres 0 ou 1, à condition qu'au moins un paramètre représente 1,
- une unité donneuse formée à partir des groupes bd, E et D comprenant au moins 10 électrons conjugués et
- les liaisons **caractérisées par** l'étoile * indiquant les liaisons vers d'autres groupes dans les composés.

2. Composé selon la revendication 1, dans lequel r = s = 0 et D est choisi parmi

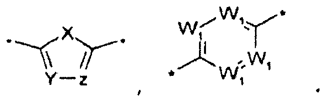

3. Composés selon la revendication 1, dans lesquels le
groupe E = 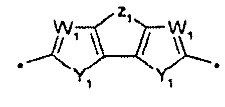 avec Y1 = S et les groupes D = 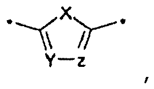 de formule générale :

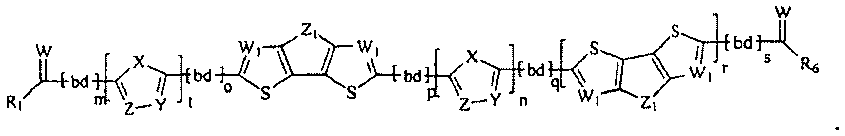

4. Composés selon la revendication précédente, dans lesquels m, r, s et q = 0, de formule générale : 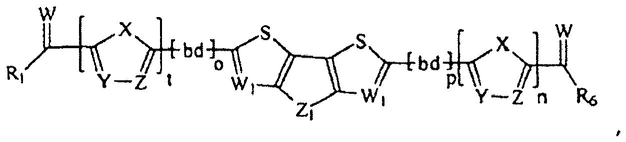 avec de préférence o et p = 0 et t et n = 1 et W1 = C-R, de formule générale :

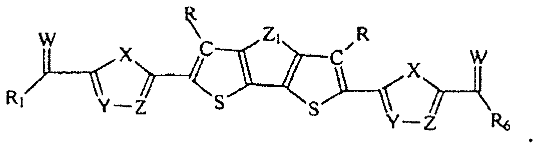

5. Composés selon la revendication 3, dans lesquels t, m, o et s = 0 et r = 1, de formule générale : 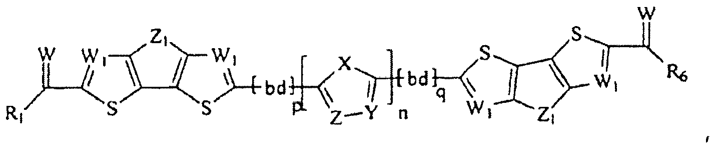 avec de préférence p et q = 0 et W1 = C-R, de formule générale :

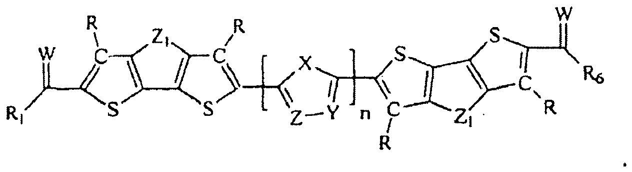

6. Composés selon la revendication 1, avec D = 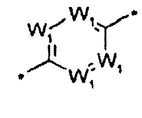 et E = 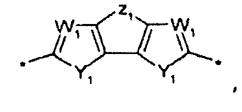 avec de préférence m, r, s et q = 0 et t et n chacun = 1, de formule générale .

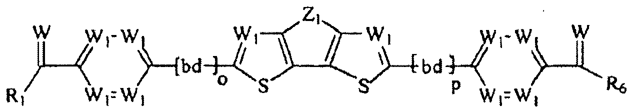

7. Composés selon la revendication 6, dans lesquels o et p = 0 et W1 = C-R.

8. Composés selon la revendication 1, dans lesquels D = 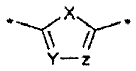 et E est choisi parmi :

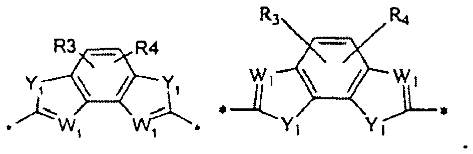

9. Composés selon la revendication 8 précédente, dans lesquels m, q, r et s = 0 et t et n chacun = 1, de formule générale :

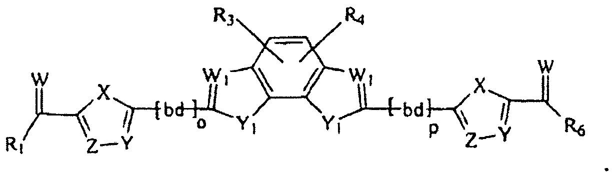

10. Composés selon la revendication précédente, dans lesquels X = S, et Y1 = S et W1 = C-R.

11. Composés selon la revendication 1, dans lesquels m, t, n, o, q et s = 0, et r = 1, E étant choisi parmi : 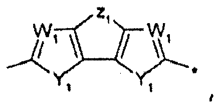 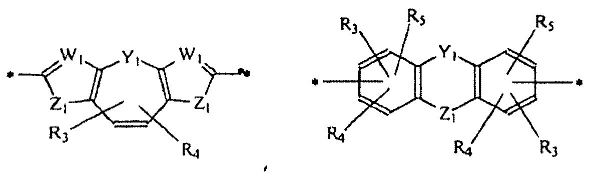 et

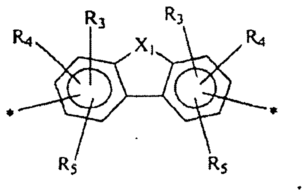

12. Composés selon la revendication précédente, dans lesquels en outre p = 0.

13. Composés selon l'une quelconque des revendications précédentes, dans lesquels W est choisi parmi : C(CN)₂, CHCN, et R1 et R6 sont choisis parmi : H et CN.

14. Composant optoélectronique comprenant une électrode (2) et une contre-électrode (6) et au moins une couche photosensible organique (4) entre l'électrode (2) et la contre-électrode (6), **caractérisé en ce que** la couche photosensible organique (4) comprend au moins un composé selon les revendications précédentes.

15. Utilisation d'un composé selon l'une quelconque des revendications 1 à 13 dans un composant optoélectronique.
